(19) Europäisches Patentamt
European Patent Office
Office européen des brevets

(11) EP 3 815 696 A1

(12) # EUROPEAN PATENT APPLICATION
published in accordance with Art. 153(4) EPC

(43) Date of publication:
**05.05.2021 Bulletin 2021/18**

(21) Application number: **19826094.5**

(22) Date of filing: **26.06.2019**

(51) Int Cl.:
***A61K 31/7088*** (2006.01) ***A61P 21/04*** (2006.01)
***C12N 15/113*** (2010.01) ***C12N 15/12*** (2006.01)

(86) International application number:
**PCT/JP2019/026393**

(87) International publication number:
**WO 2020/004675 (02.01.2020 Gazette 2020/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.06.2018 US 201862690270 P**
**01.10.2018 US 201862739386 P**

(71) Applicant: **Nippon Shinyaku Co., Ltd.**
**Kyoto-shi**
**Kyoto 601-8550 (JP)**

(72) Inventors:
- **UNO, Tomonori**
  **Kyoto-shi, Kyoto 601-8550 (JP)**
- **NATSUKAWA, Takashi**
  **Kyoto-shi, Kyoto 601-8550 (JP)**
- **EGAWA, Youichi**
  **Kyoto-shi, Kyoto 601-8550 (JP)**
- **SATOU, Youhei**
  **Ridgewood Avenue, Suite 280S, Paramus, New Jersey**
  **07652 (US)**

(74) Representative: **Hoffmann Eitle**
**Patent- und Rechtsanwälte PartmbB**
**Arabellastraße 30**
**81925 München (DE)**

(54) **COMPOSITION COMPRISING ANTISENSE OLIGONUCLEOTIDE AND USE THEREOF FOR TREATMENT OF DUCHENNE MUSCULAR DYSTROPHY**

(57) The present invention relates to a composition containing an antisense oligonucleotide, and the use thereof to treat Duchenne muscular dystrophy. The present invention particularly relates to the above-described composition that is effective for the treatment of Duchenne muscular dystrophy when it is administered at a dose for the treatment, and the use thereof.

Figure 2

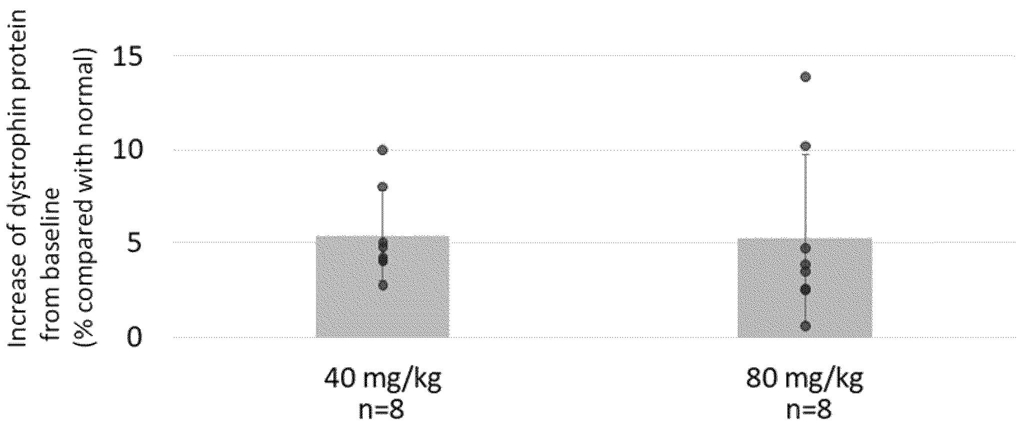


EP 3 815 696 A1

## Description

### Technical Field

The present invention relates to dosage and administration of an antisense oligomer capable of the exon 53 skipping of a human dystrophin gene, and a pharmaceutical composition comprising the oligomer.

### Background Art

Duchenne muscular dystrophy (DMD) is most frequently occurring hereditary progressive muscular atrophy, and DMD affects about one in 3,500 boys at birth. In infancy, the DMD patients exhibit motor functions that are almost the same as those of healthy humans, but muscle weakness is observed around the age of four or five. Then, muscle weakness progresses, and most are unable to walk by the age of 12. The patients die due to heart failure or respiratory failure in their 20s. Hence, DMD is very severe disease. At present, there are no effective therapeutic methods to DMD, and thus, it has been strongly desired to develop a novel therapeutic agent.

It has been known that DMD is caused by a mutation of a dystrophin gene. The dystrophin gene is an enormous gene consisting of DNA of 2,200,000 base pairs, which exists on the X chromosome. The DNA is transcribed into an mRNA precursor, and introns are then removed by splicing, so that mRNA containing 79 exons is synthesized. From this mRNA, 3,685 amino acids are translated, so that a dystrophin protein is generated. The dystrophin protein is associated with the maintenance of membrane stability of muscle cells, and is necessary to prevent destruction of the muscle cells. Since the dystrophin gene of DMD patients has a mutation, a dystrophin protein functioning in muscle cells is hardly expressed. Thus, in the body of DMD patients, the structure of muscle cells cannot be maintained, and a large amount of calcium ions flow into the muscle cells. As a result, a reaction similar to inflammation occurs, fibrosis progresses, and thereby, the muscle cells are not regenerated.

Becker muscular dystrophy (BMD) is also caused by a mutation of a dystrophin gene. The symptoms of BMD also exhibit muscle weakness due to muscle atrophy, but the symptoms of muscle weakness are generally milder than those of DMD, and the muscle weakness progresses slowly. In a majority of cases, BMD is developed in adulthood. It has been considered that such differences in clinical symptoms between DMD and BMD are caused by whether the amino acid reading frame is destroyed due to mutation or is maintained when the mRNA of dystrophin is translated into a dystrophin protein (Non Patent Literature 1). That is to say, in DMD, there is a mutation of shifting the amino acid reading frame, and thus almost no functional dystrophin proteins are expressed. On the other hand, in BMD, although some exons are deleted due to mutation, the amino acid reading frame is maintained, and thus, functional dystrophin proteins are generated, although the function is insufficient.

As a therapeutic method for DMD, an exon-skipping method is expected. According to this method, the amino acid reading frame of dystrophin mRNA is restored by modifying splicing, and the expression of a dystrophin protein having a partially recovered function is induced (Non Patent Literature 2). The amino acid sequence portion as a target of exon skipping is lost. As such, the dystrophin protein expressed as a result of this treatment becomes shorter than a normal dystrophin protein. However, since the amino acid reading frame is maintained, the function to stabilize muscle cells is partially retained. Accordingly, it is expected that, as a result of exon skipping, DMD becomes to exhibit symptoms similar to those of milder BMD. The exon-skipping method has been subjected to animal experiments using mice or dogs, and now, clinical studies are conducted on human DMD patients.

Exon skipping can be induced by the binding of antisense nucleic acids that targets either one or both of 5' and 3' splice sites, or the inside of an exon. The exon is included in mRNA, only when both of the splice sites are recognized by a spliceosome complex. Accordingly, by targeting splice sites with antisense nucleic acids, exon skipping can be induced. Moreover, in order to recognize the exon by the splicing mechanism, it has been considered necessary that the SR protein binds to an exon splicing enhancer (ESE), and exon skipping can also be induced by targeting ESE.

The mutation of the dystrophin gene is different depending on individual DMD patients. Thus, tailored antisense nucleic acids are necessary depending on the position or type of a genetic mutation. To date, antisense nucleic acids that induce exon skipping to all of the 79 exons have been produced by Steve Wilton et al. at the University of Western Australia (Non Patent Literature 3), and also, antisense nucleic acids that induce exon skipping to 39 exons have been produced by Annemieke Aartsma-Rus et al., in the Netherlands (Non Patent Literature 4).

It has been considered that approximately 10% of the total DMD patients can be treated by skipping the 53th exon (hereinafter referred to as "exon 53"). In recent years, multiple research institutions have reported studies regarding exon skipping of exon 53 of a dystrophin gene (Patent Literatures 1 to 4; and Non Patent Literatures 5 and 6).

Citation List

Patent Literature

Patent Literature 1: International Publication No. WO 2006/000057
Patent Literature 2: International Publication No. WO 2004/048570
Patent Literature 3: U.S. Patent Laid-Open Publication No. US 2010/0168212
Patent Literature 4: International Publication No. WO 2010/048586

Non Patent Literature

Non Patent Literature 1: Monaco A. P. et al., Genomics 1988; 2: pp. 90-95
Non Patent Literature 2: Matsuo M., Brain Dev 1996; 18: pp. 167-172
Non Patent Literature 3: Wilton S. D. et al., Molecular Therapy 2007: 15: pp. 1288-96
Non Patent Literature 4: Annemieke Aartsma-Rus et al., (2002) Neuromuscular Disorders 12: S71-S77
Non Patent Literature 5: Linda J. Popplewell et al., (2010) Neuromuscular Disorders, vol. 20, no. 2, pp. 102-10
Non Patent Literature 6: Bladen C. L. et al., Human Mutation (2015) 36: 395-402

Summary of Invention

The present invention is as follows, but is not restricted thereto.

<1> A pharmaceutical composition for treating a human patient with Duchenne muscular dystrophy, the pharmaceutical composition comprising an antisense oligomer consisting of a base sequence complementary to a sequence consisting of nucleotides at positions 36 to 56 from the 5'-terminus of exon 53 of a human dystrophin gene, or a pharmaceutically acceptable salt thereof, or a hydrate thereof, wherein
the treatment comprises intravenously administering to the human patient, the antisense oligomer, or a pharmaceutically acceptable salt thereof, or a hydrate thereof at a dose of between 40 mg/kg/week inclusive and 80 mg/kg/week inclusive.
<2> The pharmaceutical composition according to the above < 1 >, wherein the antisense oligomer, or a pharmaceutically acceptable salt thereof, or a hydrate thereof is intravenously administered to the human patient at a dose of 40 mg/kg/week.
<3> The pharmaceutical composition according to the above < 1 >, wherein the antisense oligomer, or a pharmaceutically acceptable salt thereof, or a hydrate thereof is intravenously administered to the human patient at a dose of 80 mg/kg/week.
<4> The pharmaceutical composition according to the above < 1 >, wherein the human patient has a mutation that results in a deficiency of any exon selected from the group consisting of exons 43-52, 45-52, 47-52, 48-52, 49-52, 50-52, or 52, in a dystrophin gene.
<5> The pharmaceutical composition according to the above < 1 >, wherein the expression of a dystrophin protein in the human patient before the treatment is 1% or less compared with that of a healthy subject, as measured by Western blotting or mass spectrometry.
<6> The pharmaceutical composition according to the above < 5 >, wherein the expression of a dystrophin protein is not found in the human patient before the treatment.
< 7 > The pharmaceutical composition according to the above < 1 >, wherein the base sequence of the antisense oligomer consists of the sequence as set forth in SEQ ID NO: 3.
<8> The pharmaceutical composition according to the above < 1 >, wherein the antisense oligomer, or a pharmaceutically acceptable salt thereof, or a hydrate thereof is Viltolarsen or an equivalent thereof.
<9> The pharmaceutical composition according to the above < 1 >, comprising the antisense oligomer, or a pharmaceutically acceptable salt thereof, or a hydrate thereof, in a concentration of between 2.5 mg/ml inclusive and 500 mg/ml inclusive, or between 10 mg/ml inclusive and 100 mg/ml inclusive.
<10> The pharmaceutical composition according to the above < 1 >, comprising the antisense oligomer, or a pharmaceutically acceptable salt thereof, or a hydrate thereof, in a concentration of 25 mg/ml.
<11> The pharmaceutical composition according to the above < 1 >, comprising the antisense oligomer, or a pharmaceutically acceptable salt thereof, or a hydrate thereof, in a concentration of 50 mg/ml.

<12> The pharmaceutical composition according to the above < 1 >, further comprising at least one component selected from the group consisting of a tonicity agent, a pH adjuster, and a solvent.

<13> The pharmaceutical composition according to the above < 12 >, wherein the tonicity agent is at least one selected from the group consisting of sodium chloride, potassium chloride, glucose, fructose, maltose, sucrose, lactose, mannitol, sorbitol, xylitol, trehalose, and glycerin.

<14> The pharmaceutical composition according to the above < 12> or < 13 >, wherein the pH adjuster is at least one selected from the group consisting of hydrochloric acid, sodium hydroxide, citric acid, lactic acid, phosphate (sodium hydrogen phosphate, sodium dihydrogen phosphate, and potassium dihydrogen phosphate), and monoethanolamine.

< 15 > The pharmaceutical composition according to any one of the above < 12 > to < 14 >, wherein the solvent is water.

<16> The pharmaceutical composition according to the above < 1 >, which comprises the antisense oligomer in a concentration of between 2.5 mg/ml inclusive and 500 mg/ml inclusive, or between 10 mg/ml inclusive and 100 mg/ml inclusive, and sodium chloride in a concentration of between 8 mg/ml inclusive and 10 mg/ml inclusive, and which is an aqueous solution with pH 7.2 to 7.4.

<17> The pharmaceutical composition according to the above < 1 >, wherein the treatment provides at least one effect selected from the group consisting of the following effects (1) to (6):

(1) the average value of the expression level of a dystrophin protein in the skeletal muscle of the patient is 9-fold or more increased in comparison to baseline, after administration of the pharmaceutical composition for 24 weeks;
(2) a change in the velocity obtained from time to stand (TTSTAND) is -0.055 times/sec or more in comparison to baseline, at the time of the 25th week after administration of the pharmaceutical composition for 24 weeks;
(3) a change in the velocity obtained from time to run/walk 10 meters (TTRW) is -0.025 meters/sec or more in comparison to baseline, at the time of the 25th week after administration of the pharmaceutical composition for 24 weeks;
(4) a change in the velocity obtained from time to climb 4 stairs (TTCLIMB) is -0.060 times/sec or more in comparison to baseline, at the time of the 25th week after administration of the pharmaceutical composition for 24 weeks;
(5) a change in the score of North Star Ambulatory Assessment (NSAA) is -2.2 scores or more in comparison to baseline, at the time of the 25th week after administration of the pharmaceutical composition for 24 weeks; and
(6) a change in 6-minute walk test (6MWT) is -7.5 meters or more in comparison to baseline, at the time of the 25th week after administration of the pharmaceutical composition for 24 weeks.

<18> The pharmaceutical composition according to the above < 1 >, wherein when the treatment is performed on 7- to 9-year-old human patients with Duchenne muscular dystrophy for 84 weeks, at least one effect selected from the group consisting of the following effects (1) to (6) is provided:

(1) the percentage of patients who lose rise ability is less than 20% by the time of the 85th week after initiation of the treatment;
(2) the percentage of patients who lose ability to climb 4 stairs is less than 10% by the time of the 85th week after initiation of the treatment;
(3) the percentage of patients who lose independent walking ability is less than 10% by the time of the 85th week after initiation of the treatment;
(4) a reduction in the velocity of running/walking 10 meters due to aging is not observed by the time of the 85th week after initiation of the treatment;
(5) a reduction in the velocity of climbing 4 stairs due to aging is not observed by the time of the 85th week after initiation of the treatment; and
(6) a reduction in rise velocity due to aging is not observed by the time of the 85th week after initiation of the treatment.

<19> The pharmaceutical composition according to the above < 1 >, wherein when the treatment is performed on 10- to 12-year-old human patients with Duchenne muscular dystrophy for 84 weeks, at least one effect selected from the group consisting of the following effects (1) to (6) is provided:

(1) the percentage of patients who lose rise ability is less than 60% by the time of the 85th week after initiation of the treatment;
(2) the percentage of patients who lose ability to climb 4 stairs is less than 50% by the time of 85th week after initiation of the treatment;

(3) the percentage of patients who lose independent walking ability is less than 50% by the time of the 85th week after initiation of the treatment;
(4) a reduction in the velocity of running/walking 10 meters due to aging is not observed by the time of the 85th week after initiation of the treatment;
(5) a period in which the velocity of climbing 4 stairs increases is observed by the time of the 85th week after initiation of the treatment; and
(6) a period in which rise velocity increases is observed by the time of the 85th week after initiation of the treatment.

<20> A method for treating Duchenne muscular dystrophy, comprising intravenously administering a pharmaceutical composition comprising an antisense oligomer consisting of a base sequence complementary to a sequence consisting of nucleotides at positions 36 to 56 from the 5'-terminus of exon 53 of a human dystrophin gene, or a pharmaceutically acceptable salt thereof, or a hydrate thereof, to a human patient once a week at a dose of between 40 mg/kg/week inclusive and 80 mg/kg/week inclusive of the antisense oligomer, or a pharmaceutically acceptable salt thereof, or a hydrate thereof.
<20-1> The treatment method according to the above < 20 >, wherein at least one of the effects according to claim 17, at least one of the effects according to claim 18, or at least one of the effects according to claim 19 is provided by the treatment method.
<21 > An antisense oligomer consisting of a base sequence complementary to a sequence consisting of nucleotides at positions 36 to 56 from the 5'-terminus of exon 53 of a human dystrophin gene, or a pharmaceutically acceptable salt thereof, or a hydrate thereof, for use in a method for treating a human patient with Duchenne muscular dystrophy, wherein
the antisense oligomer, or a pharmaceutically acceptable salt thereof, or a hydrate thereof is intravenously administered to the human patient once a week at a dose of between 40 mg/kg/week inclusive and 80 mg/kg/week inclusive.
<21-1> The antisense oligomer according to the above < 21 >, or a pharmaceutically acceptable salt thereof, or a hydrate thereof, wherein at least one of the effects according to claim 17, at least one of the effects according to claim 18, or at least one of the effects according to claim 19 is provided by administration of the antisense oligomer, or a pharmaceutically acceptable salt thereof, or a hydrate thereof.
<22> Use of an antisense oligomer consisting of a base sequence complementary to a sequence consisting of nucleotides at positions 36 to 56 from the 5'-terminus of exon 53 of a human dystrophin gene, or a pharmaceutically acceptable salt thereof, or a hydrate thereof, for the manufacture of a pharmaceutical composition for treating a human patient with Duchenne muscular dystrophy, wherein
the antisense oligomer, or a pharmaceutically acceptable salt thereof, or a hydrate thereof is intravenously administered to the human patient once a week at a dose of between 40 mg/kg/week inclusive and 80 mg/kg/week inclusive.

In addition, as another aspect, the present invention is as follows, but is not restricted thereto.

[1] A method for treating a subject with Duchenne muscular dystrophy amenable to a treatment involving exon 53 skipping, wherein the method comprises a step of intravenously administering NS-065/NCNP-01 to the subject at a dose of about 40 mg/kg/week.
[2] A method for treating a subject with Duchenne muscular dystrophy amenable to a treatment involving exon 53 skipping, wherein the method comprises a step of intravenously administering NS-065/NCNP-01 to the subject at a dose of about 80 mg/kg/week.
[3] A method for treating a subject with Duchenne muscular dystrophy amenable to a treatment involving exon 53 skipping, wherein the method comprises a step of intravenously administering NS-065/NCNP-01 to the subject at a dose of 40 mg/kg/week or more and 80 mg/kg/week or less.
[4] The method according to the above [1], wherein what is administered is an aqueous solution comprising:

NS-065/NCNP-01 in a concentration of 2.5 mg/mL or more and 500 mg/mL or less; and
sodium chloride as a tonicity agent in a concentration of 8.55 mg/mL or more and 9.45 mg/mL or less, and
wherein the aqueous solution has a pH value of about 7.3.

[5] The method according to the above [2], wherein what is administered is an aqueous solution comprising:

NS-065/NCNP-01 in a concentration of 2.5 mg/mL or more and 500 mg/mL or less; and
sodium chloride as a tonicity agent in a concentration of 8.55 mg/mL or more and 9.45 mg/mL or less, and
wherein the aqueous solution has a pH value of about 7.3.

[6] The method according to the above [3], wherein what is administered is an aqueous solution comprising:

NS-065/NCNP-01 in a concentration of 2.5 mg/mL or more and 500 mg/mL or less; and
sodium chloride as a tonicity agent in a concentration of 8.55 mg/mL or more and 9.45 mg/mL or less, and
wherein the aqueous solution has a pH value of about 7.3.

[7] A method for inducing generation of a dystrophin protein in a subject with Duchenne muscular dystrophy amenable to a treatment involving exon 53 skipping, wherein the method comprises a step of intravenously administering NS-065/NCNP-01 to the subject at a dose of about 40 mg/kg/week.
[8] A method for inducing generation of a dystrophin protein in a subject with Duchenne muscular dystrophy amenable to a treatment involving exon 53 skipping, wherein the method comprises a step of intravenously administering NS-065/NCNP-01 to the subject at a dose of about 80 mg/kg/week.
[9] A method for inducing generation of a dystrophin protein in a subject with Duchenne muscular dystrophy amenable to a treatment involving exon 53 skipping, wherein the method comprises a step of intravenously administering NS-065/NCNP-01 to the subject at a dose of 40 mg/kg/week or more and 80 mg/kg/week or less.
[10] The method according to the above [7], wherein what is administered is an aqueous solution comprising:

NS-065/NCNP-01 in a concentration of 2.5 mg/mL or more and 500 mg/mL or less; and
sodium chloride as a tonicity agent in a concentration of 8.55 mg/mL or more and 9.45 mg/mL or less, and
wherein the aqueous solution has a pH value of about 7.3.

[11] The method according to the above [8], wherein what is administered is an aqueous solution comprising:

NS-065/NCNP-01 in a concentration of 2.5 mg/mL or more and 500 mg/mL or less; and
sodium chloride as a tonicity agent in a concentration of 8.55 mg/mL or more and 9.45 mg/mL or less, and
wherein the aqueous solution has a pH value of about 7.3.

[12] The method according to the above [9], wherein what is administered is an aqueous solution comprising:

NS-065/NCNP-01 in a concentration of 2.5 mg/mL or more and 500 mg/mL or less; and
sodium chloride as a tonicity agent in a concentration of 8.55 mg/mL or more and 9.45 mg/mL or less, and
wherein the aqueous solution has a pH value of about 7.3.
In the above [1] to [12], NS-065/NCNP-01 (which is also referred to as "Viltolarsen" in the present description) may also be an equivalent thereof. In addition, in the above [1] to [12], the subject may also be a human patient.

According to the present invention, provided is a pharmaceutical composition for use in the treatment of Duchenne muscular dystrophy, which has s stable composition of Viltolarsen. Moreover, with regard to the pharmaceutical composition comprising Viltolarsen, are provided dosage and administration method of Viltolarsen, which exhibit effective therapeutic effects on Duchenne muscular dystrophy and are in a safe range for human patients. Using the pharmaceutical composition, the symptoms of Duchenne muscular dystrophy can be effectively reduced with low side effects.

Brief Description of Drawings

[Figure 1] Figure 1 shows a design of the US phase 2 dose-finding study NS-065/NCNP-01-201.
[Figure 2] Figure 2 shows the results of the measurement of the *de novo* expression level of a dystrophin protein in skeletal muscle according to a Western blot method.
[Figure 3] Figure 3 shows a design of the US/Canada phase 2 dose-finding study NS-065/NCNP-01-201.
[Figure 4] Figure 4 shows comparisons regarding changes from the baseline in the results of timed function tests conducted over 24 weeks. In the five graphs, the term "Viltolarsen" means the international nonproprietary name (INN) of NS-065/NCNP-01.
[Figure 5] Figure 5 shows the results of evaluation of the stability of Viltolarsen in a Britton-Robinson buffer (pH 3 to 11).
[Figure 6] Figure 6 shows the results of evaluation of the stability of Viltolarsen in a potassium phosphate-borax buffer (pH 6 to 9).
[Figure 7] Figure 7 shows the results of examination of pH adjusters at 121°C.
[Figure 8] Figure 8 includes graphs showing changes from the baseline in individual motor function test results.
[Figure 9] Figure 9 is a graph showing the results of a 6-minute walk test conducted on individual tested patients of Viltolarsen administered groups (40 mg/kg dose group and 80 mg/kg dose group) at the time of the 85th week after 84 weeks from initial administration).

[Figure 10] Figure 10 is a graph showing the scores of a North Star Ambulatory Assessment conducted on individual tested patients of Viltolarsen administered groups (40 mg/kg dose group and 80 mg/kg dose group) at the time of the 85th week (after 84 weeks from initial administration).

[Figure 11] Figure 11 is a graph showing the results of the velocity of a time to stand test conducted on individual tested patients of Viltolarsen administered groups (40 mg/kg dose group and 80 mg/kg dose group) at the time of the 85th week (after 84 weeks from initial administration).

[Figure 12] Figure 12 is a graph showing the results of the velocity of a time to climb 4 stairs test conducted on individual tested patients of Viltolarsen administered groups (40 mg/kg dose group and 80 mg/kg dose group) at the time of the 85th week (after 84 weeks from initial administration).

[Figure 13] Figure 13 is a graph showing the results of the velocity of a time to run/walk 10 meters test conducted on individual tested patients of Viltolarsen administered groups (40 mg/kg dose group and 80 mg/kg dose group) at the time of the 85th week (after 84 weeks from initial administration).

[Figure 14] Figure 14 is a graph showing a correlation between a change in the expression level of dystrophin from the baseline of a quantitative dystrophin value measured by WB and a change in the velocity of a time to stand test from the baseline, in individual tested patients of Viltolarsen administered groups (40 mg/kg dose group and 80 mg/kg dose group) at the time of the 49th week (after 48 weeks from initial administration).

[Figure 15] Figure 15 is a graph showing a correlation between a change in the expression level of dystrophin from the baseline of a quantitative dystrophin value measured by WB and a change in the velocity of a time to climb 4 stairs test from the baseline, in individual tested patients of Viltolarsen administered groups (40 mg/kg dose group and 80 mg/kg dose group) at the time of the 49th week (after 48 weeks from initial administration).

[Figure 16] Figure 16 is a graph showing a correlation between a change in the expression level of dystrophin from the baseline of a quantitative dystrophin value measured by WB and a change in the velocity of a time to run/walk 10 meters test from the baseline, in individual tested patients of Viltolarsen administered groups (40 mg/kg dose group and 80 mg/kg dose group) at the time of the 49th week (after 48 weeks from initial administration).

## Description of Embodiments

Hereinafter, the present invention will be described in detail. The following embodiments are provided as examples for explaining the present invention, and thus, are not intended to limit the present invention to only these embodiments. The present invention may be carried out in various embodiments, without departing from the spirit of the invention.

All publications and patent publications such as patent laid-open publications or patent applications cited in the present description are incorporated herein by reference in their entirety. Moreover, the present description includes the contents described in the specifications and drawings of U.S. provisional patent application (US62/690,270) filed on June 26, 2018, and U.S. provisional patent application (US62/739,386) filed on October 1, 2018, to both of which the present application claims priority.

### I. First Embodiment

In a first embodiment of the present invention, a pharmaceutical composition for treating Duchenne muscular dystrophy is provided. Specifically, the pharmaceutical composition of the present invention is a pharmaceutical composition for treating a human patient with Duchenne muscular dystrophy, the pharmaceutical composition comprising an antisense oligomer consisting of a base sequence complementary to a sequence consisting of nucleotides at positions 36 to 56 from the 5'-terminus of exon 53 of a human dystrophin gene (hereinafter also referred to as " the oligomer of the present invention"), or a pharmaceutically acceptable salt thereof, or a hydrate thereof, wherein
the treatment comprises intravenously administering to the human patient, the antisense oligomer, or a pharmaceutically acceptable salt thereof, or a hydrate thereof at a dose of between 40 mg/kg/week inclusive and 80 mg/kg/week inclusive.

### [53th Exon of human dystrophin gene]

In the present invention, the term "gene" includes cDNA, an mRNA precursor, and mRNA, as well as a genomic gene. The gene is preferably an mRNA precursor, namely, pre-mRNA.

In the human genome, a human dystrophin gene is present in gene locus Xp21.2. The human dystrophin gene has a size of 3.0 Mbp, and this is the largest gene among known human genes. However, the size of the coding region of the human dystrophin gene is only 14 kb, and the coding region is dispersed as 79 exons in the dystrophin gene (Roberts, RG., et al., Genomics, 16: 536-538 (1993)). Pre-mRNA as a transcriptional product of the human dystrophin gene generates 14-kb mature mRNA as a result of splicing. The base sequence of the mature mRNA of a wild-type human dystrophin gene has been known (GenBank Accession No. NM 004006).

The base sequence of the exon 53 of the wild-type human dystrophin gene is as set forth in SEQ ID NO: 1.

The pharmaceutical composition of the present invention comprises an antisense oligomer consisting of a base sequence complementary to a sequence consisting of nucleotides at positions 36 to 56 from the 5'-terminus of exon 53 of a human dystrophin gene (the oligomer of the present invention), or a pharmaceutically acceptable salt thereof, or a hydrate thereof.

Herein, the oligomer of the present invention is produced for the purpose of modifying a protein encoded by a DMD-type dystrophin gene to a BMD-type dystrophin protein by skipping the exon 53. Accordingly, the exon 53 of a dystrophin gene as a target of the exon skipping with the oligomer of the present invention includes not only wild-type exon 53 but also mutant-type exon 53.

A specific example of such mutant-type exon 53 of a human dystrophin gene may be a polynucleotide having an identity of 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more, to the base sequence as set forth in SEQ ID NO: 1. In the present description, the term "polynucleotide" means DNA or RNA.

Besides, the identity of base sequences can be determined by using algorithm BLAST (Basic Local Alignment Search Tool) by Carlin and Arthur (Proc. Natl. Acad. Sci. USA 872264-2268, 1990; Proc Natl Acad Sci USA 90: 5873, 1993). Based on the algorithm of BLAST, programs called BLASTN or BLASTX have been developed (Altschul SF, et al: J Mol Biol 215: 403, 1990). When a base sequence is analyzed using BLASTN, parameters are set to be, for example, score = 100 and wordlength = 12. When BLAST and Gapped BLAST programs are used, the default parameters of individual programs are used.

In the present description, the "complementary base sequence" is not limited to a base sequence that forms a Watson-Crick base pair with the target base sequence, but also includes a base sequence that forms a wobble base pair. Herein, the term "Watson-Crick base pair" means a base pair in which hydrogen bonds are formed between adenine-thymine, adenine-uracil and guanine-cytosine, whereas the term "wobble base pair" means a base pair in which hydrogen bonds are formed between guanine-uracil, inosine-uracil, inosine-adenine and inosine-cytosine. Moreover, the "complementary base sequence" may not have complementarity of 100% to the target base sequence, and for example, the complementary base sequence may comprise 1, 2, 3, 4, or 5 non-complementary bases with respect to the target base sequence. Furthermore, the complementary base sequence may also be a base sequence that is shorter than the target base sequence by 1, 2, 3, 4, or 5 bases.

Examples of the sequence consisting of nucleotides at positions 36 to 56 from the 5'-terminus of exon 53 (SEQ ID NO: 2) and a base sequence complementary to the aforementioned sequence (SEQ ID NO: 3) are shown in the following Table 1.

[Table 1]

Table 1

| | |
|---|---|
| 5'-GAACACCTTCAGAACCGGAGG-3' | SEQ ID NO: 2 |
| 5'-CCTCCGGTTCTGAAGGTGTTC-3' | SEQ ID NO: 3 |

Herein the thymine "T" and the uracil "U" can be mutually exchanged with each other. Even if the base is either "T" or "U," it does not substantially affect the exon skipping activity of the oligomer of the present invention. Accordingly, in the present application, even if the "T" in the base sequence having a certain sequence number is "U," it is shown with the same sequence number. Therefore, the sequence disclosed in the present application inevitably includes both a "T" sequence and a "U" sequence.

In view of the foregoing, the base sequence of the oligomer of the present invention may consist of the sequence as set forth in SEQ ID NO: 3. Moreover, the oligomer of the present invention may not have a base sequence that is 100% complementary to the target sequence, as long as it enables the skipping of the exon 53 of a human dystrophin gene. For example, the oligomer of the present invention may comprise 1, 2, 3, 4, or 5 non-complementary bases with respect to SEQ ID NO: 2 as a target sequence. Otherwise, the oligomer of the present invention may be a base sequence that is shorter than the target base sequence by 1, 2, 3, 4, or 5 bases.

Whether or not the skipping of the exon 53 of a human dystrophin gene has occurred can be confirmed by: introducing the oligomer of the present invention into dystrophin-expressing cells (e.g., human rhabdomyosarcoma cells), amplifying a peripheral region of the exon 53 of the mRNA of a human dystrophin gene, from the total RNA of the above-described dystrophin-expressing cells, by RT-PCR; and then performing nested PCR or sequence analysis on the PCR amplified product. Alternatively, whether or not such skipping has occurred can also be confirmed by measuring the amount of exon 53 by a method such as RT-PCR, Western blot, or mass spectrometry in a sample derived from a patient

to whom the oligomer of the present invention has been administered.

Skipping efficiency can be obtained by recovering the mRNA of a human dystrophin gene from test cells, then measuring the polynucleotide amount "A" of a band involving exon 53 skipping and the polynucleotide amount "B" of a band not involving exon 53 skipping in the mRNA, and then calculating the skipping efficiency according to the following equation based on the measurement values "A" and "B."

$$\text{Skipping efficiency (\%)} = A / (A + B) \times 100$$

The oligomer of the present invention may include an oligonucleotide, a morpholino oligomer, and a peptide nucleic acid (PNA) oligomer. The oligomer of the present invention is preferably a morpholino oligomer.

The above-described oligonucleotide (hereinafter referred to as "the oligonucleotide of the present invention") is an oligomer of the present invention comprising a nucleotide as a constitutional unit, and such a nucleotide may be any of a ribonucleotide, a deoxyribonucleotide or a modified nucleotide.

The modified nucleotide means a ribonucleotide or a deoxyribonucleotide, in which the entire or a part of nucleic acid bases, sugar portions, and phosphate linkage portions that constitute the ribonucleotide or the deoxyribonucleotide is modified.

Examples of the nucleic acid base may include adenine, guanine, hypoxanthine, cytosine, thymine, uracil, and a modified nucleotide thereof. Example of such a modified nucleotide may include, but not limited to, pseudouracil, 3-methyluracil, dihydrouracil, 5-alkylcytosine (e.g., 5-methylcytosine), 5-alkyluracil (e.g., 5-ethyluracil), 5-halouracil (5-bromouracil), 6-azapyrimidine, 6-alkylpyrimidine (6-methyluracil), 2-thiouracil, 4-thiouracil, 4-acetylcytosine, 5-(carboxyhydroxymethyl)uracil, 5'-carboxymethylaminomethyl-2-thiouracil, 5-carboxymethylaminomethyluracil, 1-methyladenine, 1-methylhypoxanthine, 2,2-dimethylguanine, 3-methylcytosine, 2-methyladenine, 2-methylguanine, N6-methyladenine, 7-methylguanine, 5-methoxyaminomethyl-2-thiouracil, 5-methylaminomethyluracil, 5-methylcarbonylmethyluracil, 5-methyloxyuracil, 5-methyl-2-thiouracil, 2-methylthio-N6-isopentenyladenine, uracil-5-oxyacetic acid, 2-thiocytosine, purine, 2,6-diaminopurine, 2-aminopurine, isoguanine, indole, imidazole, and xanthine.

Examples of modification of a sugar portion may include modification of position 2' of ribose and modification of other positions of a sugar portion. Modification of position 2' of ribose may be, for example, modification of substituting the -OH group at position 2' of ribose with OR, R, R', OR, SH, SR, $NH_2$, NHR, $NR_2$, $N_3$, CN, F, Cl, Br, or I. Herein, R indicates alkyl or aryl. R' indicates alkylene.

Examples of modification of other positions of a sugar portion may include, but not limited to, substitution of O at position 4' of ribose or deoxyribose with S, and crosslinking between position 2' and position 4' of a sugar portion, such as LNA (Locked Nucleic Acid) or ENA (2'-O,4'-C-Ethylene-bridged Nucleic Acids).

Examples of modification of a phosphate linkage portion may be modification of substituting a phosphodiester bond with a phosphorothioate bond, a phosphorodithioate bond, an alkylphosphonate bond, a phosphoroamidate bond, or a boranophosphate bond (Enya et al.: Bioorganic & Medicinal Chemistry, 2008, 18, 9154-9160) (see, for example, Re-publication of PCT International Publication Nos. 2006/129594 and 2006/038608).

As an alkyl, a linear or branched alkyl containing 1 to 6 carbon atoms is preferable. Specific examples of such an alkyl may include methyl, ethyl, n-propyl, isopropyl, n-butyl, isobutyl, sec-butyl, tert-butyl, n-pentyl, isopentyl, neopentyl, tert-pentyl, n-hexyl, and isohexyl. The alkyl may be substituted. Examples of such a substituent may include halogen, alkoxy, cyano, and nitro. The alkyl may be substituted with 1 to 3 of these substituents.

As a cycloalkyl, a cycloalkyl containing 5 to 12 carbon atoms is preferable. Specific examples of such a cycloalkyl may include cyclopentyl, cyclohexyl, cycloheptyl, cyclooctyl, cyclodecyl, and cyclododecyl.

Examples of a halogen may include fluorine, chlorine, bromine, and iodine.

Examples of an alkoxy may include a linear or branched alkoxy containing 1 to 6 carbon atoms, such as methoxy, ethoxy, n-propoxy, isopropoxy, n-butoxy, isobutoxy, sec-butoxy, tert-butoxy, n-pentyloxy, isopentyloxy, n-hexyloxy, and isohexyloxy. Among others, an alkoxy containing 1 to 3 carbon atoms is preferable.

As an aryl, an aryl containing 6 to 10 carbon atoms is preferable. Specific examples of such an aryl may include phenyl, $\alpha$-naphthyl, and $\beta$-naphthyl. Among others, phenyl is preferable. The aryl may be substituted. Examples of such a substituent may include alkyl, halogen, alkoxy, cyano, and nitro. The aryl may be substituted with 1 to 3 of these substituents.

As an alkylene, a linear or branched alkylene containing 1 to 6 carbon atoms is preferable. Specific examples of such an alkylene may include methylene, ethylene, trimethylene, tetramethylene, pentamethylene, hexamethylene, and 2-(ethyl)trimethylene, 1-(methyl)tetramethylene.

Examples of an acyl may include a linear or branched alkanoyl and aroyl. Examples of the alkanoyl may include formyl, acetyl, 2-methylacetyl, 2,2-dimethylacetyl, propionyl, butyryl, isobutyryl, pentanoyl, 2,2-dimethylpropionyl, and hexanoyl. Examples of the aroyl may include benzoyl, toluoyl, and naphthoyl. Such an aroyl may be substituted at a replaceable position, and may be substituted with an alkyl.

In an aspect in which the oligomer of the present invention is an oligonucleotide, the oligonucleotide may preferably comprise, as a constitutional unit, a group represented by the following general formula, in which the -OH group at position 2' of ribose is substituted with a methoxy and the phosphate linkage portion is a phosphorothioate bond:

[Formula 1]

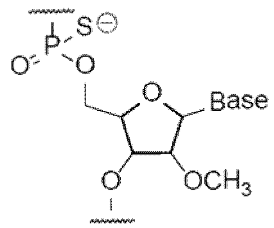

wherein Base indicates a nucleic acid base.

Such an oligonucleotide can be easily synthesized using various types of automatic synthesizers (e.g., AKTA oligopilot plus 10/100 (GE Healthcare)). Otherwise, the oligonucleotide can also be produced by outsourcing the synthesis thereof to a third-party organization (e.g., Promega or Takara), etc.

When the oligomer of the present invention is a morpholino oligomer, the morpholino oligomer may comprise, as a constitutional unit, a group represented by the following general formula:

[Formula 2]

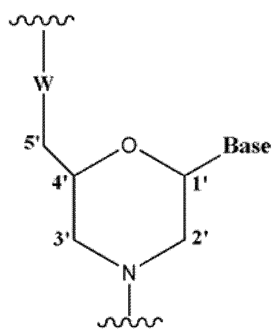

wherein Base is as defined above; and

W represents a group represented by any of the following formulae:

[Formula 3]

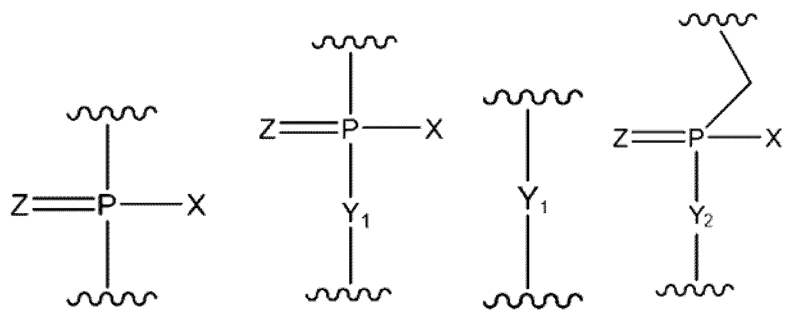

wherein X represents $-CH_2R^1$, $-O-CH_2R^1$, $-S-CH_2R^1$, $-NR^2R^3$, or F;

$R^1$ represents H or alkyl;
$R^2$ and $R^3$, which are the same or different, each represent H, alkyl, cycloalkyl, or aryl;
$Y_1$ represents O, S, $CH_2$, or $NR^1$;
$Y_2$ represents O, S, or $NR^1$; and
Z represents O or S.

The morpholino oligomer is preferably an oligomer comprising, as a constitutional unit, a group represented by the following formula (i.e., a phosphorodiamidate morpholino oligomer (hereinafter referred to as "PMO")):

[Formula 4]

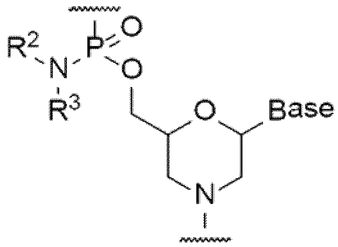

wherein Base, $R^2$, and $R^3$ are as described above.

The morpholino oligomer can be produced, for example, according to International Publication No. WO 1991/009033 or International Publication No. WO 2009/064471. In particular, PMO can be produced according to the method described in International Publication No. WO 2009/064471, or according to the method described below.

In one aspect, PMO may include, for example, a compound represented by the following general formula (I) (hereinafter referred to as "PMO (I)"):

[Formula 5]

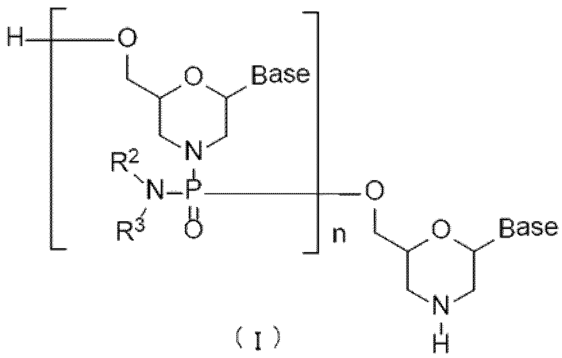

(I)

wherein each Base, $R^2$, and $R^3$ are as defined above; and

n represents any given integer in the range from 1 to 99, and preferably represents any given integer in the range from 18 to 28.

PMO (I) can be produced according to a known method, and compounds and reagents used in the production of PMO (I) are not particularly limited, as long as they are commonly used in production of PMO. In addition, the production can be carried out by a liquid phase method or a solid phase method (in which manuals or commercially available solid phase automatic synthesizers are used). When PMO is produced by a solid phase method, a method of using an automatic synthesizer is desirable from the viewpoint of simplification of operational procedures and accuracy in synthesis.

The peptide nucleic acid is an oligomer of the present invention comprising, as a constitutional unit, a group represented by the following general formula:

[Formula 6]

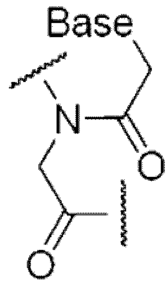

wherein Base is as described above.

The peptide nucleic acid can be produced, for example, according to the following publications:

1) P. E. Nielsen, M. Egholm, R. H. Berg, O. Buchardt, Science, 254, 1497 (1991)
2) M. Egholm, O. Buchardt, P. E. Nielsen, R. H. Berg, Jacs., 114, 1895 (1992)
3) K. L. Dueholm, M. Egholm, C. Behrens, L. Christensen, H. F. Hansen, T. Vulpius, K. H. Petersen, R. H. Berg, P. E. Nielsen, O. Buchardt, J. Org. Chem., 59, 5767 (1994) 4) L. Christensen, R. Fitzpatrick, B. Gildea, K. H. Petersen, H. F. Hansen, T. Koch, M. Egholm, O. Buchardt, P. E. Nielsen, J. Coull, R. H. Berg, J. Pept. Sci., 1, 175 (1995) 5) T. Koch, H. F. Hansen, P. Andersen, T. Larsen, H. G. Batz, K. Otteson, H. Orum, J. Pept. Res., 49, 80 (1997)

Moreover, the 5'-terminus of the oligomer of the present invention may be a group represented by any of the following chemical formulae (1) to (3). It is preferably -OH shown in (3).

[Formula 7]

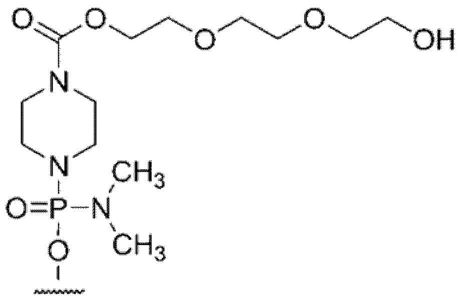

(1)

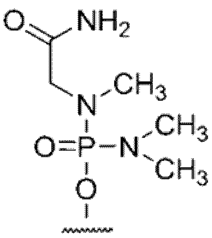

(2)

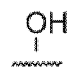

(3)

Hereafter, the groups represented by the above formulae (1), (2), and (3) are referred to as "group (1)," "group (2)," and "group (3)," respectively.

Examples of the pharmaceutically acceptable salt of the oligomer of the present invention may include: alkaline metal salts, such as sodium salts, potassium salts, or lithium salts; alkaline-earth metal salts, such as calcium salts, or magnesium salts; metal salts, such as aluminum salts, iron salts, zinc salts, copper salts, nickel salts, or cobalt salts; ammonium salts; organic amine salts, such as t-octylamine salts, dibenzylamine salts, morpholine salts, glucosamine salts, phenylglycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts, or tris(hydroxymethyl)aminomethane salts; hydrohalogenic acid salts, such as hydrofluoride, hydrochloride, hydrobromide, or hydroiodide; inorganic acid salts, such as nitrate, perchlorate, sulfate, or phosphate; lower alkane sulfonates, such as methanesulfonate, trifluoromethanesulfonate, or ethanesulfonate; arylsulfonates such as benzenesulfonate or p-toluenesulfonate; organic acid salts, such as acetate, malate, fumarate, succinate, citrate, tartrate, oxalate, or maleate; and amino acid salts, such as glycine salts, lysine salts, arginine salts, ornithine salts, glutamate, or aspartate. These salts can be produced according to a known method. Otherwise, the oligomer of the present invention may be in the form of

a hydrate thereof.

In another aspect, the oligomer of the present invention may be Viltolarsen or an equivalent thereof.

Viltolarsen is the international nonproprietary name (INN) of NS-065/NCNP-01. In the present description, NS-065/NCNP-01 is also referred to as NS-065/NCNP-01 (Viltolarsen) or Viltolarsen, as well as NS-065/NCNP-01.

NS-065/NCNP-01 (Viltolarsen) is an antisense oligonucleotide drug substance for treating patients with Duchenne muscular dystrophy (DMD) amenable to a treatment involving exon 53 skipping. NS-065/NCNP-01 (Viltolarsen) is a compound disclosed as "PMO No. 8" in U.S. Patent No. 9,079,934 B2. The base sequence of NS-065/NCNP-01 (Viltolarsen) is as set forth in SEQ ID NO: 35 (5'-CCTCCGGTTC TGAAGGTGTTC-3'; SEQ ID NO: 3 in the present description), and the 5'-terminus thereof is -OH. The content of U.S. Patent No. 9,079,934 B2 is incorporated in the present description by reference in its entirety. In addition, U.S. Patent No. 9,079,934 B2 discloses a method for synthesizing PMO No. 8, namely, NS-065/NCNP-01 (Viltolarsen).

NS-065/NCNP-01 (Viltolarsen) has a morpholino backbone that is anticipated to provide higher safety than phosphorothioate oligonucleotide. For example, the development of a phosphorothioate oligonucleotide, drisapersen (by BioMarin), has been suspended due to safety concerns. On the other hand, a morpholino oligonucleotide, eteplirsen (Exondys51® by Sarepta), has been approved by FDA. Both drisapersen and eteplirsen are for DMD patients amenable to a treatment involving exon 51 skipping. Eteplirsen is disclosed in U.S. Patent No. 9,506,058 B2, and the content thereof is incorporated in the present description by reference in its entirety.

As disclosed in U.S. Patent No. 9,079,934 B2, NS-065/NCNP-01 (Viltolarsen) has been designed to exhibit specific exon 53 skipping activity in order to produce a functional dystrophin protein in DMD patients with specific deficiencies of exons, including exons 43-52, 45-52, 47-52, 48-52, 49-52, 50-52, or 52. Examples of DMD-causing mutations theoretically curable by skipping a specified exon are given in Table 3 of Aartsma-Rus et al., 2002. The content of the publication by Aartsma-Rus et al. is incorporated by reference in its entirety (Annemieke Aartsma-Rus, Mattie Bremmer-Bout, Anneke A.M. Janson, Johan T. den Dunnen, Gert-Jan B. van Ommen, and Judith C.T. van Deutekom, "Targeted exon skipping as a potential gene correction therapy for Duchenne muscular dystrophy," Neuromuscular Disorders, Vol. 12, pp. S71-S77 (2002)).

The "equivalent" of Viltolarsen is a compound that is a generic drug of Viltolarsen or an active ingredient thereof. Such equivalents have obtained manufacturing and sales approval according to the Pharmaceutical Affairs Act, based on safety and effectiveness confirmed by clinical trials of Viltolarsen, without undergoing the clinical trials of the equivalents themselves, and the equivalents are expected to have exon 53 skipping activity similar to that of Viltolarsen. In a certain aspect, the "equivalent" of Viltolarsen has the same base sequence as that of Viltolarsen, and the "equivalent" of Viltolarsen includes equivalents, in which the entire or a part of the nucleic acid bases, sugar portions, and phosphate linkage portions of the equivalent are modified in the same manner as that for Viltolarsen, or are modified differently from Viltolarsen. The aspect of such modification is the same as the above-described aspect. Moreover, the "equivalent" of Viltolarsen may be in the form of a free body, a pharmaceutically acceptable salt, or a hydrate.

## 2. Pharmaceutical Product Composition

The pharmaceutical composition of the present invention may also be in the form of an aqueous solution. The pharmaceutical composition of the present invention may comprise the oligomer of the present invention, or a pharmaceutically acceptable salt thereof, or a hydrate thereof, in a concentration of 2.5 to 500 mg/ml, 5 to 450 mg/ml, 10 to 400 mg/ml, 15 to 350 mg/ml, 20 to 300 mg/ml, 20 to 250 mg/ml, 20 to 200 mg/ml, 20 to 150 mg/ml, 20 to 100 mg/ml, 20 to 50 mg/ml, 20 to 40 mg/ml, 20 to 30 mg/ml, 23 to 27 mg/ml, 24 to 26 mg/ml, or 25 mg/ml. Otherwise, the pharmaceutical composition of the present invention may comprise the oligomer of the present invention, or a pharmaceutically acceptable salt thereof, or a hydrate thereof, in a concentration of 10 to 100 mg/ml, 15 to 95 mg/ml, 20 to 80 mg/ml, 25 to 75 mg/ml, 30 to 70 mg/ml, 35 to 65 mg/ml, 40 to 60 mg/ml, 45 to 55 mg/ml, 47 to 53 mg/ml, 48 to 52 mg/ml, 49 to 51 mg/ml, or 50 mg/ml.

In the pharmaceutical composition of the present invention, the concentration of Viltolarsen in the aqueous solution may be changed. In order to prepare an aqueous solution of Viltolarsen, for example, 250 mg of Viltolarsen may be mixed into 0.5 mL to 100 mL of water (corresponding to a Viltolarsen concentration of 2.5 mg/mL to 500 mg/mL), more preferably 1 mL to 50 mL of water (corresponding to a Viltolarsen concentration of 5 mg/mL to 250 mg/mL), and most preferably 5 mL to 10 mL of water (corresponding to a Viltolarsen concentration of 25 mg/mL to 50 mg/mL).

The administration form of the pharmaceutical composition of the present invention is intravenous administration. The possible dosage form of the pharmaceutical composition of the present invention is, for example, an injection solution (including a drip liquid).

The pharmaceutical composition of the present invention may further comprise at least one component selected from a tonicity agent, a pH adjuster, and a solvent.

The tonicity agent comprised in the pharmaceutical composition of the present invention may be at least one selected from sodium chloride, potassium chloride, glucose, fructose, maltose, sucrose, lactose, mannitol, sorbitol, xylitol, trehalose, and glycerin.

10 mL of an aqueous solution comprising 250 mg of Viltolarsen suitable for injection may comprise, as a tonicity agent, 72.0 mg or more and 108.0 mg or less of sodium chloride (corresponding to sodium chloride in a concentration of 7.2 mg/mL to 10.8 mg/mL), more preferably 81.0 mg or more and 99.0 mg or less of sodium chloride (corresponding to sodium chloride in a concentration of 8.1 mg/mL to 9.9 mg/mL), and most preferably 85.5 mg or more and 94.5 mg or less of sodium chloride (corresponding to sodium chloride in a concentration of 8.55 mg/mL to 9.45 mg/mL).

As a tonicity agent, a phosphate buffer may be used. Examples of such a phosphate buffer may include a citrate buffer, a lactate buffer, and an acetate buffer. Moreover, as a tonicity agent, sugars (other than glucose) may also be used. Examples of such sugar may include sorbitol and mannitol. When a composition containing Viltolarsen is prepared, a plurality of tonicity agents may also be used.

The pH adjuster comprised in the pharmaceutical composition of the present invention may be at least one selected from hydrochloric acid, sulfuric acid, phosphoric acid, acetic acid, sodium hydroxide, potassium hydroxide, triethanolamine, citric acid, lactic acid, phosphate (sodium hydrogen phosphate, sodium dihydrogen phosphate, and potassium dihydrogen phosphate), and monoethanolamine.

In the case of using a phosphate buffer for the oligomer of the present invention, for example, for Viltolarsen, the concentration of the phosphate buffer is preferably less than 100 mM. Accordingly, the concentration of the phosphate buffer in the pharmaceutical composition of the present invention may be adjusted to 90 mM or less, 80 mM or less, 70 mM or less, 60 mM or less, 50 mM or less, 40 mM or less, 30 mM or less, 20 mM or less, 10 mM or less, or 5 mM or less, or the pharmaceutical composition of the present invention may not comprise a phosphate buffer.

The solvent comprised in the pharmaceutical composition of the present invention may be water.

The pH value of the aqueous solution comprising Viltolarsen suitable for injection may be pH 6.0 or more and 8.5 or less, more preferably pH 6.5 or more and 8.0 or less, and most preferably 7.0 or more and 7.5 or less.

The oligomer of the present invention exhibits stability in a wide range of pH values. The pH value of the pharmaceutical composition of the present invention is preferably adjusted to pH 7.0 to 7.5, 7.0 to 7.4, 7.1 to 7.5, 7.1 to 7.4, 7.2 to 7.5, 7.2 to 7.4, 7.3 to 7.5, 7.3 to 7.4, or 7.3.

In addition, the pharmaceutical composition of the present invention may be in the form of an aqueous solution comprising the oligomer of the present invention in a concentration of between 2.5 mg/ml inclusive and 500 mg/ml inclusive, or between 10 mg/ml inclusive and 100 mg/ml inclusive, and sodium chloride in a concentration of between 8 mg/ml inclusive and 10 mg/ml inclusive, and having a pH value of 7.2 to 7.4.

Alternatively, the pharmaceutical composition of the present invention may be in the form of an aqueous solution comprising the oligomer of the present invention in a concentration of 25 mg/ml and having a pH value that is adjusted to pH 7.3, without containing a buffer. In this pharmaceutical composition, a pH value is adjusted by using hydrochloric acid and/or sodium hydroxide.

As an example of the pharmaceutical composition of the present invention, a composition containing 250 mg of Viltolarsen for use in injection, which was used in the US/Canada phase 2 clinical program, is shown in the following Table 2. Hereafter, the composition shown in Table 2 is referred to as "NS-065/NCNP-01 (Viltolarsen) Injection 250 mg."

[Table 2]

Table 2 "NS-065/NCNP-01 (Viltolarsen) Injection 250 mg" Composition of (250 mg in 10 mL, or equivalently, 25 mg/mL)

| **Component (USP = US Pharmacopoeia)** | **Function** | **Amount (per vial)** |
|---|---|---|
| NS-065/NCNP-01 (Viltolarsen) | Drug substance | 250 mg |
| Sodium chloride (USP quality) | Tonicity agent | 90 mg |
| Hydrochloric acid (USP quality) | pH adjuster | q.s.[1] |
| Sodium hydroxide (USP quality) | pH adjuster | q.s.[1] |
| Water for injection (USP quality) | Medium | q.s. |
| **Total amount** | | **10 mL** |
| [1]The "q.s." of hydrochloric acid and sodium hydroxide is an "amount sufficient" to adjust the pH value to pH 7.3, and at the same time, it is an amount that does not substantially affect the isotonicity of the composition. | | |

Besides, in the composition of "NS-065/NCNP-01 (Viltolarsen) Injection 250 mg," the amounts of the water for injection and sodium chloride used as a tonicity agent may be each set at approximately a half amount, so that the total amount is set at 5 mL. A pharmaceutical composition comprising Viltolarsen with the aforementioned composition in a

concentration of 50 mg/mL is also included in the present invention.

The volume of the aqueous solution containing Viltolarsen may be increased or decreased, as long as the concentrations of NS-065NCNP-01 and the used tonicity agent, and the weight ratio between Viltolarsen and the used tonicity agent, are maintained at the same levels as those described above.

The composition comprising Viltolarsen may also comprise a carrier for promoting the delivery of Viltolarsen into muscle tissues. Such a carrier is not particularly limited, as long as it is pharmaceutically acceptable carrier. Examples of such a carrier may include cationic carriers (e.g., cationic liposomes and cationic polymers) and carriers using viral envelope. Examples of the cationic liposomes may include liposomes comprising, as essential components, 2-O-(2-diethylaminoethyl)carbamoyl-1,3-O-dioleoylglycerol and phospholipid, such as Oligofectamine® (manufactured by Thermo Fisher Scientific), Lipofectin® (manufactured by Thermo Fisher Scientific), Lipofectamine® (manufactured by Thermo Fisher Scientific), Lipofectamine® 2000 (manufactured by Thermo Fisher Scientific), DMRIE-C (manufactured by Thermo Fisher Scientific), GeneSilencer® (manufactured by Gene Therapy Systems), TransMessenger® (manufactured by QIAGEN), and TransIT-TKO®. Examples of the cationic polymers may include JetSI® (manufactured by GeneX India Bioscience) and Jet-PEI® (polyethyleneimine, manufactured by GeneX India Bioscience). An example of the carriers using viral envelope may be GenomeOne® (HVJ-E liposome, manufactured by ISHIHARA SANGYO KAISHA, LTD.).

Furthermore, the pharmaceutical composition of the present invention may comprise an emulsification aid (e.g., fatty acid containing 6 to 22 carbon atoms or a pharmaceutically acceptable salt thereof, albumin, and dextran) and a stabilizer (e.g., cholesterol and phosphatidic acid).

In the pharmaceutical composition of the present invention comprising Viltolarsen and a carrier, the weight ratio between Viltolarsen and a carrier (i.e., carrier/Viltolarsen) may be changed depending on the type of a carrier used. The weight ratio is suitably in the range of 0.1 to 100, preferably in the range of 1 to 50, and more preferably in the range of 10 to 20.

The aqueous solution containing Viltolarsen can be administered to patients via intravenous drip infusion or drip infusion.

## 3. Indication/Effects and Administration/Dosage

When the pharmaceutical composition of the present invention is used in a treatment, the oligomer of the present invention, or a pharmaceutically acceptable salt thereof, or a hydrate thereof is intravenously administered to a human patient at a dose of between 40 mg/kg/week inclusive and 80 mg/kg/week inclusive (hereinafter referred to as "the dosage and administration method of the present invention"). Alternatively, the dosage and administration method of the present invention may be as follows: the oligomer of the present invention, or a pharmaceutically acceptable salt thereof, or a hydrate thereof is intravenously administered to a human patient at a dose of 40 mg/kg/week. Alternatively, the dosage and administration method of the present invention may be as follows: the oligomer of the present invention, or a pharmaceutically acceptable salt thereof, or a hydrate thereof is intravenously administered to a human patient at a dose of 80 mg/kg/week. Herein, the numerator "mg" in the dosage unit "mg/kg" indicates the amount of the oligomer of the present invention, or a pharmaceutically acceptable salt thereof, or a hydrate thereof, that is indicated with the unit "milligram," whereas the denominator "kg" indicates 1 kilogram of body weight of a human patient.

NS-065/NCNP-01 (Viltolarsen) Injection 250 mg has been developed for use in intravenous drip infusion that is performed once a week for purpose of the treatment of DMD patients amenable to a treatment involving exon 53 skipping. The US/Canada phase 2 clinical program has been designed to evaluate Viltolarsen administered at two types of doses, that are, at a dose of 40 mg/kg/week and at a dose of 80 mg/kg/week. Herein, kg is a unit indicating the body weight of a patient. For example, when Viltolarsen is administered at a dose of 40 mg/kg/week to a patient with a body weight of 40 kg, it means that 1,600 mg (= 40 mg x 40) of Viltolarsen is administered to the patient once a week.

## 4. Disease

DMD is a muscular disease caused by a loss-of-function mutation of a dystrophin gene, and this disease brings on a loss of a dystrophin protein in the muscle of patients (Hoffman et al., 1987). The dystrophin gene is present on the X chromosome, and shows a high spontaneous mutation rate. In all of the researched populations over the world, the incidence rate of DMD is one in 5,000 boys at surviving birth. According to the recent evaluation, 10.1% of 7,149 DMD patients evaluated in global patient database were shown to be amenable to a treatment involving exon 53 skipping (Bladen et al., 2015). The clinical symptoms are typically exhibited at young school age (by 4 to 6 of age), where affected boys show difficulty in keeping up physically with peers due to proximal muscle weakness (such as difficulty in climbing stairs, or in running). Difficulty in rising from the floor is seen with most patients in whom the typical Gower’s maneuver is used to achieve a standing position (namely, using hand support on the legs, knees, and thighs to achieve a standing position). (Hoffman EP, Brown RH, and Kunkel LM. (1987). Dystrophin: the protein product of the Duchenne muscular dystrophy locus. Cell 51, 919-928. and Bladen CL, Salgado D, Monges S, Foncuberta ME, Kekou K, Kosma K, Dawkins

H, Lamont L, Roy AJ, Chamova T, Guergueltcheva V, Chan S, Korngut L, Campbell C, Dai Y, Wang J, Barišić N, Brabec P, Lahdetie J, Walter MC, Schreiber-Katz O, Karcagi V, Garami M, Viswanathan V, Bayat F, Buccella F, Kimura E, Koeks Z, van den Bergen JC, Rodrigues M, Roxburgh R, Lusakowska A, Kostera-Pruszczyk A, Zimowski J, Santos R, Neagu E, Artemieva S, Rasic VM, Vojinovic D, Posada M, Bloetzer C, Jeannet PY, Joncourt F, Diaz-Manera J, Gallardo E, Karaduman AA, Topaloğlu H, El SherifR, Stringer A, Shatillo AV, Martin AS, Peay HL, Bellgard MI, Kirschner J, Flanigan KM, Straub V, Bushby K, Verschuuren J, Aartsma-Rus A, Béroud C, Lochmüller H. (2015). The TREAT-NMD DMD Global Database: analysis of more than 7,000 Duchenne muscular dystrophy mutations. Hum Mutat. 36(4): 395-402).

Muscle tissues of DMD patients show chronic inflammation, with bouts of muscle degeneration and regeneration leading to muscle wasting, disability, and early death. Patients typically lose ambulation in the second decade of life and require assistance with many aspects of daily living by the third decade. This disease usually leads to death in adolescence or early adulthood, although use of ventilator assistance devices can sometimes extend life into the fourth decade and very occasionally the fifth decade of life.

DMD gene mutation in a subject may be detected by the use of multiplex ligation-dependent probe amplification (MLPA) (Murugan et al., 2010). The content of this article by Murugan et al. is incorporated by reference in its entirety (Sakthivel Murugan S.M., Arthi Chandramohan and Bremadesam Raman Lakshmi, "Use of multiplex ligation-dependent probe amplification (MLPA) for Duchenne muscular dystrophy (DMD) gene mutation analysis," Indian Journal of Medical Research, Vol. 132, pp. 303-311 (September 2010)).

The human patient of interest of the treatment using the pharmaceutical composition of the present invention (hereinafter referred to as a "patient of interest of the present invention") is not particularly limited, as long as the patient is diagnosed to be affected with DMD by a medical doctor. In a certain aspect, the patient may have a mutation that results in deficiency in any exon selected from the group consisting of exons 43-52, 45-52, 47-52, 48-52, 49-52, 50-52, or 52, in a dystrophin gene.

Moreover, the patient of interest of the present invention may be characterized in that the expression of a dystrophin protein before the treatment using the pharmaceutical composition of the present invention or the oligomer of the present invention is 1% or less compared with that of a healthy subject (100%), as measured by Western blotting or mass spectrometry. Herein, the "healthy subject" is a human who does not have disease associated with a dystrophin protein. The expression level of a dystrophin protein in a healthy subject may be either a generally known value, or a value obtained from an individual healthy subject. Furthermore, the patient of interest of the present invention may also be characterized in that the expression of a dystrophin protein is not seen before the treatment using the pharmaceutical composition of the present invention or the oligomer of the present invention. The phrase "the expression of a dystrophin protein is not seen" means that the expression of a dystrophin protein is at almost the same expression level as that of a negative control by the measurement using Western blotting or mass spectrometry, or that the expression of a dystrophin protein is below the lower limit of detection. Further, Western blot and mass spectrometry are not particularly limited, as long as these are methods generally used in the present technical field. As examples of Western blot and mass spectrometry, the experimental methods applied in the present Examples can be referred to.

5. Therapeutic Rationale

DMD is a severe inherited muscle disorder. This disease occurs most commonly when out-of-frame amino acid translation is caused by a deletion of one or more exons from the dystrophin gene. A patient's functional dystrophin protein, which is important for muscle function, is not expressed due to such out-of-frame amino acid translation. A less severe form of the disorder, Becker muscular dystrophy (BMD), occurs most commonly when the absence of one or more exons in the dystrophin gene results in in-frame amino acid translation of the remaining exons. Patients with BMD generally have slower disease progression and a lower degree of disability. Medical treatment of DMD patients generally includes glucocorticoid treatment to delay the onset of symptoms in the muscles of the limbs or those that support respiration. There is a significant unmet medical need in DMD patients as expressed in the final February 2018 FDA guidance "Duchenne Muscular Dystrophy and Related Dystrophinopathies: Developing Drugs for Treatment Guidance for Industry" (available from https://www.fda.gov/downloads/Drugs/GuidanceComplianceRegulatoryInformation/Guidances /UCM450229.pdf.).

One therapeutic approach to treating DMD patients is to employ an "exon skipping" strategy in order to produce functional dystrophin protein that may make DMD patients transition to a BMD phenotype. Exon skipping allows for the restoration of the amino acid reading frame due to induced skipping of the exon next to the missing exon (Cirak et al., 2011; Voit et al., 2014; Yokota et al., 2012). With exon 53 skipping, a dystrophin protein is expressed that is slightly shorter than normal but retains partial functional activity. NS-065/NCNP-01 (Viltolarsen) Injection 250 mg is expected to shift the DMD phenotype to the milder disease of BMD in which patients' disease progression slows and patients' quality of life improves (Cirak S, Arechavala-Gomeza V, Guglieri M, Feng L, Torelli S, Anthony K, Abbs S, Garralda ME, Bourke J, Wells DJ, Dickson G, Wood MJ, Wilton SD, Straub V, Kole R, Shrewsbury SB, Sewry C, Morgan JE, Bushby

K, Muntoni F. (2011). Exon skipping and dystrophin restoration in patients with Duchenne muscular dystrophy after systemic phosphorodiamidate morpholino oligomer treatment: an open-label, phase 2, dose-escalation study. Lancet 378: 595-605., Voit T, Topaloglu H, Straub V, Muntoni F, Deconinck N, Campion G, De Kimpe SJ, Eagle M, Guglieri M, Hood S, Liefaard L, Lourbakos A, Morgan A, Nakielny J, Quarcoo N, Ricotti V, Rolfe K, Servais L, Wardell C, Wilson R, Wright P, Kraus JE. (2014). Safety and efficacy of drisapersen for the treatment of Duchenne muscular dystrophy (DEMAND II): an exploratory, randomised, placebo-controlled phase 2 study. Lancet Neurol. 13(10): 987-96., and Yokota T, Nakamura A, Nagata T, Saito T, Kobayashi M, Aoki Y, Echigoya Y, Partridge T, Hoffman EP, Takeda S. (2012). Extensive and Prolonged Restoration of Dystrophin Expression with Vivo-Morpholino-Mediated Multiple Exon Skipping in Dystrophic Dogs. Nucleic Acid Ther 22(5): 306-15.).

Therefore, the pharmaceutical composition of the present invention is administered to a human patient with DMD according to the aforementioned dosage and administration method of the present invention, so that DMD can be treated.

The term "treat" is used herein to mean to reduce the symptoms of DMD in a patient.

The treatment using the pharmaceutical composition of the present invention in accordance with the dosage and administration method of the present invention may provide at least one effect selected from the group consisting of the following effects (1) to (6) (wherein mean ± standard deviation is shown in parentheses):

(1) the average value of the expression level of a dystrophin protein in the skeletal muscle of the patient is 9-fold or more increased in comparison to baseline, after administration of the pharmaceutical composition for 24 weeks;
(2) a change in the velocity obtained from time to stand (TTSTAND) is -0.055 times/sec or more, or 0.024 ± 0.075 times/sec or more in comparison to baseline, at the time of the 25th week after administration of the pharmaceutical composition for 24 weeks;
(3) a change in the velocity obtained from time to run/walk 10 meters (TTRW) is -0.025 meters/sec or more, or 0.227 ± 0.251 meters/sec or more in comparison to baseline, at the time of the 25th week after administration of the pharmaceutical composition for 24 weeks;
(4) a change in the velocity obtained from time to climb 4 stairs (TTCLIMB) is -0.060 times/sec or more, or 0.032 ± 0.088 times/sec or more in comparison to baseline, at the time of the 25th week after administration of the pharmaceutical composition for 24 weeks;
(5) a change in the score of North Star Ambulatory Assessment (NSAA) is -2.2 scores or more, or 0.8 ± 2.9 scores or more in comparison to baseline, at the time of the 25th week after administration of the pharmaceutical composition for 24 weeks; and
(6) a change in 6-minute walk test (6MWT) is -7.5 meters or more, or 28.9 ± 36.3 meters or more in comparison to baseline, at the time of the 25th week after administration of the pharmaceutical composition for 24 weeks.

With regard to the above-described effects (1) to (6), the term "baseline" means an average value in an untreated patient group. In addition, the term "change" used in the effects (2) to (6) means a change in average values.

In a certain embodiment, when the pharmaceutical composition of the present invention is administered to 7- to 9-year-old human patients with Duchenne muscular dystrophy in accordance with the aforementioned dosage and administration method of the present invention for 84 weeks, at least one effect selected from the group consisting of the following effects (7) to (12) may be provided:

(7) the percentage of patients who lose rise ability is less than 20% by the time of the 85th week after initiation of the treatment;
(8) the percentage of patients who lose ability to climb 4 stairs is less than 10% by the time of the 85th week after initiation of the treatment;
(9) the percentage of patients who lose independent walking ability is less than 10% by the time of the 85th week after initiation of the treatment;
(10) a reduction in the velocity of running/walking 10 meters due to aging is not observed by the time of the 85th week after initiation of the treatment;
(11) a reduction in the velocity of climbing 4 stairs due to aging is not observed by the time of the 85th week after initiation of the treatment; and
(12) a reduction in rise velocity due to aging is not observed by the time of the 85th week after initiation of the treatment.

The week in which the treatment has been initiated is defined as a first week, and the above-described effects (7) to (12) may be provided at any time point from the 1st week to the 85th week, from the 1st week to the 80th week, from the 1st week to the 75th week, from the 1st week to the 70th week, from the 1st week to the 65th week, and from the 1st week to the 60th week.

In a certain embodiment, at least one effect selected from the group consisting of the following effects (13) to

(18) is provided by administering the pharmaceutical composition of the present invention to 10- to 12-year-old human patients with Duchenne muscular dystrophy in accordance with the aforementioned dosage and administration method of the present invention:

(13) the percentage of patients who lose rise ability is less than 60% by the time of the 85th week after initiation of the treatment;
(14) the percentage of patients who lose ability to climb 4 stairs is less than 50% by the time of the 85th week after initiation of the treatment;
(15) the percentage of patients who lose independent walking ability is less than 50% by the time of the 85th week after initiation of the treatment;
(16) a reduction in the velocity of running/walking 10 meters due to aging is not observed by the time of the 85th week after initiation of the treatment;
(17) a period in which the velocity of climbing 4 stairs increases is observed by the time of the 85th week after initiation of the treatment; and
(18) a period in which rise velocity increases is observed by the time of the 85th week after initiation of the treatment.

The week in which the treatment has been initiated is defined as a first week, and the above-described effects (13) to (18) may be provided at any time point from the 1st week to the 85th week, from the 1st week to the 80th week, from the 1st week to the 75th week, from the 1st week to the 70th week, from the 1st week to the 65th week, and from the 1st week to the 60th week.
That is to say, the pharmaceutical composition of the present invention may provide at least one of the above-described effects (1) to (18).

II. Second Embodiment

In a second embodiment, the present invention provides a method for treating Duchenne muscular dystrophy, comprising intravenously administering a pharmaceutical composition comprising an antisense oligomer consisting of a base sequence complementary to a sequence consisting of nucleotides at positions 36 to 56 from the 5'-terminus of exon 53 of a human dystrophin gene, or a pharmaceutically acceptable salt thereof, or a hydrate thereof, to a human patient once a week at a dose of between 40 mg/kg/week inclusive and 80 mg/kg/week inclusive of the antisense oligomer, or a pharmaceutically acceptable salt thereof, or a hydrate thereof (hereinafter referred to as "the treatment method of the present invention").
The meanings of individual configurations regarding the treatment method of the present invention are the same as those of configurations regarding the pharmaceutical composition of the present invention already explained in the section "I. First Embodiment."
Moreover, the treatment method of the present invention may provide at least one effect of the effects (1) to (18) explained in the sub-section "5. Therapeutic Rationale" in the section "I.

First Embodiment."

III. Third Embodiment

Furthermore, in a third embodiment, the present invention provides an antisense oligomer consisting of a base sequence complementary to a sequence consisting of nucleotides at positions 36 to 56 from the 5'-terminus of exon 53 of a human dystrophin gene, or a pharmaceutically acceptable salt thereof, or a hydrate thereof, for use in a method for treating a human patient with Duchenne muscular dystrophy, wherein
the antisense oligomer, or a pharmaceutically acceptable salt thereof, or a hydrate thereof is intravenously administered to the human patient once a week at a dose of between 40 mg/kg/week inclusive and 80 mg/kg/week inclusive (hereinafter referred to as "the use-limited embodiment of the present invention").
The meanings of individual configurations regarding the use-limited embodiment of the present invention are the same as those of configurations regarding the pharmaceutical composition of the present invention already explained in the section "I. First Embodiment."
Moreover, the use-limited embodiment of the present invention may provide at least one effect of the effects (1) to (18) explained in the sub-section "5. Therapeutic Rationale" of the section "I. First Embodiment."

IV. Fourth Embodiment

Further, in a fourth embodiment, the present invention provides use of an antisense oligomer consisting of a

base sequence complementary to a sequence consisting of nucleotides at positions 36 to 56 from the 5'-terminus of exon 53 of a human dystrophin gene, or a pharmaceutically acceptable salt thereof, or a hydrate thereof, for the manufacture of a pharmaceutical composition for treating a human patient with Duchenne muscular dystrophy, wherein the antisense oligomer, or a pharmaceutically acceptable salt thereof, or a hydrate thereof is intravenously administered to the human patient once a week at a dose of between 40 mg/kg/week inclusive and 80 mg/kg/week inclusive (hereinafter referred to as "the Swiss-type use of the present invention").

The meanings of individual configurations regarding the Swiss-type use of the present invention are the same as those of configurations regarding the pharmaceutical composition of the present invention already explained in the section "I. First Embodiment."

Moreover, the Swiss-type use of the present invention may provide at least one effect of the effects (1) to (18) explained in the sub-section "5. Therapeutic Rationale" of the section "I.

First Embodiment."

Hereinafter, the present invention will be described in more detail in the following examples. However, the present invention is not limited to the scope of the invention shown in these examples.

Examples

[Production Example]

(1) Production of 4-{[(2S,6R)-6-(4-benzamide-2-oxopyrimidin-1-yl)-4-tritylmorpholin-2-yl]methoxy}-4-oxobutanoic acid supported on aminomethyl polystyrene resin

Step 1: Production of 4-{[(2S,6R)-6-(4-benzamide-2-oxopyrimidin-1(2H)-yl)-4-tritylmorpholin-2-yl]methoxy}-4-oxobutanoic acid

Under an argon atmosphere, 22.0 g of N-{1-[(2R,6S)-6-(hydroxymethyl)-4-tritylmorpholin-2-yl]-2-oxo-1,2-dihydropyrimidin-4-yl}benzamide and 7.04 g of 4-dimethylaminopyridine (4-DMAP) were suspended in 269 mL of dichloromethane, and 5.76 g of succinic anhydride was then added to the suspension, and the mixture was then stirred at room temperature for 3 hours. Thereafter, 40 mL of methanol was added to the reaction solution, and the obtained solution was then concentrated under reduced pressure. The residue was subjected to an extraction operation using ethyl acetate and a 0.5 M potassium dihydrogen phosphate aqueous solution. The obtained organic layer was successively washed with a 0.5 M potassium dihydrogen phosphate aqueous solution, water, and a saturated saline. The obtained organic layer was dried over sodium sulfate, and was then concentrated under reduced pressure to obtain 25.9 g of a product of interest.

Step 2: Production of 4-{[(2S,6R)-6-(4-benzamide-2-oxopyrimidin-1-yl)-4-tritylmorpholin-2-yl]methoxy}-4-oxobutanoic acid supported on aminomethyl polystyrene resin

4-{[(2S,6R)-6-(4-Benzamide-2-oxopyrimidin-1(2H)-yl)-4-tritylmorpholin-2-yl]methoxy}-4-oxobutanoic acid (23.5 g) was dissolved in 336 mL of pyridine (dehydrated), and thereafter, 4.28 g of 4-DMAP and 40.3 g of 1-ethyl-3-(3-dimethylaminopropyl)carbodiimide hydrochloride were added to the solution. Subsequently, 25.0 g of Aminomethyl Polystyrene Resin cross-linked with 1% DVB (manufactured by Tokyo Chemical Industry Co., Ltd., A1543) and 24 mL of triethylamine were added to the mixture, and the thus obtained mixture was then shaken at room temperature for 4 days. After completion of the reaction, the resin was collected by filtration. The obtained resin was washed with pyridine, methanol, and dichloromethane in this order, and was then dried under reduced pressure. To the obtained resin, 150 mL of tetrahydrofuran (dehydrated), 15 mL of acetic anhydride, and 15 mL of 2,6-lutidine were added, and the obtained mixture was then shaken at room temperature for 2 hours. The resin was collected by filtration, and was then washed with pyridine, methanol, and dichloromethane in this order, followed by drying under reduced pressure, to obtain 33.7g of a product of interest.

With regard to the amount of the product of interest loaded, the molar amount of trityl per gram of resin was determined by measuring the UV absorbance at 409 nm according to a known method. The amount loaded on the resin was found to be 397.4 μmol/g.

UV measurement conditions

Apparatus: U-2910 (Hitachi, Ltd.)

Solvent: methanesulfonic acid

Wavelength: 265 nm

ε value: 45000

(2) Production of PMO No. 8

PMO No. 8 targets the sequence at positions "36 to 56" in exon 53, the group at the 5'-terminus thereof is "group (3)," and the sequence of a base portion thereof is as set forth in SEQ ID NO: 3.

4-{[(2S,6R)-6-(4-benzamide-2-oxopyrimidin-1(2H)-yl)-4-tritylmorpholin-2-yl]methoxy}-4-oxobutanoic acid (Reference Example 1) (2 g (800 μmol)) supported on an aminomethyl polystyrene resin was transferred into a reaction tank, and 30 mL of dichloromethane was then added thereto, followed by leaving the obtained mixture at rest for 30 minutes. Thereafter, the reaction mixture was further washed with 30 mL of dichloromethane twice, and the following synthetic cycles were initiated. A desired morpholino monomer compound was added in each cycle, so as to obtain the base sequence of the compound of interest.

[Table 3]

Table 3

| Step | Reagent | Amount (mL) | Time (min) |
|---|---|---|---|
| 1 | Deblock solution | 30 | 2.0 |
| 2 | Deblock solution | 30 | 2.0 |
| 3 | Deblock solution | 30 | 2.0 |
| 4 | Deblock solution | 30 | 2.0 |
| 5 | Deblock solution | 30 | 2.0 |
| 6 | Deblock solution | 30 | 2.0 |
| 7 | Neutralizing solution | 30 | 1.5 |
| 8 | Neutralizing solution | 30 | 1.5 |
| 9 | Neutralizing solution | 30 | 1.5 |
| 10 | Neutralizing solution | 30 | 1.5 |
| 11 | Neutralizing solution | 30 | 1.5 |
| 12 | Neutralizing solution | 30 | 1.5 |
| 13 | Dichloromethane | 30 | 0.5 |
| 14 | Dichloromethane | 30 | 0.5 |
| 15 | Dichloromethane | 30 | 0.5 |
| 16 | Coupling solution B | 20 | 0.5 |
| 17 | Coupling solution A | 6-11 | 90.0 |
| 18 | Dichloromethane | 30 | 0.5 |
| 19 | Dichloromethane | 30 | 0.5 |
| 20 | Dichloromethane | 30 | 0.5 |
| 21 | Capping solution | 30 | 3.0 |
| 22 | Capping solution | 30 | 3.0 |

(continued)

| Step | Reagent | Amount (mL) | Time (min) |
|---|---|---|---|
| 23 | Dichloromethane | 30 | 0.5 |
| 24 | Dichloromethane | 30 | 0.5 |
| 25 | Dichloromethane | 30 | 0.5 |

It is to be noted that a mixture of trifluoroacetic acid (2 equivalents) and triethylamine (1 equivalent) that was dissolved in a dichloromethane solution containing 1% (v/v) ethanol and 10% (v/v) 2,2,2-trifluoroethanol to result in 3% (w/v) was used as a deblock solution. As a neutralizing solution, N,N-diisopropylethylamine dissolved in a dichloromethane solution containing 25% (v/v) 2-propanol to result in 5% (v/v) was used. As a coupling solution A, a morpholino monomer compound dissolved in 1,3-dimethyl-2-imidazolidinone containing 10% (v/v) N,N-diisopropylethylamine to result in 0.15 M was used. As a coupling solution B, N,N-diisopropylethylamine dissolved in 1,3-dimethyl-2-imidazolidinone to result in 10% (v/v) was used. As a capping solution, a mixture of 20% (v/v) acetic anhydride and 30% (v/v) 2,6-lutidine that was dissolved in dichloromethane was used.

The thus synthesized PMO-supported aminomethyl polystyrene resin was recovered from the reaction vessel, and was then dried at room temperature under reduced pressure for 2 hours or more. The PMO that was supported on the dried aminomethyl polystyrene resin was placed in a reaction vessel, and 200 mL of 28% ammonia water-ethanol (1/4) was then added thereto, followed by stirring the obtained mixture at 55°C for 15 hours. Thereafter, the aminomethyl polystyrene resin was collected by filtration, and washing was then carried out with 50 mL of water-ethanol (1/4). The obtained filtrate was concentrated under reduced pressure. The obtained residue was dissolved in 100 mL of a mixed solvent (4/1) of 20 mM acetic acid-triethylamine buffer (TEAA buffer) and acetonitrile, and the obtained solution was then filtrated through a membrane filter. The obtained filtrate was purified by reserve phase HPLC. The applied conditions are as follows.

[Table 4]

Table 4

| | |
|---|---|
| Column | XTerra MS 18 (Waters, ϕ 50 x 100 mm, 1 CV = 200 mL) |
| Flow rate | 60 mL/min |
| Column temperature | Room temperature |
| Solution A | 20 mM TEAA buffer |
| Solution B | $CH_3CN$ |
| Gradient | (B) conc. 20 →- 50%/9 CV |

Each fraction was analyzed, and a product of interest was recovered using 100 mL of acetonitrile-water (1/1). 200 mL of ethanol was added thereto, and the obtained mixture was then concentrated under reduced pressure. The resultant was further dried under reduced pressure to obtain a white solid. To the obtained solid, 300 mL of a 10 mM phosphoric acid aqueous solution was added, so that the solid was suspended therein. Thereafter, 10 mL of a 2 M phosphoric acid aqueous solution was added to the suspension, and the obtained mixture was then stirred for 15 minutes. Then, 15 mL of a 2 M sodium hydroxide aqueous solution was further added to the reaction mixture for neutralization. Then, 15 mL of a 2 M sodium hydroxide aqueous solution was further added to the reaction mixture to make the mixture alkaline, and the mixture was then filtrated through a membrane filter (0.45 μm). The resultant was fully washed with 100 mL of a 10 mM sodium hydroxide aqueous solution, so as to obtain a product of interest in the form of an aqueous solution.

The obtained aqueous solution containing the product of interest was purified with an anion exchange resin column. The applied conditions are as follows.

[Table 5]

Table 5

| | |
|---|---|
| Column | Source 30Q (GE Healthcare, ϕ 40 x 150 mm, 1 CV = 200 mL) |
| Flow rate | 80 mL/min |
| Column temperature | Room temperature |
| Solution A | 10 mM sodium hydroxide aqueous solution |
| Solution B | 10 mM sodium hydroxide aqueous solution, 1 mM sodium chloride aqueous solution |
| Gradient | (B) conc. 5 →_ 35%/15 CV |

Each fraction was analyzed (HPLC), and a product of interest was obtained in the form of an aqueous solution. To the obtained aqueous solution, 225 mL of 0.1 M phosphate buffer (pH 6.0) was added for neutralization. The obtained mixture was filtrated through a membrane filter (0.45 μm). Subsequently, ultrafiltration was carried out under the following conditions for desalination.

[Table 6]

Table 6

| | |
|---|---|
| Filter | PELLICON2 MINI FILTER PLBC 3K Regenerated Cellulose, Screen Type C |
| Size | 0.1 $m^2$ |

The filtrate was concentrated to obtain approximately 250 mL of an aqueous solution. The obtained aqueous solution was filtrated through a membrane filter (0.45 μm). The obtained aqueous solution was freeze-dried to obtain 1.5 g of a compound of interest in the form of a white flocculent solid.

ESI-TOF-MS calculated value: 6924.82

Measured value: 6923.54

[Example 1] US Phase 2 Study

The US phase 2 dose-finding study, "Study NS-065/NCNP-01-201" was initiated on December 2016, under "IND127474." This study was carried out under ClinicalTrials.gov recognition No. "NCT02740972" with the title of "Safety and Dose Finding Study of NS-065/NCNP-01 in Boys With Duchenne Muscular Dystrophy (DMD)." The study implementation protocols of the present study are available from https://www.clinicaltrials.gov/ct2/show/study/NCT02740972, and the contents thereof are incorporated in the present description by reference in its entirety. The present study was mainly directed towards evaluating the safety of NS-065/NCNP-01 (Viltolarsen) to be delivered in the form of an intravenous drip infusion at a high dose (80 mg/kg) and at a low dose (40 mg/kg) to patients with Duchenne muscular dystrophy (DMD) amenable to a treatment involving exon 53 skipping. Moreover, further purposes of the present study include evaluation of tolerability, muscular function and muscular strength, pharmacokinetics, and pharmacodynamics.

More specifically, the present study was a Phase 2, multiple-center, two-period, randomized, placebo-controlled, dose-finding study, and NS-065/NCNP-01 (Viltolarsen) was administered by drip infusion once a week for 24 weeks to ambulant boys of ages of 4 years or older and less than 10 years with DMD. Two dose level cohorts were enrolled. Period 1 of the study was conducted in a double-blind fashion. Randomized patients received weekly intravenous drip infusions of NS-065/NCNP-01 (Viltolarsen) or placebo for the first 4 weeks of their participation (Period 1), and then, intravenous drip infusion of NS-065/NCNP-01 (Viltolarsen) for 5 to 24 weeks (20 weeks of active treatment - Period 2). Analysis of safety data from Period 1 of the 40 mg/kg dose cohort was completed prior to enrolling patients in the 80 mg/kg dose cohort. Patients completing the 24-week study were eligible for an open-label extension study.

Clinical efficacy was assessed at regularly scheduled study visits. All patients underwent a muscle biopsy of the bicep at baseline and a second muscle biopsy at Week 24.

Safety was assessed through the collection of adverse events (AEs), blood and urine laboratory tests, electrocardiograms (ECGs), vital signs, and physical examinations throughout the study. Serial blood samples were taken at

four of the study visits to assess the pharmacokinetics of NS-065/NCNP-01 (Viltolarsen).

< Study Type: Interventional (Clinical Trial) >

- Actual Enrollment: 16 participants.
- Allocation: Randomized.
- Intervention Model: Parallel Assignment.
- Masking: Quadruple (Participant, Care Provider, Investigator, Outcomes Assessor).
- Primary Purpose: Treatment.
- Actual Study Start Date: December 2016.
- Primary Completion Date: March 2018.

< Patient Groups and Interventions >

- Experimental: NS-065/NCNP-01 (Viltolarsen) 40 mg/kg

Six patients with confirmed DMD with genetic deletions amenable to a treatment involving exon 53 skipping were administered an intravenous drip infusion of NS-065/NCNP-01 (Viltolarsen) at 40 mg/kg dose once a week for 24 weeks.

- Experimental: NS-065/NCNP-01 (Viltolarsen) 80 mg/kg

Six patients with confirmed DMD with genetic deletions amenable to a treatment involving exon 53 skipping were administered an intravenous drip infusion of NS-065/NCNP-01 (Viltolarsen) at 80 mg/kg dose once a week for 24 weeks.

- Placebo Comparator: Placebo

Two or three patients in each of the dose groups were administered placebo as an intravenous drip infusion once a week for 4 weeks followed by 20 weeks of open label treatment.

< Inclusion Criteria >

- Male $\geq$ 4 years and < 10 years of age.
- Confirmed DMD mutation(s) in the dystrophin gene that is amenable to a treatment involving skipping of exon 53 to restore the dystrophin mRNA reading frame.
- Able to walk independently without assistive devices.
- Patients with ability to complete the time to stand, time to run/walk, and time to climb assessments.
- Stable dose of glucocorticoid for at least 3 months.

< Exclusion Criteria >

- Acute illness within 4 weeks prior to the first dose of study medication.
- Evidence of symptomatic cardiomyopathy. (Asymptomatic cardiac abnormality on investigation would not be exclusionary.)
- Severe allergy or hypersensitivity to medications.
- Severe behavioral or cognitive problems that preclude participation in the study, in the opinion of the Investigator.
- Previous or ongoing medical condition, medical history, physical findings or laboratory abnormalities that could affect safety, make it unlikely that treatment and follow-up would be correctly completed, or impair the assessment of study results, in the opinion of the Investigator.
- Patient was taking any other investigational drug currently or within 3 months prior to the start of study treatment.
- Patient had had surgery within the 3 months prior to the first anticipated administration of NS-065/NCNP-01 (Viltolarsen) or surgery was planned for anytime during the duration of the study.

- Patient had previously participated in this study or any other study during which NS-065/NCNP-01 (Viltolarsen) had been administered.

To recap, the study was a 24-week, 2-cohort study testing 40 mg/kg/week and 80 mg/kg/week doses in 16 male patients amenable to a treatment involving exon 53 skipping and on stable glucocorticoid dose for over 3 months. Placebo group patients in each cohort received an initial 4-week randomization period as a control for adverse events (safety outcomes). Thereafter, both placebo- and active-treated patients continued the study for a 20-week treatment period. Trial NS-065/NCNP-01-201 evaluated the effect of NS-065/NCNP-01 (Viltolarsen) Injection on *de novo* expression of dystrophin protein following 20-24 weeks of administration in two dose cohorts: 40 mg/kg/week low dose and 80 mg/kg/week high dose (as shown in Figure 1). The goal of Trial NS-065/NCNP-01-201 was to identify a safe and effective dose based on *de novo* expression of dystrophin protein in skeletal muscle measured using Western blot (WB) methodology (primary surrogate endpoint). Secondary surrogate endpoints included immunofluorescence (IF) staining and mass spectrometry detection of *de novo* expression of dystrophin protein, as well as RT-PCR detection of *de novo* dystrophin mRNA levels. Several functional endpoints were also included as secondary endpoints in the Phase 2 study. Trial NS-065/NCNP-01-201 endpoints were:

< Primary Endpoints >

- Safety and tolerability of low dose (40 mg/kg/week) and high dose (80 mg/kg/week) intravenous (IV) administrations of NS-065/NCNP-01 (Viltolarsen) Injection.
- Effects of low and high IV doses of NS-065/NCNP-01 (Viltolarsen) Injection were determined as an induction of dystrophin protein in muscle after 20-24 weeks of treatment measured by Western blot.

In Figure 1, the timeline for "High Dose: 80 mg/kg/week" presented in the lower half is offset to a later time from the timeline for "Low Dose: 40 mg/kg/week" presented in the upper half to indicate that the high-dose administration was commenced only after safety was confirmed with the low-dose administration.

Measurement Contents:

1. Induction of dystrophin mRNA in muscle as measured by real time polymerase chain reaction (RT-PCR) for mRNA analysis. (Time Frame: 20-24 weeks of treatment)

RT-PCR measures altered splicing of the dystrophin RNA. In this method, RNA is isolated from the frozen muscle biopsy section, and is reverse-transcribed to cDNA. PCR primers are designed flanking the exon 53 site on the dystrophin mRNA. RT-PCR bands corresponding to specific versions of the spliced dystrophin mRNA are visualized by gel electrophoresis, and the amount of different mRNA isoforms are compared. If the drug successfully binds to the RNA target, then exon 53 is excluded from the resulting mRNA transcripts.

2. Induction of dystrophin protein in muscle as measured by western blot for protein analysis. (Time Frame: 20-24 weeks of treatment)

The primary biochemical outcome measure is measurement of drug-induced increase in dystrophin production by Immunoblot (Western blot). Dystrophin immunoblot uses solubilized muscle biopsy cryosections, with proteins fractionated by molecular weight using gel electrophoresis (SDS-PAGE), electroblotting to nitrocellulose, then incubation of nitrocellulose with antibodies to detect dystrophin protein. The immunoblot signal for dystrophin from a patient's biopsy is then compared to the signal of a standard curve of dystrophin on the same gel (mixed DMD and normal controls). This provides a semi-quantitative assessment of dystrophin content in the muscle.

US Phase 2 Study: EFFICACY

Outcome Measure 1

The results obtained from Outcome Measure 1 (i.e., RT-PCR detection of *de novo* dystrophin mRNA levels) were as follows.

[Table 7]

Table 7

| Dose | Baseline mean % (standard deviation) | On-treatment mean % (standard deviation) |
|---|---|---|
| 40 mg/kg/week | 0.0 (0.0) | 17.4 (7.2) |
| 80 mg/kg/week | 0.0 (0.0) | 43.9 (16.7) |

Outcome Measure 2

The results obtained from Outcome Measure 2 (i.e., measurement of *de novo* expression of dystrophin protein in skeletal muscle using Western blot methodology) represented a 19.0-fold increase (for 40 mg/kg/week for 24 weeks) and a 9.8-fold increase (for 80 mg/kg/week for 24 weeks) from baseline as compared between an average value of baseline and that of on-treatment, and a 27.2-fold increase (for each of 40 and 80 mg/kg/week for 24 weeks) which was calculated as an average value of the increase rate for each patient. The data obtained are summarized in the following Table 8 and Figure 2.

[Table 8]

Table 8

| Dose | Baseline mean % (range, standard deviation) | On-treatment mean % (range, standard deviation) | Fold increase 1) | Fold increase 2) |
|---|---|---|---|---|
| 40mg/kg/ week | 0.3 (0.1-0.4, 0.1) | 5.7 (3.2-10.3,2.4) | 19.0 | 27.2 |
| 80mg/kg/ week | 0.6 (0.1-2.6, 0.8) | 5.9 (1.1-14.4,4.5) | 9.8 | 27.2 |

1) Comparison between average value of baseline and that of on-treatment
2) Average value of increase rate of each patient

The degree of dystrophin rescue by NS-065/NCNP-01 (Viltolarsen) (40 or 80 mg/kg/week, 24 weeks) was approximately 3- to 7-fold or 8.8 to 9.7-fold higher than previously reported for Exondys 51® (eteplirsen) (30 mg/kg/week, 48 and 180 weeks) amenable to a treatment involving exon 51 skipping at a moderate estimate, which was launched by Sarepta Therapeutics in 2016. Specifically, for comparison purposes, the following are the corresponding Western blot data for Exondys 51® (eteplirsen). In 3 out of 16 patients who were administered NS-065/NCNP-01 (Viltolarsen) for 24 weeks, dystrophin level increased by more than 10% from baseline. An increase in the dystrophin value of 3% or more was seen in 12 out of 16 patients who were administered NS-065/NCNP-01 (Viltolarsen) for 24 weeks. Hoffman et al. have reported that: "Among the patients with Duchenne's (< 3% of dystrophin of normal level) or Becker's dystrophy and an abnormal dystrophin phenotype, there was a clear correlation between the severity of the clinical phenotype and the results of the dystrophin assessment." (Eric P. Hoffman, et al., "Characterization of Dystrophin in Muscle-Biopsy Specimens from Patients with Duchenne's or Becker's Muscular Dystrophy." N. Engl. J. Med., 318: 1363-1368 (1988))
- eteplirsen Western blotting: a 2.8-fold increase, 0.16% → 0.44 % (30 mg/kg/week for 48 weeks). ("In the 12 patients with evaluable results, the pre-treatment dystrophin level was 0.16% ± 0.12% (mean ± standard deviation) of the dystrophin level in a healthy subject and 0.44% ± 0.43% after 48 weeks of treatment with EXONDYS 51 ($p < 0.05$).") (available from https://www.fda.gov/downloads/AdvisoryCommittees/CommitteesMeetingMaterials/Pediatric AdvisoryCommittee/UCM557917.pdf.)
- eteplirsen Western blotting: a 3.1-fold increase, 0.3% → 0.93% (30 mg/kg/week for 180 weeks). ("In Western blots from biopsies of extensor digitorum brevis (EDB), dystrophin levels averaged about 0.3% of normal, but ranged

from undetectable to ≈ 1% of normal or somewhat higher." "By Western blot, the most accurate quantitative method used by the applicant, the mean dystrophin level after ~3.5 years of eteplirsen treatment was 0.93% ± 0.84% of normal (mean ± standard deviation).") (available from https://www.fda.gov/downloads/advisorycommittees/committeesmeetingmaterials/drugs/periph eralandcentralnervoussystemdrugsadvisorycommittee/ucm497063. pdf.)

- eteplirsen Western blotting: 0.93% at week 180, range 0% - 2.47% (30 mg/kg/week for 180 weeks) (Kenji Rowel Q Lim, et al., "Eteplirsen in the treatment of Duchenne muscular dystrophy." Drug Des. Devel. Ther., 11: 533-545 (2017); https://www.ncbi.nlm.nih.gov/pmc/articles/PMC5338848/)
- In addition: "For eteplirsen, no difference in the dose-response for the amount of dystrophin produced between a 30 mg/kg and 50mg/kg weekly dose was seen in the conducted studies at Week 180, which suggests that this approach may not be successful for other exons." (Kenji Rowel Q Lim, et al., "Eteplirsen in the treatment of Duchenne muscular dystrophy." Drug Des. Devel. Ther., 11: 533-545 (2017); https://www.ncbi.nlm.nih.gov/pmc/articles/PMC5338848/)

It is also shown that Viltolarsen achieved the superior effects compared with SRP-4053 (golodirsen; by Sarepta), which is a morpholino oligonucleotide for DMD patients amenable to a treatment involving exon 53 skipping, in particular in the increased amount of dystrophin level observed. Specifically, for comparison purposes, the following is the corresponding Western blot data for SRP-4053 (golodirsen), which were obtained after 48 weeks of administration rather than 24 weeks of administration for Viltolarsen.

- golodirsen Western blot: 0.095% → 1.019% (30 mg/kg for 48 weeks). ("Mean dystrophin protein increased to 1.019% of normal compared to a mean baseline of 0.095% of normal (p < 0.001) as measured by Western blot, the primary biological endpoint in the study, representing a 10.7-fold increase from baseline.") (available from http://investorrelations.sarepta.com/news-releases/news-release-details/sarepta-therapeutics-announces-positive-results-its-study.)
- golodirsen Western blot: Min 0.09%, Max 4.30% (30 mg/kg/week for 48 weeks). An increase in the dystrophin level of 3% or more was seen in 2 out of 25 patients (22nd International Congress of the World Muscle Society (3-7 October 2017, St. Malo, France), (http://www.google.co.jp/url?sa=t&source=web&cd=2&ved=2ahUKEwid7ZTQ9OrbAhUETL wKHX3xDk8QFjABegQIARAB&url=http%3A%2F%2Finvestorrelations.sarepta.com%2Fsta tic-files%2F64d8d897-2e4a-4119-80b4-115cbae17993&usg=AOvVaw1Amh1Mq1VauvLkPvYWfVdR).

US Phase 2 Study: SAFETY

As for safety data from 16 DMD patients treated with high and low doses of NS-065/NCNP-01 (Viltolarsen) (40 mg/kg/week and 80 mg/kg/week), no major concerns were raised in the study execution, data quality, or participant safety. No participant discontinued treatment at the time of 36 weeks. Specifically, there were no serious adverse events, no adverse events leading to discontinuation, and no drug-related adverse events. All adverse events (AEs) were mild or moderate.

[Example 2] US/Canada Phase 2 Study

The US phase 2 dose-finding study, "Study NS-065/NCNP-01-201" was initiated on December 2016, under "IND127474." This study was carried out under ClinicalTrials.gov recognition No. "NCT02740972" with the title of "Safety and Dose Finding Study of NS-065/NCNP-01 in Boys With Duchenne Muscular Dystrophy (DMD)." The clinical protocol of the present study is available from https://www.clinicaltrials.gov/ct2/show/study/NCT02740972, and the contents thereof are incorporated in the present description by reference in its entirety. The present study was mainly directed towards evaluating the safety of NS-065/NCNP-01 (Viltolarsen) to be delivered in the form of an intravenous drip infusion at a high dose (80 mg/kg) and at a low dose (40 mg/kg) to patients with Duchenne muscular dystrophy (DMD) amenable to a treatment involving exon 53 skipping. Moreover, further purposes of the present study include evaluation of tolerability, muscular function and muscular strength, pharmacokinetics, and pharmacodynamics.

More specifically, the present study was a Phase 2, multiple-center, two-period, randomized, placebo-controlled, dose-finding study, and NS-065/NCNP-01 (Viltolarsen) was administered by drip infusion once a week for 24 weeks to ambulant boys of ages of 4 years or older and less than 10 years with DMD. Two dose level cohorts were enrolled. Period 1 of the study was conducted in a double-blind fashion. Randomized patients received weekly intravenous drip infusions of NS-065/NCNP-01 (Viltolarsen) or placebo for the first 4 weeks of their participation (Period 1), and then, intravenous drip infusion of NS-065/NCNP-01 (Viltolarsen) for 5 to 24 weeks (20 weeks of active treatment - Period 2). Analysis of safety data from Period 1 of the 40 mg/kg dose cohort was completed prior to enrolling patients in the 80

mg/kg dose cohort. Patients completing the 24-week study were eligible for an open-label extension study.

Clinical efficacy was assessed at regularly scheduled study visits. All patients underwent a muscle biopsy of the bicep at baseline and a second muscle biopsy at Week 24.

Safety was assessed through the collection of adverse events (AEs), blood and urine laboratory tests, electrocardiograms (ECGs), vital signs, and physical examinations throughout the study. Serial blood samples were taken at four of the study visits to assess the pharmacokinetics of NS-065/NCNP-01 (Viltolarsen).

< Study Type: Interventional (Clinical Trial) >

- Actual Enrollment: 16 participants.
- Allocation: Randomized.
- Intervention Model: Parallel Assignment.
- Masking: Quadruple (Participant, Care Provider, Investigator, Outcomes Assessor).
- Primary Purpose: Treatment.
- Actual Study Start Date: December 2016.
- Primary Completion Date: March 2018.

< Patient Groups and Interventions >

- Experimental: NS-065/NCNP-01 (Viltolarsen) 40 mg/kg

Six patients with confirmed DMD with genetic deletions amenable to a treatment involving exon 53 skipping were administered an intravenous drip infusion of NS-065/NCNP-01 (Viltolarsen) at 40 mg/kg dose once a week for 24 weeks.

- Experimental: NS-065/NCNP-01 (Viltolarsen) 80 mg/kg

Five patients with confirmed DMD with genetic deletions amenable to a treatment involving exon 53 skipping were administered an intravenous drip infusion of NS-065/NCNP-01 (Viltolarsen) at 80 mg/kg dose once a week for 24 weeks.

- Placebo Comparator: Placebo

Two patients in the 40 mg/kg dose group and three patients in the 80 mg/kg dose group were each administered placebo as an intravenous drip infusion once a week for 4 weeks followed by 20 weeks of open label treatment.

< Inclusion Criteria >

- Male ≥ 4 years of age at the time of consent and < 10 years of age at the time of first intravenous drip infusion.
- Confirmed DMD mutation(s) in the dystrophin gene that is amenable to a treatment involving skipping of exon 53 to restore the dystrophin mRNA reading frame.
- Able to walk independently without assistive devices.
- Patients with ability to participate in the assessments of the time to stand (TSTAND), the time to run/walk 10 meters (TTRW), and the time to climb 4 stairs (TTCLIMB), which were judged by the motor function evaluator.
- Stable dose of glucocorticoid for at least 3 months.

< Exclusion Criteria >

- Acute illness within 4 weeks prior to the first dose of study medication.
- Evidence of symptomatic cardiomyopathy. (Asymptomatic cardiac abnormality on investigation are not exclusionary.)
- Severe allergy or hypersensitivity to medications.
- Severe behavioral or cognitive problems that preclude participation in the study, in the opinion of the Investigator.

- Previous or ongoing medical condition, medical history, physical findings or laboratory abnormalities that could affect safety, make it unlikely that treatment and follow-up would be correctly completed, or impair the assessment of study results, in the opinion of the Investigator.
- Patient was taking any other investigational drug currently or within 3 months prior to the start of study treatment.
- Patient had had surgery within the 3 months prior to the first anticipated administration of NS-065/NCNP-01 (Viltolarsen) or surgery was planned for anytime during the duration of the study.
- Patient had previously participated in this study or any other study during which NS-065/NCNP-01 (Viltolarsen) had been administered.

To recap, the study was a 24-week, 2-cohort study testing 40 mg/kg/week and 80 mg/kg/week doses in 16 male patients amenable to a treatment involving exon 53 skipping and on stable glucocorticoid dose for over 3 months. Placebo group patients in each cohort received an initial 4-week randomization period as a control for adverse events (safety outcomes). Thereafter, both placebo- and active-treated patients continued the study for a 20-week treatment period. Study NS-065/NCNP-01-201 evaluated the effect of NS-065/NCNP-01 (Viltolarsen) Injection 250 mg on *de novo* expression of dystrophin protein following 20-24 weeks of administration in two dose cohorts: 40 mg/kg/week low dose and 80 mg/kg/week high dose (as shown in Figure 3). The goal of Study NS-065/NCNP-01 -201 was to identify a safe and effective dose based on *de novo* expression of dystrophin protein in skeletal muscle measured using Western blot (WB) methodology (primary surrogate endpoint). Secondary surrogate endpoints included immunofluorescence (IF) staining and mass spectrometry detection of *de novo* expression of dystrophin protein, as well as RT-PCR detection of *de novo* dystrophin mRNA levels. Several functional endpoints were also included as secondary endpoints in the Phase 2 study. Study NS-065/NCNP-01-201 endpoints were:

< Primary Endpoints >

- Safety and tolerability of low dose (40 mg/kg/week) and high dose (80 mg/kg/week) intravenous (IV) administrations of NS-065/NCNP-01 (Viltolarsen) Injection 250 mg.
- Effects of low and high IV doses of NS-065/NCNP-01 (Viltolarsen) Injection 250 mg on induction of dystrophin protein in muscle after 20-24 weeks of treatment measured by Western blot.
- Blood drug concentration of NS-065/NCNP-01 (Viltolarsen)

< Secondary Endpoints >

- To evaluate the effects of low dose and high dose IV administrations of NS-065/NCNP-01 (Viltolarsen) Injection 250 mg on induction of dystrophin mRNA and protein in muscle after 20-24 weeks of treatment as measured by RT-PCR for mRNA analysis and immunofluorescence staining and mass spectrometry methods for protein analysis.
- To investigate the effects of low dose and high dose IV administrations of NS-065/NCNP-01 (Viltolarsen) Injection 250 mg after 20-24 weeks of treatment on muscle strength, mobility, and functional exercise capacity, as measured by Time to Stand (TTSTAND), Time to Run/Walk 10 meters (TTRW), Time to Climb 4 stairs (TTCLIMB), North Star Ambulatory Assessment (NSAA), 6-Minute Walk Test (6MWT), and Quantitative Muscle Testing (QMT) versus a matched natural history control group.

In Figure 3, the timeline for "High Dose: 80 mg/kg/week" presented in the lower half is offset to a later time from the timeline for "Low Dose: 40 mg/kg/week" presented in the upper half to indicate that the high-dose administration was commenced only after safety was confirmed with the low-dose administration.

US/Canada Phase 2 Study secondary endpoints: Timed function tests versus external untreated, natural history comparator (Cooperative International Neuromuscular Research Group (CINRG) Duchenne Natural History Study (DNHS))

Patients enrolled in the US/Canada Phase 2 Study NS-065/NCNP-01-201 were clinically evaluated for muscle function at baseline, at Week 13, and at Week 25. Adjustment was not made for the four-week placebo period for this purpose. These evaluations included several types of timed function tests: Time to stand from supine (TTSTAND); Time to run/walk 10 meters (TTRW); Time to climb four stairs (TTCLIMB); 6-minute walk test (6MWT); and North Star Ambulatory Assessment (NSAA). Changes over time were compared to disease trajectories in matched patients studied in CINRG DNHS.

CINRG DNHS is a study in which each of 440 DMD patients was followed over a period of years (with the total

duration of the study about 10 years). CINRG stands for Cooperative International Neuromuscular Research Group, and "is a consortium of medical and scientific investigators from academic and research centers who share the common goal of wanting to positively impact the lives of neuromuscular disease patients and their families by conducting well-controlled clinical studies" (from http://www.cinrgresearch.org/). DNHS stands for Duchenne Natural History Study, and is "the largest prospective multicenter natural history study to date in Duchenne muscular dystrophy (DMD)" established by CINRG (from http://www.cinrgresearch.org/duchenne-natural-history/). (McDonald CM, Henricson EK, Abresch RT, Han JJ, Escolar DM, Florence JM, Duong T, Arrieta A, Clemens PR, Hoffman EP, and Cnaan A, "CINRG Investigators. The cooperative international neuromuscular research group Duchenne natural history study - a longitudinal investigation in the era of glucocorticoid therapy: design of protocol and the methods used," Muscle Nerve., 48(1), 32-54 (2013)., Henricson EK, Abresch RT, Cnaan A, Hu F, Duong T, Arrieta A, Han J, Escolar DM, Florence JM, Clemens PR, Hoffman EP, and McDonald CM, "CINRG Investigators. The cooperative international neuromuscular research group Duchenne natural history study: glucocorticoid treatment preserves clinically meaningful functional milestones and reduces rate of disease progression as measured by manual muscle testing and other commonly used clinical trial outcome measures," Muscle Nerve., 48(1), 55-67 (2013). and McDonald CM, Henricson EK, Abresch RT, Duong T, Joyce NC, Hu F, Clemens PR, Hoffman EP, Cnaan A, and Gordish-Dressman H, "CINRG Investigators. Long-term effects of glucocorticoids on function, quality of life, and survival in patients with Duchenne muscular dystrophy: a prospective cohort study," Lancet, 391(10119), 451-461 (2018)).

The NS-065/NCNP-01-201 trial was carried out by clinical sites participating in the CINRG network. The standard operating procedures (clinical manuals) and clinical evaluator training protocols were very similar for the NS-065/NCNP-01-201 clinical trial and the CINRG DNHS. Matching of patients enrolled in NS-065/NCNP-01-201 versus CINRG DNHS was done using the following set of criteria.

- Boys between age 4 and < 10.5 years old at baseline.
- Patients with at least 12 months of timed function test data.
- Geographic region: North America (US and Canada).
- On steroids for at least 3 months and continuous steroid use throughout the 12/24 month observation period.
- Cannot be a participant in another clinical trial.

Query of the CINRG DNHS database for patients matched to the patients enrolled in the NS-065/NCNP-01-201 clinical trial, without specifying a dystrophin mutation amenable to a treatment involving exon 53 skipping, but excluding those with an exon 3-7 deletion and those with a dystrophin deletion amenable to a treatment involving exon 44 skipping, resulted in 69 subjects as shown in Table 9 below. Those with an exon 3-7 deletion and those with a dystrophin deletion amenable to a treatment involving exon 44 skipping were excluded because these patients have been reported to exhibit comparatively mild symptoms.

[Table 9]

Table 9: Patients matched from CINRG DNHS

| Parameter | Exon 53 Skip n=6 | Non-Exon 53 Skip n = 63 | Total n = 69 |
|---|---|---|---|
| Mean age, years (range) | 6.8 (4.5 - 10.3) | 7.4 (4.0 - 10.5) | 7.3 (4.0 - 10.5) |
| Mean weight, kg (range) | 25.0 (16.6 - 39.1) | 25.6 (15.1 - 50.6) | 25.6 (15.1 - 50.6) |
| Deletion type, n (%) | | | |
| Single deletion | 2 (33%) | 9(14%) | 11(16%) |
| Multiple deletions | 4 (67%) | 31(49%) | 35(51%) |
| Single exon duplication | 0 | 2 (3%) | 2(3%) |
| Multiple exon duplications | 0 | 3(5%) | 3 (4%) |
| No large del/dup | 0 | 16 (25%) | 16 (23%) |
| * Non-exon 53 skip: Two patients did not have data regarding deletion type. | | | |

In the top row of Table 9, "Exon 53 Skip" means DMD patients amenable to a treatment involving exon 53 skipping, whereas "Non-Exon 53 Skip" means all other patients (but with the exclusions mentioned above). In the bottom row of Table 9, "No large del/dup" encompasses such other categories as point mutations (which are not amenable to a treatment involving exon skipping).

The number of CINRG DNHS patients who had data for 6MWT was less than the numbers of patients for the other outcomes. This is because 6MWT was added late in the CINRG DNHS protocol, leading to a limited amount of data relative to the other timed function tests.

Comparison of disease trajectories over 24 weeks between the 16 patients enrolled in NS-065/NCNP-01-201 and the 69 patients enrolled in CINRG DNHS showed that patients in the natural history comparator had declines in the performances of timed function tests over the 24-week time frame. In contrast, patients enrolled in NS-065/NCNP-01-201 showed an average improvement in timed function tests over the same time period of 24 weeks. Three of these improvements reached statistical significance: TTRW (at Week 13 and at Week 25); TTSTAND (at Week 25); and 6MWT (at Week 25). No statistically significant difference was observed between the doses of 40 mg/kg/week and 80 mg/kg/week of NS-065/NCNP-01 (Viltolarsen).

Figure 4 (a total of five graphs and one table) shows comparisons of changes from baseline in timed function tests over a 24-week period in patients enrolled in NS-065/NCNP-01-201 (indicated as "Viltolarsen" in blue solid line) and matched patients in CINRG DNHS (indicated as "DNHS" in red broken line).

In the five graphs of Figure 4, "Viltolarsen" is the international nonproprietary name (INN) of NS-065/NCNP-01. Also, in all of the graphs, changes from baseline were compared between the Viltolarsen administered groups and the CINRG DNHS patient groups using a restricted maximum likelihood (REML)-based mixed model for repeated measures (MMRM) analysis.

(1) Timed Function Tests

The timed function tests were TTSTAND, TTRW, TTCLIMB, and 6MWT. TTSTAND and TTRW were pre-specified as distinct and separate outcome measures, and were assessed based on time and a 6-point scale. TTSTAND and TTRW are also components of the NSAA. Thus, they were measured once, and the data used as both a stand-alone endpoint and as part of the combined NSAA test. TTSTAND and TTRW are described further below in the context of the combined NSAA scale. TTCLIMB was used to assess the time (in seconds) it took for a patient to climb 4 stairs. It is to be noted that the clinical protocol mentioned in the above "US/Canada Phase 2 Study" incorrectly described the TTCLIMB test as being administered as part of the NSAA.

6MWT is a widely used and accepted test in numerous diseases; the version adapted for use in DMD was used in this study. This test is considered a simple, standardized, low-technology, and cost-effective means of clinically assessing: 1) functional motor status; and 2) integrated and global responses to exercise. To perform the present test, 2 points (cones) were set 25 meters apart, and patients were asked to walk back and forth between the cones quickly and safely for 6 minutes. The total distance in meters that the patient walked in 6 minutes was recorded. The clinical evaluator(s) measured the number of steps taken by the patient for the first 50 meters and the total meters walked in 6 minutes (Craig M. McDonald, MD, Erik K. Henricson, MPH, Jay J. Han, MD, R. Ted Abresch MS, Alina Nicorici, BS, Gary L. Elfring, MS, Leone Atkinson MD, PhD, Allen Reha BS, Samit Hirawat MD, and Langdon L. Miller MD, "The 6-minute Walk Test as a New Outcome Measure in Duchenne Muscular Dystrophy," Muscle & Nerve, Vol. 41, pp. 500-510, April 2010, Wiley Periodicals, Inc. and Craig M. McDonald, MD, Erik K. Henricson, MPH, R. Ted Abresch, MS, Julaine Florence, PhD, Michelle Eagle, PhD, Eduard Gappmaier, PhD, Allan M. Glanzman, DPT, Robert Spiegel, MD, Jay Barth, MD, Gary Elfring, MS, Allen Reha, MS, and Stuart W. Peltz, PhD, "The 6-minute Walk Test and Other Clinical Endpoints in Duchenne Muscular Dystrophy: Reliability, Concurrent Validity, and Minimal Clinically Important Differences from a Multicenter Study, Muscle & Nerve, Vol. 48, pp. 357-368, September 2013, Wiley Periodicals, Inc.).

Quantitative Muscle Testing (QMT) assessments are designed to measure muscle force production during an isometric contraction, and are a well-established method for measuring muscle weakness in neuromuscular disease. The methods here used the CINRG Quantitative Muscle System (CQMS). CQMS included an audio-visual feedback process that increases the compliance of children with DMD. Patients were placed on an examination table with a back-support system to eliminate the need for manual back stabilization. Following a single practice administration, each patient completed a scored QMT evaluation (perform 2 tests; with the higher of the 2 values used for data analysis). QMT was performed by recording force in pounds through a direct computer interface with a strain gauge. Testing positions and test order were standardized. Bilateral testing of the muscle groups listed below were performed (Mayhew JE, Florence JM, Mayhew TP, Henricson EK, Leshner RT, Mccarter RJ, et al., "Reliable surrogate outcome measures in multicenter clinical trials of Duchenne muscular dystrophy," Muscle & Nerve, 2007; 35(1): 36-42. Epub 2006/09/14.):

- Handgrip.
- Elbow flexors (biceps).

- Elbow extensors (triceps).
- Knee flexors (hamstrings).
- Knee extensors (quadriceps).

For QMT, there were observed small mean decreases in strength in all parameters over the 24 week treatment period, with the exception of elbow extensors. The elbow extensors showed small increases (improvements) in strength. None of these changes from baseline was statistically significant at Week 25.

The strength and function tests were performed in the following order: TTSTAND, TTRW, TTCLIMB, NSAA, 6MWT, and QMT.

(2) North Star Ambulatory Assessment (NSAA)

NSAA is a clinician-rated, 17-item, functional scale originally designed for boys with DMD able to ambulate at least 10 meters (E.S. Mazzone, S. Messina, G. Vasco, M. Main, M. Eagle, A. D'Amico, L. Doglio, L. Politano, F. Cavallaro, S. Frosini, L. Bello, F. Magri, A. Corlatti, E. Zucchini, B. Brancalion, F. Rossi, M. Ferretti, M.G. Motta, M.R. Cecio, A. Berardinelli, P. Alfieri, T. Mongini, A. Pini, G. Astrea, R. Battini, G. Comi, E. Pegoraro, L. Morandi, M. Pane, C. Angelini, C. Bruno, M. Villanova, G. Vita, M.A. Donati, E. Bertini, and E. Mercuri, "Reliability of the North Star Ambulatory Assessment in a multicentric setting," Neuromuscular Disorders, Vol. 19, Issue 7, pp. 458-461, Jul 2009, Elsevier B.V.). This evaluation tool assesses functional activities including standing, getting up from the floor, negotiating steps, hopping, and running. The assessment is based on a 3-point rating scale of: 2 = ability to perform the test normally; 1 = modified method or assistance to perform test; and 0 = unable to perform the test. Thus, a total score can range from 0 (completely non-ambulant) to 34 (no impairment) on these assessments. Individual test item scores and a total score were recorded. TTSTAND and TTRW were administered as part of NSAA.

- TTSTAND is routinely performed during standard clinical examinations of patients with DMD. It was used to assess the time it took for a patient to go from lying flat on the floor to standing. The number of seconds required to perform the test and the 6-point rating scale of how the patient attained the standing position were documented.
- TTRW was used to assess the time (in seconds) that it took a patient to run/walk 10 meters (including a 6-point rating scale for quality of the run/walk).

US/Canada Phase 2 Study: Safety

As for safety data from 16 DMD patients treated with high dose and low dose of NS-065/NCNP-01 (Viltolarsen) (40 mg/kg/week or 80 mg/kg/week), no major concerns were raised in the study execution, data quality, or participant safety. No participant discontinued treatment at the time of 48 weeks. Specifically, there were no TEAEs (treatment emergent adverse events) that required discontinuation or dose reduction of NS-065/NCNP-01 (Viltolarsen). All adverse events (AEs) were mild or moderate.

[Example 3] Influence of pH on Stability of Viltolarsen

(A) pH Stability Evaluation (1)

In order to examine the relationship between the liquid properties of a solution and the stability of Viltolarsen, a Britton-Robinson buffer (pH 3, 4, 5, 6, 7, 8, 9, 10 or 11) was used to evaluate the stability of Viltolarsen in the solutions having various pH values.

[Test Conditions]

Concentration of drug solution: Viltolarsen 2 mg/mL
Solution: Britton-Robinson buffer (pH 3, 4, 5, 6, 7, 8, 9, 10 or 11)
Preservation conditions: (1) at 121°C (treated in an autoclave) for 10, 30 or 60 minutes; or (2) at 80°C in an incubator for 4 to 7 days
Endpoints: appearance (confirmation by visual observation), pH (pH meter), purity test (HPLC method), recovery rate (survival rate) (HPLC method)
HPLC conditions:

Flow rate: 1.0 mL/min
Detector: Ultraviolet absorptiometer (measurement wavelength: 264 nm)
Column: A stainless steel tube with an inner diameter of 4.6 mm and a length of 15 cm, filled with 3.5 μm of octadecylsilylated silica gel for liquid chromatography (Waters, X-bridge C18).
Column temperature: 60°C

Mobile phase:

- 1.42 g of Disodium hydrogen phosphate was dissolved in approximately 750 mL of water, and a sodium hydroxide aqueous solution was then added thereto to adjust the pH value of the obtained solution to pH 12.0. Thereafter, water was added to the solution to result in 1000 mL (solution P).
- 300 mL of acetonitrile was added to 700 mL of the solution P, and 16.12 g of tetrabutylammonium bromide was then dissolved in the obtained solution (mobile phases A).
- 300 mL of acetonitrile was added to 300 mL of the solution P, and 9.67 g of tetrabutylammonium bromide was then dissolved in the obtained solution (mobile phases B).
- Liquid feeding of mobile phases: mobile phases A/B were changed from (100 vol%/0 vol%) to (0 vol%/100 vol%) over 30 minutes.

In the present example and the following examples, the percentage of the main peak area of Viltolarsen (main peak area (%)) is shown as a value of the purity test, when a sum of the detected total peak areas comprising impurities was set at 100.

[Test Results]

The test results are shown in Table 10 and Figure 5. "ST" means a Viltolarsen aqueous solution diluted with purified water in the same drug solution concentration, and it was prepared upon every preparation (and was not preserved). As a result, it was found that Viltolarsen was instable in a solution in an acidic range, but was relatively stable in a neutral to weakly alkaline solution.

[Table 10]

buffer (pH 3 to 11) 10 Results of evaluation of stability of Viltolarsen in Britton-Robinson

| Measurement period | Measurement item | ST | pH 3 | pH4 | pH 5 | pH6 | pH7 | pH8 | pH9 | pH10 | pH 11 |
|---|---|---|---|---|---|---|---|---|---|---|---|
| Upon preparation | Appearance | | Clear and colorless | Clear and colorless | Clear and colorless | Clear and colorless | Clear and colorless | Clear and colorless | Clear and colorless | Clear and colorless | Clear and colorless |
| | pH | - | 3.15 | 4.10 | 5.09 | 6.09 | 7.08 | 8.03 | 8.99 | 9.87 | 10.49 |
| | Purity: main peak area (%) | 86.53 | 85.94 | 86.75 | 86.81 | 86.70 | 86.58 | 86.74 | 85.99 | 86.56 | 86.34 |
| | Recovery rate (survival rate) (%) | 100 | 96.6 | 97.5 | 97.9 | 97.6 | 97.8 | 98.7 | 95.1 | 95.8 | 96.5 |

(continued)

| Measurement period | | Measurement item | ST | pH 3 | pH4 | pH 5 | pH6 | pH7 | pH8 | pH9 | pH10 | pH 11 |
|---|---|---|---|---|---|---|---|---|---|---|---|---|
| 121°C | 10 min | Appearance | - | No change | No change | No change | No change | No change | No change | No change | No change | No change |
| | | Purity: main peak area (%) | 86.49 | 29.71 | 68.72 | 81.19 | 84.04 | 84.91 | 85.08 | - | - | - |
| | | Recovery rate (survival rate) (%) | 100 | 25.3 | 69.9 | 89.2 | 92.4 | 93.0 | 95.2 | - | - | - |
| | 30 min | Appearance | | No change | No change | No change | No change | No change | No change | No change | No change | No change |
| | | pH | | 3.51 | 4.16 | 5.08 | | | | | | |
| | | Purity: main peak area (%) | 86.60 | 15.40 | 56.09 | 77.90 | 81.85 | 82.53 | 82.61 | 81.71 | 78.77 | 75.94 |
| | | Recovery rate (survival rate) (%) | 100 | 10.6 | 57.5 | 85.1 | 91.0 | 91.1 | 92.4 | 92.2 | 87.9 | 85.2 |
| | 60 min | Appearance | - | - | - | - | No change | No change | No change | No change | No change | No change |
| | | pH | - | - | - | - | 6.13 | 7.10 | 8.10 | 9.04 | 9.87 | 10.33 |
| | | Purity: main peak area (%) | 86.61 | - | - | - | 78.95 | 79.75 | 79.03 | 77.13 | 73.26 | 68.70 |
| | | Recover rate (survival rate) (%) | 100 | - | - | - | 84.9 | 85.5 | 85.7 | 83.4 | 77.9 | 72.4 |

(continued)

| Measurement period | | Measurement item | ST | pH 3 | pH4 | pH 5 | pH6 | pH7 | pH8 | pH9 | pH10 | pH 11 |
|---|---|---|---|---|---|---|---|---|---|---|---|---|
| 80°C | 4 days | Appearance | - | No change | No change | No change | No change | No change | No change | No change | No change | No change |
| | | pH | - | 3.51 | 4.16 | 5.08 | | | | | | |
| | | Purity: main peak area (%) | 85.71 | 0.38 | 18.01 | 67.16 | 79.64 | 82.94 | 82.65 | 82.58 | 81.32 | 80.52 |
| | | Recover rate (survival rate) (%) | 100 | 0.1 | 12.1 | 64.4 | 87.7 | 93.5 | 91.8 | 91.7 | 89.4 | 88.0 |
| | 7 days | Appearance | - | - | - | - | No change | No change | No change | No change | No change | No change |
| | | pH | - | - | - | - | 6.12 | 7.08 | 8.10 | 9.10 | 9.97 | 10.53 |
| | | Purity: main peak area (%) | 85.06 | - | - | - | 71.42 | 77.43 | 79.75 | 77.54 | 76.48 | 70.71 |
| | | Recover rate (survival rate) (%) | 100 | - | - | - | 78.5 | 86.5 | 88.4 | 84.2 | 82.7 | 75.2 |

(B) pH Stability Evaluation (2)

In order to further specifically examine a pH region in which Viltolarsen is stable, a potassium phosphate-borax buffer (pH 6.0, 6.5, 7.0, 7.5, 8.0, 8.5, 9.0 or 9.2) was used to evaluate the stability of Viltolarsen in the solutions having various pH values (pH 6 to 9).

[Test Conditions]

Concentration of drug solution: Viltolarsen 2 mg/mL

Solution: potassium phosphate-borax buffer (pH 6.0, 6.5, 7.0, 7.5, 8.0, 8.5, 9.0 or 9.2)

Preservation conditions: (1) at 121°C (treated in an autoclave) for 10, 30 or 60 minutes; or (2) at 80°C in an incubator for 1, 4, 7 or 14 days

Endpoints: appearance (confirmation by visual observation), pH (pH meter), purity test (HPLC method), recovery rate (survival rate) (HPLC method)

HPLC conditions:

The same as those applied in the above (A), with the exception that the column temperature was set at 50°C.

[Test Results]

The test results are shown in Table 11 and Figure 6. As a result, it was found that Viltolarsen was most stable in a solution with pH 7 to 7.5.

[Table 11]

Table 11 Results of evaluation of stability of Viltolarsen in potassium phosphate-borax buffer (pH 6 to 9)

| Measurement period | | Measurement item | ST | pH 6 | pH 6.5 | pH7 | pH 7.5 | pH8 | pH 8.5 | pH 9 | pH 9.2 |
|---|---|---|---|---|---|---|---|---|---|---|---|
| Upon preparation | | Appearance | - | Clear and colorless | Clear and colorless | Clear and colorless | Clear and colorless | Clear and colorless | Clear and colorless | Clear and colorless | Clear and colorless |
| | | pH | - | 6.02 | 6.50 | 7.00 | 7.51 | 7.99 | 8.49 | 8.99 | 9.18 |
| | | Purity: main peak area (%) | 86.49 | 85.80 | 85.87 | 85.70 | 86.07 | 85.73 | 85.73 | 85.77 | 85.81 |
| | | Recover rate (survival rate) (%) | 100 | 98.0 | 98.7 | 97.5 | 97.4 | 98.8 | 98.5 | 98.4 | 99.3 |
| 121°C | 10 min | Purity: main peak area (%) | 86.07 | 82.44 | 83.91 | 84.74 | 84.27 | 83.90 | 83.87 | 82.99 | 82.90 |
| | | Recover rate (survival rate) (%) | 100 | 93.9 | 95.2 | 96.7 | 95.4 | 95.4 | 93.5 | 93.4 | 95.2 |
| | 30 min | Purity: main peak area (%) | 86.16 | 79.99 | 80.60 | 81.13 | 80.86 | 80.72 | 79.69 | 78.26 | 77.30 |
| | | Recover rate (survival rate) (%) | 100 | 93.0 | 91.1 | 93.4 | 93.0 | 93.5 | 92.3 | 90.1 | 88.1 |
| | 60 min | Appearance | - | No change | No change | No change | No change | No change | No change | No change | No change |
| | | pH | - | 6.04 | 6.53 | 7.01 | 7.53 | 8.03 | 8.52 | 9.02 | 9.22 |
| | | Purity: main peak area (%) | 85.69 | 76.22 | 77.33 | 77.59 | 77.47 | 76.79 | 75.71 | 73.03 | 71.42 |
| | | Recover rate (survival rate) (%) | 100 | 85.5 | 88.5 | 88.1 | 87.0 | 86.5 | 85.1 | 81.1 | 80.4 |
| 80°C | 1 day | Purity: main peak area (%) | 86.51 | 81.47 | 83.20 | 84.99 | 84.94 | 85.08 | 84.48 | 83.81 | 83.02 |

(continued)

| Measurement period | Measurement item | ST | pH 6 | pH 6.5 | pH7 | pH 7.5 | pH8 | pH 8.5 | pH 9 | pH 9.2 |
|---|---|---|---|---|---|---|---|---|---|---|
| | Recover rate (survival rate) (%) | 100 | 92.5 | 95.0 | 96.4 | 96.7 | 97.6 | 96.8 | 96.0 | 93.6 |
| 4 days | Purity: main peak area (%) | 86.40 | 67.46 | 72.62 | 78.71 | 77.81 | 75.79 | 75.37 | 71.64 | 69.70 |
| | Recover rate (survival rate) (%) | 100 | 75.6 | 81.6 | 89.7 | 87.9 | 86.2 | 85.4 | 80.5 | 76.5 |
| 7 days | Purity: main peak area (%) | 86.12 | 59.16 | 62.98 | 73.62 | 74.22 | 70.30 | 67.95 | 66.52 | 60.86 |
| | Recover rate (survival rate) (%) | 100 | 64.1 | 72.4 | 83.0 | 83.1 | 78.8 | 74.8 | 74.0 | 66.7 |
| 14 days | Appearance | - | No change | No change | No change | No change | No change | No change | No change | No change |
| | pH | - | 6.06 | 6.54 | 7.03 | 7.54 | 8.03 | 8.52 | 9.02 | 9.21 |
| | Purity: main peak area (%) | 86.08 | 39.64 | 50.26 | 60.10 | 62.46 | 55.41 | 53.98 | 49.95 | - |
| | Recover rate (survival rate) (%) | 100 | 40.4 | 54.2 | 62.5 | 68.4 | 58.9 | 57.7 | 50.3 | - |

[Example 4] Influence of pH adjuster on stability of formulation

The present example is directed towards selecting a pH adjuster for adjusting the Viltolarsen injection solution to a stable pH range (pH 7 to 7.5). Various types of pH adjusters (10 mM $KH_2PO_4$, 10 mM $Na_2HPO_4$, 10 mM $KH_2PO_4$-$Na_2HPO_4$ or 100 mM $KH_2PO_4$-$Na_2HPO_4$) were added to a 10 mg/mL Viltolarsen solution, so that the pH of the Viltolarsen solution was adjusted to a stable pH value. Thereafter, the stability of the 10 mg/mL Viltolarsen solutions was evaluated.

[Test Conditions]

Concentration of drug solution: Viltolarsen 10 mg/mL

Prescription of drug solution: as shown in the following Table 12

Preservation conditions: at 121°C (treated in an autoclave) for 30 to 60 minutes

Endpoints: appearance (confirmation by visual observation), pH (pH meter), purity test (HPLC method), recovery rate (survival rate) (HPLC method)

[Table 12]

Table 12 Prescription of drug solution

| pH adjuster | No buffer (HCl/ NaOH) | 10 mM $KH_2PO_4$ | 10 mM $Na_2HPO_4$ | 10 mM $KH_2PO_4$-$Na_2HPO_4$ | 100 mM $KH_2PO_4$-$Na_2HPO_4$ |
|---|---|---|---|---|---|
| Viltolarsen (mg) | 10 | 10 | 10 | 10 | 10 |
| Sodium chloride (mg) | 9 | 9 | 9 | 9 | 9 |
| Potassium dihydrogen phosphate (mg) | - | 1.36 | - | 0.27 | 2.72 |
| Disodium hydrogen phosphate (mg) | - | - | 1.42 | 1.14 | 11.36 |
| 0.1 mol/mL HCl | q.s.* | - | q.s.* | q.s.* | q.s.* |
| 0.1 mol/mL Sodium hydroxide | q.s.* | q.s.* | - | q.s.* | q.s.* |
| Water for injection | q.s. | q.s. | q.s. | q.s. | q.s. |
| Total amount (mL) | 1 | 1 | 1 | 1 | 1 |
| *: pH value is adjusted to pH 7.3 | | | | | |

[Test Results]

The test results are shown in Table 13 and Figure 7. As a result, it was found that, even without using buffers, Viltolarsen was stable at a level equivalent to or greater than the case of using a phosphate buffer. In particular, without using buffers, Viltolarsen was more stable than in a high-concentration phosphate buffer (100 mM $KH_2PO_4$-$Na_2HPO_4$ in the present example).

[Table 13]

Table 13 Results of examination of pH adjusters

| Measurement period | | Measurement item | ST | No buffer | 10mM $KH_2PO_4$ | 10mM $Na_2HPO_4$ | 10mM $KH_2PO_4$ $Na_2HPO_4$ | 100mM $KH_2PO_4$ $Na_2HPO_4$ |
|---|---|---|---|---|---|---|---|---|
| Upon preparation | | Appearance | - | Clear and colorless | Clear and colorless | Clear and colorless | Clear and colorless | Clear and colorless |
| | | pH | - | 7.32 | 7.32 | 7.33 | 7.30 | 7.32 |
| | | Purity: main peak area (%) | 86.52 | 86.17 | 85.95 | 85.98 | 86.15 | 86.07 |
| | | Recovery rate (survival rate) (%) | 100 | 100.0 | 98.7 | 98.9 | 98.8 | 99.0 |
| 121°C | 10 min | Appearance | - | No change | No change | No change | No change | No change |
| | | Purity: main peak area (%) | 85.79 | 84.78 | 84.47 | 84.41 | 84.30 | 84.01 |
| | | Recovery rate (survival rate) (%) | 100 | 96.7 | 96.7 | 97.3 | 97.1 | 95.7 |
| | 30 min | Appearance | - | No change | No change | No change | No change | No change |
| | | Purity: main peak area (%) | 86.52 | 83.41 | 83.35 | 83.42 | 83.57 | 82.57 |
| | | Recovery rate (survival rate) (%) | 100 | 94.5 | 94.0 | 94.4 | 94.5 | 90.3 |
| | 60 min | Appearance | - | No change | No change | No change | No change | No change |
| | | pH | - | 7.26 | 7.27 | 7.28 | 7.26 | 7.23 |
| | | Purity: main peak area (%) | 85.79 | 80.81 | 79.72 | 79.67 | 79.63 | 78.07 |
| | | Recovery rate (survival rate) (%) | 100 | 92.3 | 89.5 | 91.2 | 91.1 | 87.5 |

[Example 5] Relationship between buffer and generation of multimers

Viltolarsen may generate multimers depending on preservation conditions, and the purity of monomers may be thereby decreased. In the present example, 0.9% sodium chloride was added as a tonicity agent to a 50 mg/mL Viltolarsen drug solution, and then, various types of buffers were further added thereto. Thereafter, generation of multimers was measured.

[Test Conditions]

Concentration of drug solution: Viltolarsen 50 mg/mL
Preservation conditions: at 60°C in an incubator for 5 days
Endpoints: Multimers (HPLC method, SEC column)

Analysis conditions:

Detector: Ultraviolet absorptiometer (measurement wavelength: 260 nm)
Column: A stainless steel tube having an inner diameter of 7.8 mm and a length of 30 cm was filled with 7 μm of styrene-based vinyl polymer gel for liquid chromatography; and two of such columns were connected in series (TSK gel G3000 PWXL, 7 μm, 7.8 mm x 30 cm, Tosoh Corporation).
Column temperature: 25°C

Mobile phase: 15.6 g of Sodium dihydrogen phosphate dihydrate was dissolved in 750 mL of water, and a sodium hydroxide sample solution was then added thereto to adjust the pH value of the solution to pH 7.3. Then, water was added to the solution to result in 1000 mL. Thereafter, 200 mL of acetonitrile was added to 800 mL of the obtained solution.
Flow rate: Flow rate was adjusted, so that the retention time of Viltolarsen monomers became approximately 21 minutes.
Area measurement range: Up to the peak of Viltolarsen monomers

[Test Results]

The test results are shown in Table 14. As a result, it was found that generation of multimers was increased by addition of a 50 mM phosphate buffer (sodium dihydrogen phosphate dihydrate-disodium hydrogen phosphate: $NaH_2PO_4 \cdot 2H_2O$-$Na_2HPO_4$) or a citrate buffer (trisodium citrate dihydrate).

[Table 14]

Table 14 Results of evaluation of multimers (preserved at 60°C)

| Buffer | Concentration of buffer (mM) | Sodium chloride (mM) | Amount of multimers (RRT 0.95) | | | |
|---|---|---|---|---|---|---|
| | | | Initial value | 1d | 2d | 5d |
| No buffer | | 150 | N.D. | N.D. | N.D. | 0.15 |
| Citrate | 10 | 150 | N.D. | N.D. | 0.12 | 0.14 |
| | 50 | 150 | N.D. | N.D. | 0.13 | 0.16 |
| Tris | 10 | 150 | N.D. | N.D. | N.D. | 0.14 |
| | 50 | 150 | N.D. | N.D. | N.D. | 0.13 |
| Phosphate | 10 | 150 | N.D. | N.D. | N.D. | 0.14 |
| | 50 | 150 | N.D. | N.D. | N.D. | 0.20 |

[Example 6] Studies of solubility (100 mg/mL)

An increase in the concentration of an injection solution was studied.

[Test Conditions]

Drug solution: the following 2 types

[Table 15]

Table 15 Drug solution (2 types)

| Main drug concentration | 100 mg/mL | |
|---|---|---|
| pH adjuster | No buffer (HCl, NaOH) | 10mM $KH_2PO_4$-$Na_2HPO_4$ |
| Viltolarsen (mg) | 100 | 100 |
| NaCl (mg) | 9 | 9 |
| $KH_2PO_4$ (mg) | - | 0.27 |
| $Na_2HPO_4$ (mg) | - | 1.14 |
| 0.1 N HCl | q.s.* | q.s.* |
| 0.1 N NaOH | q.s.* | q.s.* |
| Water for injection | q.s. | q.s. |
| Total amount (mL) | 1 | 1 |
| *: pH value is adjusted to pH 7.3 | | |

Filtration filter: made of PVDF (Millex GV, 0.22 μm, 33 mm, Millipore)

Endpoints: appearance (confirmation by visual observation), pH (pH meter), purity test (HPLC method), recovery rate after filtration (HPLC method)

[Test Results]

The test results are shown in Table 16. A 100 mg/mL Viltolarsen injection solution was prepared. The prepared Viltolarsen injection solution had no problems regarding solubility or filtration using a sterilized filter, and it was a clear and colorless solution. After preservation of the solution in a cold place, the solution became viscous, but there was no change in the appearance. From these results, it was found that it is possible to prepare a 100 mg/mL injection solution.

[Table 16]

Table 16 Results of examination of high-concentration injection solution

| Drug solution | Measurement items | Before filtration | After filtration (PVDF) |
|---|---|---|---|
| 100 mg/mL, No buffer | Appearance | Clear and colorless | No change |
| | pH | 7.28 | 7.25 |
| | Purity: Main peak area (%) | 93.31 | 93.52 |
| | Recovery rate (%) | 100.0 | 99.0 |
| 100 mg/mL, 10 mM phosphate buffer | Appearance | Clear and colorless | No change |
| | pH | 7.28 | 7.28 |
| | Purity: Main peak area (%) | 93.28 | 93.33 |
| | Recovery rate (%) | 100.0 | 98.9 |

[Example 7] Studies of solubility (50 mg/mL)

The solubility of a 50 mg/mL drug solution was evaluated. A drug solution was prepared, and the solubility thereof and filtration using a sterilized filter were evaluated.

[Test Conditions]

Drug solution:

[Table 17]

Table 17

| Main drug concentration | 50 mg/mL |
|---|---|
| pH adjuster | No buffer (HCl, NaOH) |
| Viltolarsen (g)<br>NaCl (g)<br>0.1 N HCl<br>0.1 N NaOH<br>Water for injection | 1300<br>234<br>q.s.*<br>q.s.*<br>q.s. |
| Total amount (L) | 26 |
| *: pH value is adjusted to pH 7.3 | |

Filtration filter: made of PVDF (Millidisk Cartridge Filter, 0.22 μm, MCGL40S03/one + MCGL40S03/two, Merck)

Endpoints: appearance (confirmation by visual observation), pH (pH meter), purity test (HPLC method), quantification (HPLC method)

[Test Results]

The test results are shown in Table 18. As a result, the 50 mg/mL drug solution became a clear and colorless solution, and there was no change in the content between before and after the filtration. From these results, it was found that it is possible to prepare a 50 mg/mL injection solution, and that there are no problems regarding scale-up.

[Table 18]

Table 18 Evaluation results

| Test item | | Before filtration | After filtration |
|---|---|---|---|
| Appearance | | Clear and colorless solution | Clear and colorless Solution |
| pH | | 7.3 | 7.4 |
| Purity test (related substances) | RRT = 0.80 | 0.22% | 0.22% |
| | RRT = 0.85 | 1.74% | 1.74% |
| | RRT = 0.91 | 0.72% | 0.70% |
| | RRT = 1.07 | 2.09% | 1.99% |
| | RRT = 1.24 | 0.31% | 0.30% |
| | Other individuals | 0.13% | 0.12% |
| | Total of related substances | 5.39% | 5.26% |
| Quantification | | 100.9% | 100.8% |

In the following Examples 8 to 10, tests were carried out regarding samples obtained from the patients who had participated in the clinical trial of Example 2 or the patients themselves who had participated in the clinical trial of

Example 2.

[Example 8]

Quantification of dystrophin protein according to mass spectrometry

(Experimental methods)

Outline: Biological analysis method

The sample was subjected to SDS-PAGE electrophoresis to separate a protein, and the separated protein was then subjected to in-gel digestion using trypsin to obtain peptide fragments. The peptide fragments were extracted from the gel section, and were then dried. The peptides were then re-dissolved, and thereafter, identification and quantification of a dystrophin protein were carried out by HPLC-MS/MS using a reverse phase column. When the amount of a dystrophin protein in a normal control was set at 100%, the range of a calibration curve was 1% to 25%. The concentration was calculated from a peak area ratio, using filamin C as a standardized protein. For regression equation, a least-squares method was applied, and the equation: $y = mx + b$ (y: peak area ratio, x: % dystrophin) was obtained.

In-gel digestion

The sample to be measured by LC-MS/MS analysis was obtained by separating a protein by SDS-PAGE (SDS: sodium dodecyl sulfate, PAGE: polyacrylamide gel electrophoresis), depending on the molecular weight, and subjecting the separated protein to in-gel digestion. Each gel was composed of a total of 11 samples, namely, Analytes constituting a standard curve (0.0%, 1.0%, 3.0%, 10.0%, and 25.0% dystrophins), 12.5 μg of a protein (SILAC) extracted from human myotube cells to which stable, isotope-labeled amino acids had been added and which had been then cultured (SILAC), a blank, and 4 clinical trial samples. There were 12 lanes for the gel, and the remaining one lane was used for a molecular weight marker. The Analyte was produced by mixing protein extracts from 5 types of non-DMD muscle biopsy and 2 types of DMD muscle biopsy with one another. The DMD muscle biopsy was acquired from Binghamton University and the ethical review thereof was completed. Before performing the present example, the amount of a dystrophin in each muscle biopsy had previously been measured by Western Blot. Using a cryosection preparation device, 70 slices of serial sections each having a thickness of 10 μm were obtained from the muscle biopsy. The muscle section was transferred into a microtube that had previously been cooled on dry ice. Using RIPA buffer comprising the protease/phosphatase inhibitor of Thermo Scientific, a protein was extracted from the muscle section. The protein concentration in the extract was quantified using BCA protein assay kit (Pierce). Each sample to be electrophoresed comprised 50 μg of a protein, and was prepared by adding 12.5 μg of SILAC. To the Analyte and the clinical sample, an SILAC extract was added as an internal standard for obtaining % dystrophin. Using NuPAGE 3-8% Tris-Acetate gel, electrophoresis was carried out at 150 V for 75 minutes. The gel electrophoresis was carried out in a duplicate manner, using two gels (gel A and gel B) with respect to a single sample. The electrophoresed gel was immobilized using methanol : water : acetic acid (50 : 45 : 5) for 30 minutes, followed by exchanging with water, and rehydration was then carried out twice. Thereafter, Coomassie blue staining was carried out for 1 hour. Decolorization of the gel was carried out at 4°C overnight. Gel, in which a dystrophin protein in the range from 460 kDa to 268 kDa according to the molecular weight marker was present, was cut out, and was then washed with water: acetonitrile (50 : 50) twice. In-gel digestion was performed using trypsin (Gold mass spectrometry grade, Promega Corporation) to obtain peptide fragments, and the obtained peptide fragments were then dried by vacuum centrifugation. The peptide fragments of dystrophin were preserved at -80°C, and was then used in an analysis according to Q Exactive Nano-LC-MS/MS.

Test sample

Sixteen patients participated in the clinical trial. Muscle biopsy specimens were obtained from each of 16 patients before and after administration. Sixty-four samples were obtained from 32 specimens as a result of duplication, and were then analyzed. Moreover, since 8 samples obtained from 4 specimens as a result of duplication were subjected to a reanalysis, a total of 72 samples were analyzed.

Analysis according to LC-MS/MS

The obtained peptide fragments were analyzed by a liquid chromatography mass spectrometry method (LC-MS/MS), in which a high performance liquid chromatography system, Dionex Ultimate 3000 RSLCnano (Thermo Fisher Scientific) was combined with a mass spectrometry device, Q Exactive Plus (HRMS: High Resolution Mass Spectrometer)

(Thermo Fisher Scientific).

The dried peptide fragments were re-dissolved in 2% acetonitrile (ACN) + 0.1% trifluoroacetic acid (TFA). For introduction of the sample into LC-MS/MS, an injection loop 5 μL, Dinoex nano Viper sample loop (Thermo Fisher Scientific) was used.

The liquid chromatography was carried out under the following conditions.

Analysis column: Reverse phase column, Acclaim Pepmap RSLC C18, 15 cm x 75 μm, particle size: 3 μm, EASY SPRAY (Thermo Fisher)

Analytical column temperature: 50°C
Mobile phases A: 1% formic acid (HCOOH); Mobile phases B: 0.1% formic acid in ACN
Flow rate: 0.500 μL/min (NC)
Sample injection mode: Partial loop (loop size: 5 μL)
Sample injection amount: 1.00 - 4.00 μL
Autosampler temperature: 10°C
Run time: 40.00 minutes

[Table 19]

Table 19 NC pump gradient setting

| Time (min) | Flow rate | % Mobile phase A | % Mobile phase B |
|---|---|---|---|
| 0.00 | 0.500 | 99.0 | 1.0 |
| 6.50 | 0.500 | 99.0 | 1.0 |
| 26.50 | 0.500 | 65.0 | 35.0 |
| 26.60 | 0.500 | 10.0 | 90.0 |
| 29.00 | 0.500 | 10.0 | 90.0 |
| 29.10 | 0.500 | 99.0 | 1.0 |
| 40.00 | 0.500 | 99.0 | 1.0 |

The mass spectrometry device was used under the following conditions.

Ion source: Thermo Fisher EASY-Spray

Ion mode: Cation

Scanning: Parallel reaction monitoring

Chrome peak width (full width at half maximum): 10 seconds

Minimum total cycle time: 40 minutes

Resolution: 17,500

Automatic gain control target: 1e5

Maximum sample injection time: 50 milliseconds

Quadrupole mass spectrometer separation width: 1.0 m/z unit

Spectrum data: Profile

Mass tolerance: 10 ppm

Typical Tunable Parameters (Dystrophin Tune File)

Spray Voltage: 1.8 kV

Capillary Temperature: 275°C

S-Lens RF-Level: 70

[Table 20]

Table 20 Mass selection list

| Peptide ID | Peptide sequence | Mass (m/z) | Charge number (z) | Retention time: Start (min) | Retention time: Stop (min) |
|---|---|---|---|---|---|
| DYST 2 | IFLTEQPLEGLEK | 758.9165 | 2 | 18.80 | 20.40 |
| DYST 2 IS | IFLTEQPLEGLEK^ | 762.9236 | 2 | 18.80 | 20.40 |
| FilC 1 | VAVGQEQAFSVNTR | 753.3890 | 2 | 15.40 | 17.40 |
| FilC_ 1_IS | VAVGQEQAFSVNT **R**^ | 756.3990 | 2 | 15.40 | 17.40 |
| FilC_2 | SPFVVNVAPPLDLS K | 791.9456 | 2 | 20.30 | 22.20 |
| FilC_ 2_IS | SPFVVNVAPPLDLS **K**^ | 795.9527 | 2 | 20.30 | 22.20 |
| K^ = Lys ($^{13}C6$, $^{15}N2$); **R**^ = Arg ($^{13}C6$, $^{15}N4$) - isotope-labeled amino acids | | | | | |

[Table 21]

Table 21 Predicted retention time

| Peptide ID | Retention time (min) |
|---|---|
| DYST_ 2 | 19.95 |
| DYST_ 2_ IS | 19.95 |
| FilC_ 1 | 16.24 |
| FilC_ 1_IS | 16.24 |
| FilC_ 2 | 20.68 |
| FilC_ 2_ IS | 20.68 |

Retention time changes within the range of ±1.5 minutes.

[Table 22]

Table 22 Order of injecting samples electrophoresed with same gel into LC-MS/MS

| Order | Sample |
|---|---|
| 1 | Sample excluded from analysis |
| 2 | Sample excluded from analysis |
| 3 | Blank |
| 4 | Blank |
| 5 | SILAC |
| 6 | Analyte dystrophin 0% |
| 7 | Analyte dystrophin 1% |
| 8 | Analyte dystrophin 3% |
| 9 | Analyte dystrophin 10% |
| 10 | Analyte dystrophin 25% |
| 11 | Sample excluded from analysis |
| 12 | Clinical sample 1 |
| 13 | Sample excluded from analysis |
| 14 | Clinical sample 2 |
| 15 | Sample excluded from analysis |
| 16 | Clinical sample 3 |
| 17 | Sample excluded from analysis |
| 18 | Clinical sample 4 |

Sample excluded from analysis: SILAC diluted with blank

Using LC Quan version 3.0 manufactured by Thermo Scientific, data were collected from the chromatogram. The mass tolerance was set at 20 ppm, and the integration algorithm was set at ICIS. When the peak area of dystrophin in Analyte 1% or other Analytes was 10,000 or less, attention was paid to the following. That is, it was confirmed that, in both dystrophin and filamin C, the peak area ratio between the obtained peptides and the labeled peptides is a reliable numerical value, which does not disturb % dystrophin, in comparison to Analyte 0%.

The peak area of dystrophin and that of filamin C were calculated by summing peak areas matched to product ions. The peak area of dystrophin was obtained from one type of peptide fragment of dystrophin (i.e., DYST2 amino acid sequence: IFLTEQPLEGLEK (SEQ ID NO: 4)). The peak area of filamin C was set to be an average value of two types of peptide fragments of filamin C (i.e., FILC1 amino acid sequence: VAVGQEQAFSVNTR (SEQ ID NO: 6), and FILC2 amino acid sequence: SPFWNVAPPLDLSK (SEQ ID NO: 8)).

[Table 23]

Table 23

| Dystrophin | Peptide sequence | MS1 (m/z) | Product ion |
|---|---|---|---|
| DYST_2 | IFLTEQPLEGLEK (SEQ ID NO: 4) | 758.9165 | 785.4387, 1042.5400, 1143.5870, 1256.6697 |
| DYST_ 2_ IS | IFLTEQPLEGLE**K**^ | 762.9236 | 793.4521, 1050.5500, 1151.5932, 1264.6708 |
| Filamin C | Peptide sequence | MS 1 (m/z) | Product ion |
| FilC_1 | VAVGQEQAFSVNTR (SEQ ID NO: 5) | 753.3890 | 723.3784, 794.4155, 922.4741, 1051.5167 |
| FilC_ 1_ IS | VAVGQEQAFSVNT**R**^ | 756.3990 | 729.3985, 800.4356, 928.4942, 1057.5368 |

(continued)

| Dystrophin | Peptide sequence | MS1 (m/z) | Product ion |
|---|---|---|---|
| FilC_2 | SPFWNVAPPLDLSK (SEQ ID NO: 6) | 791.9456 | 769.4454, 840.4825, 1053.5938, 1152.6623 |
| FilC_ 2_ IS | SPFVVNVAPPLDLS**K**^ | 795.9527 | 777.4596, 848.4974, 1061.6081, 1160.6765 |

The dystrophin protein level (the peak area ratio between dystrophin and filamin C) in the Analytes and individual clinical samples was calculated according to the following equation. Since dystrophin was detected even in Analyte 0%, correction was performed by subtracting the numerical value of the dystrophin protein level in Analyte 0% from the numerical value of the dystrophin protein level in each Analyte. Using the Excel template, the regression line of the numerical values of % dystrophin and dystrophin protein levels was obtained from the Analytes for each gel, and % dystrophin in clinical samples was then obtained from the numerical values of the dystrophin protein levels in the clinical samples.

[Equation 1]

$$\text{Dystrophin protein level} = \frac{\text{Dystrophin ratio}}{\text{Filamin C ratio}}$$

$$= \frac{\text{Dystrophin of each sample / Dystrophin of SILAC labeled form}}{\text{Filamin C of each sample/ Filamin C of SILAC labeled form}}$$

[Table 24]

Table 24

<table>
<tr><th></th><th colspan="2">LCquan</th><th colspan="2">Excel</th></tr>
<tr><td>1</td><td>DYST 2 peak area</td><td rowspan="2">Calculate ratio between Dyst 2 peak area and labeled Dyst 2 peak area</td><td rowspan="2">Ratio between Dyst 2 peak area and labeled Dyst 2 peak area</td><td rowspan="6">% Dystrophin (Calculated from numerical value of dystrophin protein level obtained by dividing ratio between Dyst 2 and labeled Dyst 2 peak areas by ratio between FilC and labeled FilC peak areas, and regression line of % dystrophin of Analytes)</td></tr>
<tr><td>2</td><td>DYST_ 2_IS peak area</td></tr>
<tr><td>3</td><td>FilC_ 1 peak area</td><td rowspan="2">Calculate ratio between FilC 1 peak area and labeled FilC 1 peak area</td><td rowspan="4">Ratio between FilC peak area and labeled FilC peak area (average value of the left two values)</td></tr>
<tr><td>4</td><td>FilC_ 1_IS peak area</td></tr>
<tr><td>5</td><td>FilC_ 2 peak area</td><td rowspan="2">Calculate ratio between FilC 2 peak area and labeled FilC 2 peak area</td></tr>
<tr><td>6</td><td>FilC_ 2_IS peak area</td></tr>
</table>

(Results)

% Dystrophin in the analyzed individual samples was determined to be accepted or not according to the predetermined acceptance criteria. Among the measured 72 samples, 60 samples satisfied the criteria. The measurement results are shown in Tables 25 and 26. Individual specimens were tested using duplicated samples. However, with regard to 4 specimens obtained from two patients in the 40 mg/kg dose group (patients E and F in Table 25) before and after administration, since one sample (gel B) did not satisfy the criteria, the measured value was only one from the other sample (gel A). That is to say, in the 40 mg/kg dose group, among 16 samples from 8 specimens before administration, 14 samples could be measured. One sample showed 1% of dystrophin, and 11 samples were measured to be below the lower limit of quantification. In addition, among 16 samples from 8 specimens at Week 25, 14 samples could be measured. One specimen was measured to be below the lower limit of quantification for both of the two measurements. Other than this, 1% or more of dystrophin was detected.

In the case of the 80 mg/kg dose group, all of the 16 samples from 8 specimens before administration were measured to be below the lower limit of quantification. In addition, in all of the 16 samples from 8 specimens at Week 25, dystrophin was detected at a level above the limit of quantification, and the detected amount was 4.2% on average.

From the above results, it was confirmed that the expression of a dystrophin protein is recovered by administration of Viltolarsen at doses of 40 mg/kg and 80 mg/kg.

[Table 25]

Table 25 Dystrophin protein concentrations in clinical trial sample extracts of human muscle biopsies calculated using DYST_2

| • Cohort 1 (40 mg/kg dose group) | | | |
|---|---|---|---|
| Patients to be tested | Visit date | % Dystrophin (Gel A) | % Dystrophin (Gel B) |
| Patient A | Before administration | BLQ | BLQ |
| Patient A | Week 25 | 2.9 | 2.2 |
| Patient B | Before administration | BLQ | BLQ |
| Patient B | Week 25 | 2.4 | 2.1 |
| Patient C | Before administration | BLQ | BLQ |
| Patient C | Week 25 | 2.2 | 1.5 |
| Patient D | Before administration | 1.0 | BLQ |
| Patient D | Week 25 | 3.2 | 3.4 |
| Patient E | Before administration | BLQ | NR |
| Patient E | Week 25 | 1.3 | NR |
| Patient F | Before administration | BLQ | NR |
| Patient F | Week 25 | 3.3 | NR |
| Patient G | Before administration | BLQ | BLQ |
| Patient G | Week 25 | 2.1 | 1.8 |
| Patient H | Before administration | BLQ | BLQ |
| Patient H | Week 25 | BLQ | BLQ |
| NR: Impossible to be described in reports. (Since the peak area of dystrophin was obtained only in two Analytes, it did not satisfy inspection pass criteria.)<br>BLQ: below the lower limit (1%) of quantification | | | |

[Table 26]

Table 26 Dystrophin protein concentrations in clinical trial sample extracts of human muscle biopsies calculated using DYST_2

| • Cohort 2 (80 mg/kg dose group) | | | |
|---|---|---|---|
| Patients to be tested | Visit date | % Dystrophin (Gel A) | % Dystrophin (Gel B) |
| Patient I | Before administration | BLQ | BLQ* |
| Patient I | Week 25 | 4.5 | 3.8 |
| Patient J | Before administration | BLQ | BLQ* |
| Patient J | Week 25 | 1.8 | 3.5 |
| Patient K | Before administration | BLQ | BLQ |
| Patient K | Week 25 | 2.2 | 2.6 |
| Patient L | Before administration | BLQ | BLQ |
| Patient L | Week 25 | 1.1 | 2.2 |
| Patient M | Before administration | BLQ | BLQ |
| Patient M | Week 25 | 1.3 | 1.3 |
| Patient N | Before administration | BLQ | BLQ |
| Patient N | Week 25 | 12.2 | 9.3 |
| Patient O | Before administration | BLQ | BLQ |
| Patient O | Week 25 | 10.2 | 8.6 |
| Patient P | Before administration | BLQ | BLQ |
| Patient P | Week 25 | 1.6 | 1.3 |
| BLQ: below the lower limit (1%) of quantification<br>*: Since the peak area value of Analyte dystrophin 1% was low, the lower limit of quantification became less than 3%. | | | |

[Example 9]

Motor function test (at Week 13 and at Week 25 (12 weeks and 24 weeks passed from initial administration)

(Experimental method)

The motor function of the present drug group was evaluated by comparing it with a natural history group used as a control. The natural history group was selected based on the data at baseline, from the research called Duchenne natural history study (CINRG DNHS) conducted by a cooperative international neuromuscular research group (CINRG) that is a U.S. muscular dystrophy clinical trial network.

CINRG DNHS is a longitudinal natural history study, in which each of 440 DMD male patients was followed as targets over a period of years, data was collected from years 2006 to 2016, and the patients were determined to come to the hospital at the time of baseline, four times in the first year, two times in the second year, and then, once a year, for the maximum period of 10 years. At each visit to the hospital, the patients were subjected to a timed function test, a muscular strength test, a function test by questionnaire, a lung function test, and evaluation of the quality of life. The 201 trial was carried out in facilities belonging to CINRG, and the standard operating procedures (SOP) and the clinical evaluator training protocols were matched between both tests.

As a control of the present study, patients who satisfied the following criteria including main registration criteria such as age, status of steroid use, and area for the 201 trial, were selected from CINRG DNHS.

- Have data from timed function tests for at least 12 months [data from time to stand (TTSTAND), time to climb 4 stairs (TTCLIMB) and time to run/walk 10 meters (TTRW) at baseline were required] .
- Patients between age 4 and < 10 years old at baseline.
- Geographic region: North America (US and Canada).
- Administered corticosteroid for at least 3 months and continuous corticosteroid use throughout the 12-24 month

observation period.

- Patients not simultaneously registered to other clinical studies regarding other exon skipping agents
- Patients satisfy the following genetic eligibility criteria.

(Inclusion Criteria):

- Patients having genetic test results
- Patients having duplication mutation
- Patients having nonsense mutation or minute mutation causing frame shift (Exclusion Criteria):
- Patients having mutation between promoter and exon 8 (These patients were excluded because they were reported to have slow disease progression ((1) Hum Mutat. 2018; 39: 1193-1202 and (2) Hum Mutat. 2008; 29(5): 728-37.)
- Patients amenable to a treatment involving exon 44 skipping (These patients were excluded because they were reported to have slow disease progression ((1) Hum Mutat. 2018; 39: 1193-1202.)

Patients having in-frame mutation

Consequently, 65 DMD male patients satisfied the above-described criteria. Among the patients, 9 DMD patients were amenable to a treatment involving exon 53 skipping (exon 53 skip group), and 56 DMD patients were not amenable to a treatment involving exon 53 skipping (non-exon 53 skip group).

[Table 27]

Table 27 Parameters of Viltolarsen administered group and CINRG natural history control group (DNHS)

| **Parameters** | **Viltolarsen n = 16** | **DNHS Exon 53 Skip** n=9 | **DNHS Non-Exon 53 Skip n = 56** | **DNHS Total n=65** |
|---|---|---|---|---|
| Age (years)<br>Average value (min. - max.) | 7.4 (4.3 - 9.8) | 6.3 (4.5 - 7.8) | 7.2 (4.2 - 9.6) | 7.1 (4.2 - 9.6) |
| Body weight (kg)<br>Average value (min. - max.) | 23.0 (14.9 - 35.4) | 21.6 (16.6 - 28.1) | 24.4 (148 - 38.7 | 24.0 (14.8 - 38.7) |
| Mutation type, n (%) | | | | |
| - amenable to exon 53 skipping treatment | 16 (100) | 9 (100) | 0 | 9 (13.8) |
| Single exon deletion | 0 | 3 (33.3) | 0 | 3 (4.6) |
| Multiple exon deletions | 16 (100) | 6 (66.7) | 0 | 6 (9.2) |
| - not amenable to exon 53 skipping treatment | 0 | 0 | 56 (100) | 56 (86.2) |
| Single exon deletion | 0 : | 0 | 4 (7.1) | 4 (6.2) |
| Multiple exon deletions | 0 | 0 | 30 (53.6) | 30 (46.2) |
| Single exon duplication | 0 | 0 | 3 (5.4) | 3 (4.6) |
| Multiple exon duplications | 0 | 0 | 3 (5.4) | 3 (4.6) |
| Minute mutation | 0 | 0 | 16 (28.6) | 16 (24.6) |

(Results)

Changes in the timed function tests [i.e., 6-minute walk test (6MWT), velocity regarding time to stand (TTSTAND), velocity regarding time to climb 4 stairs (TTCLIMB), and velocity regarding time to run/walk 10 meters (TTRW)] and North Star Ambulatory Assessment (NSAA) at Week 13 and at Week 25 from before administration or from baseline were compared between 16 subjects treated with Viltolarsen (Viltolarsen administered group) and 65 patients in the natural history group of CINRG DNHS (DNHS group).

In the analysis method applied in the present example, the "value before administration" or the baseline was set to be a covariate that is a background factor influencing on the results, the data obtained at Week 13 and at Week 25 were used as values repeatedly measured from specific subjects (repeated measures), and MMRM analysis was carried out. As a result, the Viltolarsen administered group of the present example was significantly improved in terms of 6-minute walking distance and the velocity regarding time to run/walk 10 meters. Moreover, the present drug group was improved rather than the natural history group even in terms of all other motor function tests.

Figure 8 includes graphs showing changes in individual motor function test results of the Viltolarsen administered group and the CINRG natural history control group (DNHS) at Week 13 of administration (12 weeks passed from initial administration) and at Week 25 of administration (24 weeks passed from initial administration) from each baseline. The changed amount indicates a least mean square of an amount at Week 13 or at Week 25 changed from baseline, the error bar indicates standard deviation, and the P value was calculated using MMRM.

The number of samples of the Viltolarsen administered group and that of the DNHS group at individual time points were as follows.

[Table 28]

Table 28

| Viltolarsen administered group | Baseline | Week 13 | Week 25 |
|---|---|---|---|
| 6-minute walk test | 16 | 15 | 15 |
| North Star Ambulatory Assessment | 16 | 15 | 16 |
| Time to stand | 16 | 16 | 16 |
| Time to climb 4 stairs | 16 | 16 | 16 |
| Time to run/walk 10 meters | 16 | 16 | 16 |

[Table 29]

Table 29

| DNHS | Baseline | Week 13 | Week 25 |
|---|---|---|---|
| 6-minute walk test | 21 | 12 | 13 |
| North Star Ambulatory Assessment | 22 | 11 | 15 |
| Time to stand | 65 | 39 | 42 |
| Time to climb 4 stairs | 65 | 40 | 42 |
| Time to run/walk 10 meters | 65 | 40 | 43 |

[Example 10]

Motor function tests (at Week 85 (84 weeks passed from initial administration))

(Experimental method)

With regard to the timed function tests [i.e., 6-minute walk test (6MWT), time to stand (TTSTAND), time to climb 4 stairs (TTCLIMB), and time to run/walk 10 meters (TTRW)] and North Star Ambulatory Assessment (NSAA), measurement was carried out at 1 week before initial administration and each time point at which every 12 weeks passed from initial administration (Week 1) (i.e., at time points of Weeks 13, 25, 37, 49, 61, 73, and 85). The 201 trial was carried out in facilities belonging to CINRG, and the motor function tests were carried out in accordance with the standard operating procedures (SOP) and clinical evaluator training protocols used in CINRG DNHS. Since the subjects were children, some items could not be carried out in some tests because, for example, the subjects were not kept interested in implementation of the tests.

(Results)

The results are shown in Figure 9 to Figure 16. In the North Star Ambulatory Assessment, many patients with 5 or more years old in the Viltolarsen administered group maintained or improved their scores. According to the publication regarding studies, in which patient entry criteria such as those of clinical studies were established based on the natural history data in Italy and England, and changes in the NSAA scores 1 or 2 years later were studied, an average changes over the course of 1 year in target patients of exon 53 skipping treatment was -4.1 points (J Neurol Neurosurg Psychicary 87, 149-55, 2016). A changed amount at Week 48 of the patients of the Viltolarsen administered group who matched to the entry criteria of the publication was +1.3 points. According to the publication reporting the results of DHNS (Muscle Nerve 48, 55-67, 2013), 20% of DMD patients of 7 to 9 years old lose their rise ability, but there were no such patients in the Viltolarsen administered group. In addition, 10% of DMD patients of 7 to 9 years old lose ability to climb 4 stairs, but there were no such patients in the Viltolarsen administered group. The velocity decreases with aging, but an increase in the velocity was observed in the Viltolarsen administered group as a whole. Moreover, 10% of DMD patients of 7 to 9 years old lose independent walking ability, but there were no such patients in NSP. The velocity regarding the time to run/walk 10 meters decreases with aging, but an increase in the velocity was observed in the Viltolarsen administered group as a whole. With regard to a change in the expression level of dystrophin from the baseline of the quantitative dystrophin value measured by WB, and changes in velocities of the time to stand test, the time to climb 4 stairs test, and the time to run/walk 10 meters test at Week 48 from baseline, the presence or absence of correlation and a linear regression equation were calculating using Excel. With regard to the time to stand test and the time to run/walk 10 meters test, there was a significant correlation between the expression level of dystrophin and velocity change ($P < 0.05$), and it was suggested that a change in the velocity of the motor function increases with an increase in dystrophin.

Industrial Applicability

According to the present invention, provided is a pharmaceutical composition for use in the treatment of Duchenne muscular dystrophy, which has s stable composition of NS-065/NCNP-01 (Viltolarsen). Moreover, with regard to a pharmaceutical composition comprising NS-065/NCNP-01 (Viltolarsen), are provided dosage and administration method, which exhibit effective DMD treatments and are in a safe range for human patients. Using the pharmaceutical composition, the symptoms of Duchenne muscular dystrophy can be effectively reduced with low side effects.

-

**Claims**

1. A pharmaceutical composition for treating a human patient with Duchenne muscular dystrophy, the pharmaceutical composition comprising an antisense oligomer consisting of a base sequence complementary to a sequence consisting of nucleotides at positions 36 to 56 from the 5'-terminus of exon 53 of a human dystrophin gene or a pharmaceutically acceptable salt thereof, or a hydrate thereof, wherein
the treatment comprises intravenously administering to the human patient, the antisense oligomer, or a pharmaceutically acceptable salt thereof, or a hydrate thereof at a dose of between 40 mg/kg/week inclusive and 80 mg/kg/week inclusive.

2. The pharmaceutical composition according to claim 1, wherein the antisense oligomer, or a pharmaceutically acceptable salt thereof, or a hydrate thereof is intravenously administered to the human patient at a dose of 40 mg/kg/week.

3. The pharmaceutical composition according to claim 1, wherein the antisense oligomer, or a pharmaceutically acceptable salt thereof, or a hydrate thereof is intravenously administered to the human patient at a dose of 80 mg/kg/week.

4. The pharmaceutical composition according to claim 1, wherein the human patient has a mutation that that results in a deficiency of any exon selected from the group consisting of exons 43-52, 45-52, 47-52, 48-52, 49-52, 50-52, or 52, in a dystrophin gene.

5. The pharmaceutical composition according to claim 1, wherein the expression of a dystrophin protein in the human patient before the treatment is 1% or less compared with that of a healthy subject, as measured by Western blotting or mass spectrometry.

6. The pharmaceutical composition according to claim 5, wherein the expression of a dystrophin protein is not found in the human patient before the treatment.

7. The pharmaceutical composition according to claim 1, wherein the base sequence of the antisense oligomer consists of the sequence as set forth in SEQ ID NO: 3.

8. The pharmaceutical composition according to claim 1, wherein the antisense oligomer, or a pharmaceutically acceptable salt thereof, or a hydrate thereof is Viltolarsen or an equivalent thereof.

9. The pharmaceutical composition according to claim 1, comprising the antisense oligomer, or a pharmaceutically acceptable salt thereof, or a hydrate thereof, in a concentration of between 2.5 mg/ml inclusive and 500 mg/ml inclusive, or between 10 mg/ml inclusive and 100 mg/ml inclusive.

10. The pharmaceutical composition according to claim 1, comprising the antisense oligomer, or a pharmaceutically acceptable salt thereof, or a hydrate thereof, in a concentration of 25 mg/ml.

11. The pharmaceutical composition according to claim 1, comprising the antisense oligomer, or a pharmaceutically acceptable salt thereof, or a hydrate thereof, in a concentration of 50 mg/ml.

12. The pharmaceutical composition according to claim 1, further comprising at least one component selected from the group consisting of a tonicity agent, a pH adjuster, and a solvent.

13. The pharmaceutical composition according to claim 12, wherein the tonicity agent is at least one selected from the group consisting of sodium chloride, potassium chloride, glucose, fructose, maltose, sucrose, lactose, mannitol, sorbitol, xylitol, trehalose, and glycerin.

14. The pharmaceutical composition according to claim 12 or 13, wherein the pH adjuster is at least one selected from the group consisting of hydrochloric acid, sodium hydroxide, citric acid, lactic acid, phosphate (sodium hydrogen phosphate, sodium dihydrogen phosphate, and potassium dihydrogen phosphate), and monoethanolamine.

15. The pharmaceutical composition according to any one of claims 12 to 14, wherein the solvent is water.

16. The pharmaceutical composition according to claim 1, which comprises the antisense oligomer in a concentration of between 2.5 mg/ml inclusive and 500 mg/ml inclusive, or between 10 mg/ml inclusive and 100 mg/ml inclusive, and sodium chloride in a concentration of between 8 mg/ml inclusive and 10 mg/ml inclusive, and which is an aqueous solution with pH 7.2 to 7.4.

17. The pharmaceutical composition according to claim 1, wherein the treatment provides at least one effect selected from the group consisting of the following effects (1) to (6):

(1) the average value of the expression level of a dystrophin protein in the skeletal muscle of the patient is 9-fold or more increased in comparison to baseline, after administration of the pharmaceutical composition for 24 weeks;
(2) a change in the velocity obtained from time to stand (TTSTAND) is -0.055 times/sec or more in comparison to baseline, at the time of the 25th week after administration of the pharmaceutical composition for 24 weeks;
(3) a change in the velocity obtained from time to run/walk 10 meters (TTRW) is -0.025 meters/sec or more in comparison to baseline, at the time of the 25th week after administration of the pharmaceutical composition for 24 weeks;
(4) a change in the velocity obtained from time to climb 4 stairs (TTCLIMB) is -0.060 times/sec or more in comparison to baseline, at the time of the 25th week after administration of the pharmaceutical composition for 24 weeks;
(5) a change in the score of North Star Ambulatory Assessment (NSAA) is -2.2 scores or more in comparison to baseline, at the time of the 25th week after administration of the pharmaceutical composition for 24 weeks; and
(6) a change in 6-minute walk test (6MWT) is -7.5 meters or more in comparison to baseline, at the time of the 25th week after administration of the pharmaceutical composition for 24 weeks.

18. The pharmaceutical composition according to claim 1, wherein when the treatment is performed on 7- to 9-year-old human patients with Duchenne muscular dystrophy for 84 weeks, at least one effect selected from the group consisting of the following effects (1) to (6) is provided:

(1) the percentage of patients who lose rise ability is less than 20% by the time of the 85th week after initiation of the treatment;
(2) the percentage of patients who lose ability to climb 4 stairs is less than 10% by the time of the 85th week after initiation of the treatment;
(3) the percentage of patients who lose independent walking ability is less than 10% by the time of the 85th week after initiation of the treatment;
(4) a reduction in the velocity of running/walking 10 meters due to aging is not observed by the time of the 85th week after initiation of the treatment;
(5) a reduction in the velocity of climbing 4 stairs due to aging is not observed by the time of the 85th week after initiation of the treatment; and
(6) a reduction in rise velocity due to aging is not observed by the time of the 85th week after initiation of the treatment.

**19.** The pharmaceutical composition according to claim 1, wherein when the treatment is performed on 10- to 12-year-old human patients with Duchenne muscular dystrophy for 84 weeks, at least one effect selected from the group consisting of the following effects (1) to (6) is provided:

(1) the percentage of patients who lose rise ability is less than 60% by the time of the 85th week after initiation of the treatment;
(2) the percentage of patients who lose ability to climb 4 stairs is less than 50% by the time of the 85th week after initiation of the treatment;
(3) the percentage of patients who lose independent walking ability is less than 50% by the time of the 85th week after initiation of the treatment;
(4) a reduction in the velocity of running/walking 10 meters due to aging is not observed by the time of the 85th week after initiation of the treatment;
(5) a period in which the velocity of climbing 4 stairs increases is observed by the time of the 85th week after initiation of the treatment; and
(6) a period in which rise velocity increases is observed by the time of the 85th week after initiation of the treatment.

**20.** A method for treating Duchenne muscular dystrophy, comprising intravenously administering a pharmaceutical composition comprising an antisense oligomer consisting of a base sequence complementary to a sequence consisting of nucleotides at positions 36 to 56 from the 5'-terminus of exon 53 of a human dystrophin gene, or a pharmaceutically acceptable salt thereof, or a hydrate thereof, to a human patient once a week at a dose of between 40 mg/kg/week inclusive and 80 mg/kg/week inclusive of the antisense oligomer, or a pharmaceutically acceptable salt thereof, or a hydrate thereof.

**21.** An antisense oligomer consisting of a base sequence complementary to a sequence consisting of nucleotides at positions 36 to 56 from the 5'-terminus of exon 53 of a human dystrophin gene, or a pharmaceutically acceptable salt thereof, or a hydrate thereof, for use in a method for treating a human patient with Duchenne muscular dystrophy, wherein
the antisense oligomer, or a pharmaceutically acceptable salt thereof, or a hydrate thereof is intravenously administered to the human patient once a week at a dose of between 40 mg/kg/week inclusive and 80 mg/kg/week inclusive.

**22.** Use of an antisense oligomer consisting of a base sequence complementary to a sequence consisting of nucleotides at positions 36 to 56 from the 5'-terminus of exon 53 of a human dystrophin gene, or a pharmaceutically acceptable salt thereof, or a hydrate thereof, for the manufacture of a pharmaceutical composition for treating a human patient with Duchenne muscular dystrophy, wherein
the antisense oligomer, or a pharmaceutically acceptable salt thereof, or a hydrate thereof is intravenously administered to the human patient once a week at a dose of between 40 mg/kg/week inclusive and 80 mg/kg/week inclusive.

Figure 1

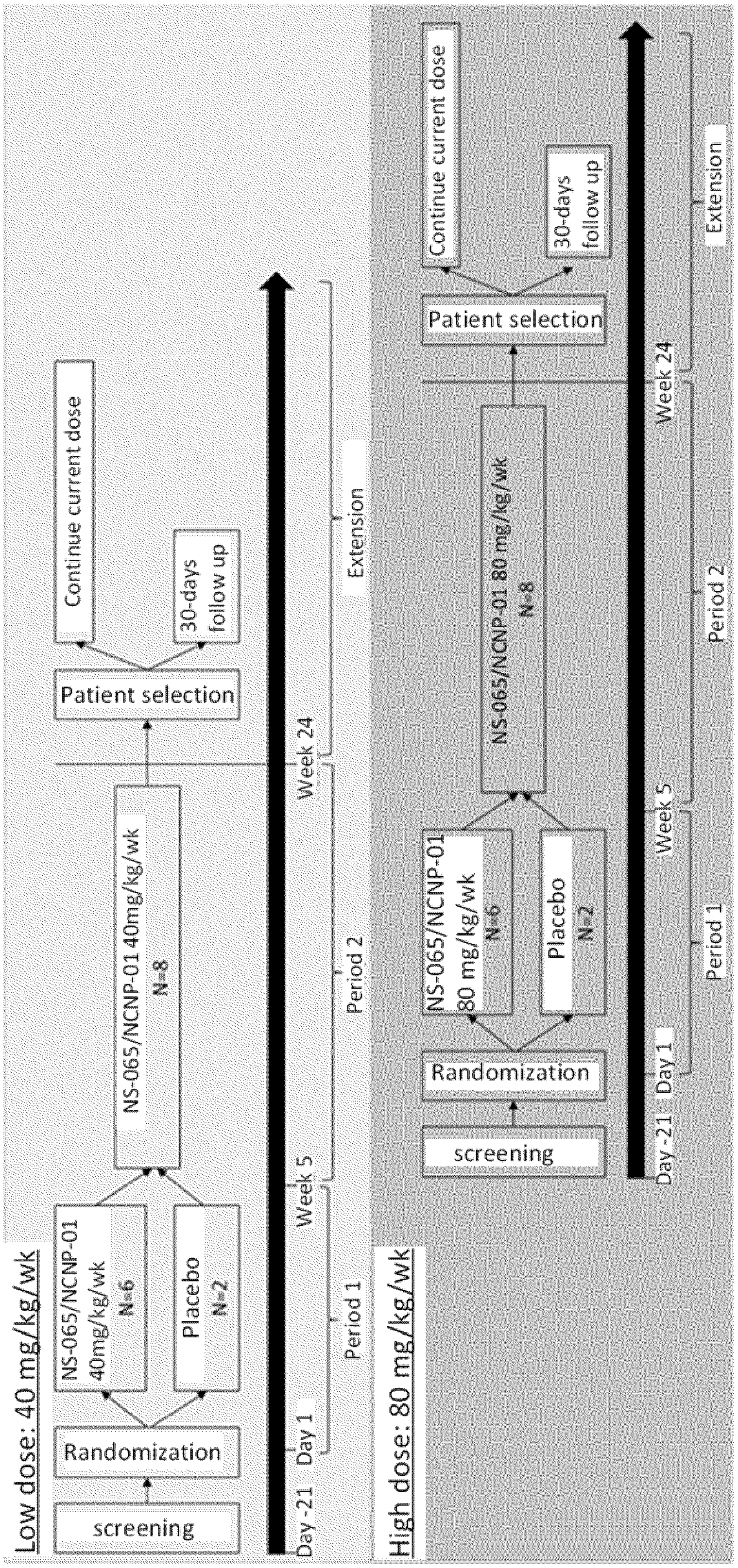


Mg: milligram; kg: kilogram

Figure 2

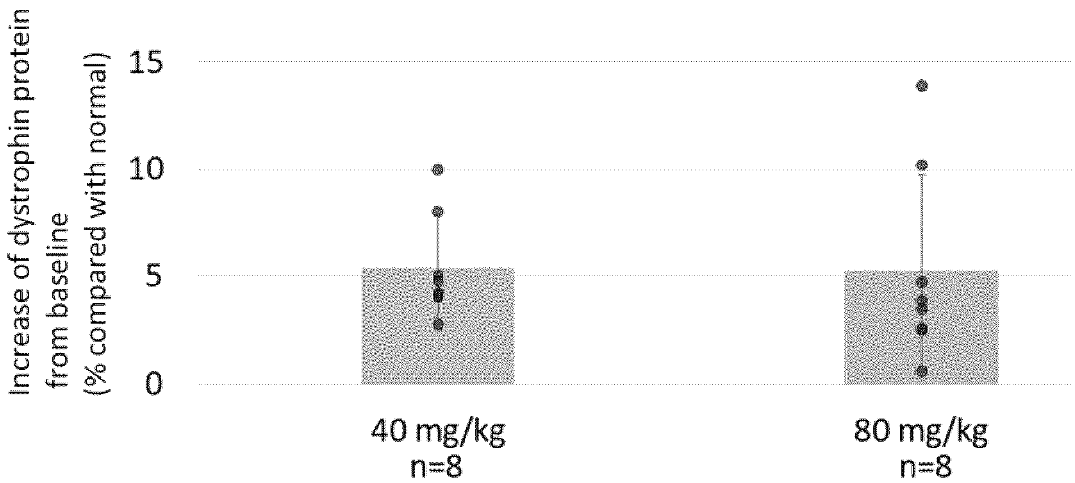
Increase of dystrophin protein
from baseline
(% compared with normal)
15
10
5
0
40 mg/kg
n=8
80 mg/kg
n=8

Figure 3

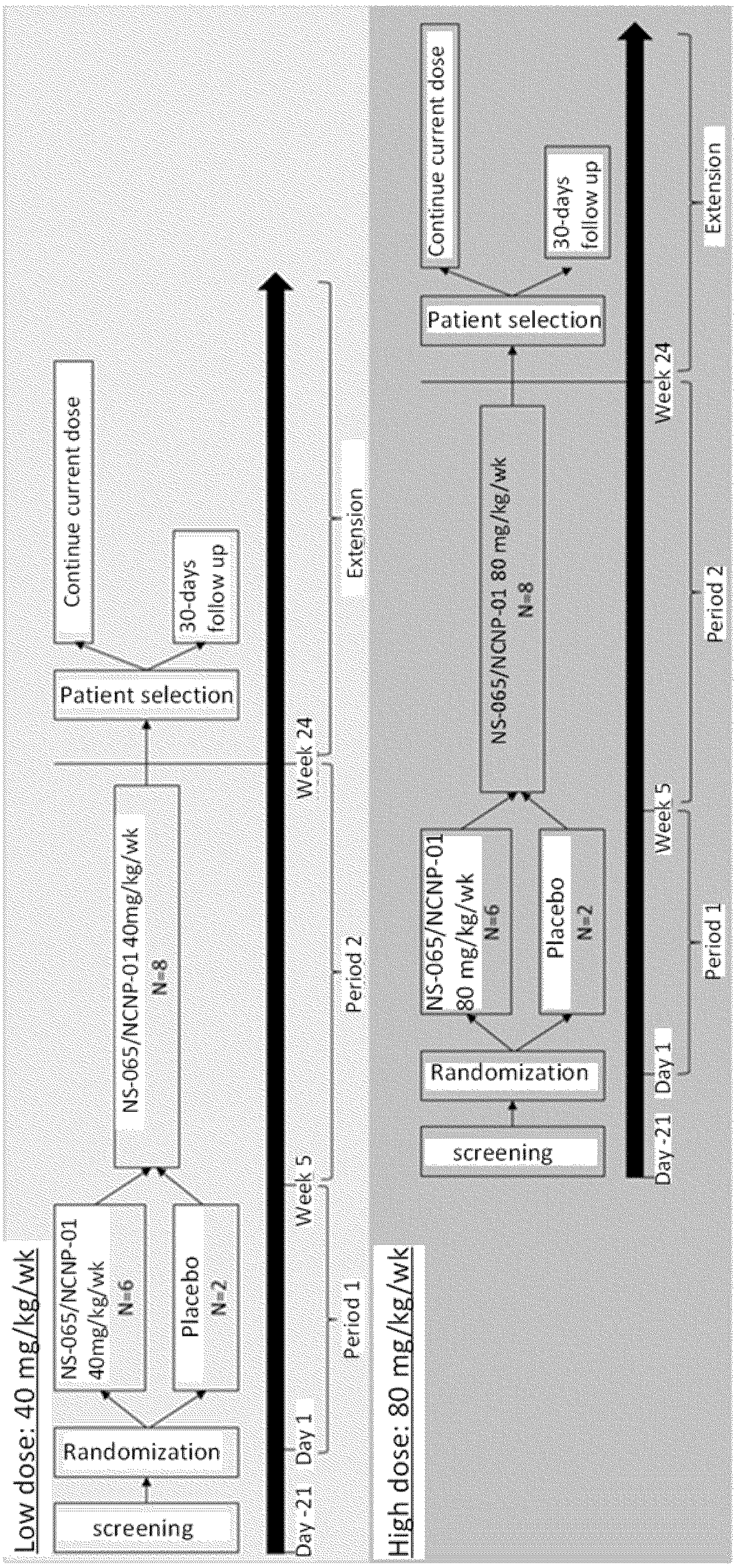


Mg: milligram; kg: kilogram

Figure 4

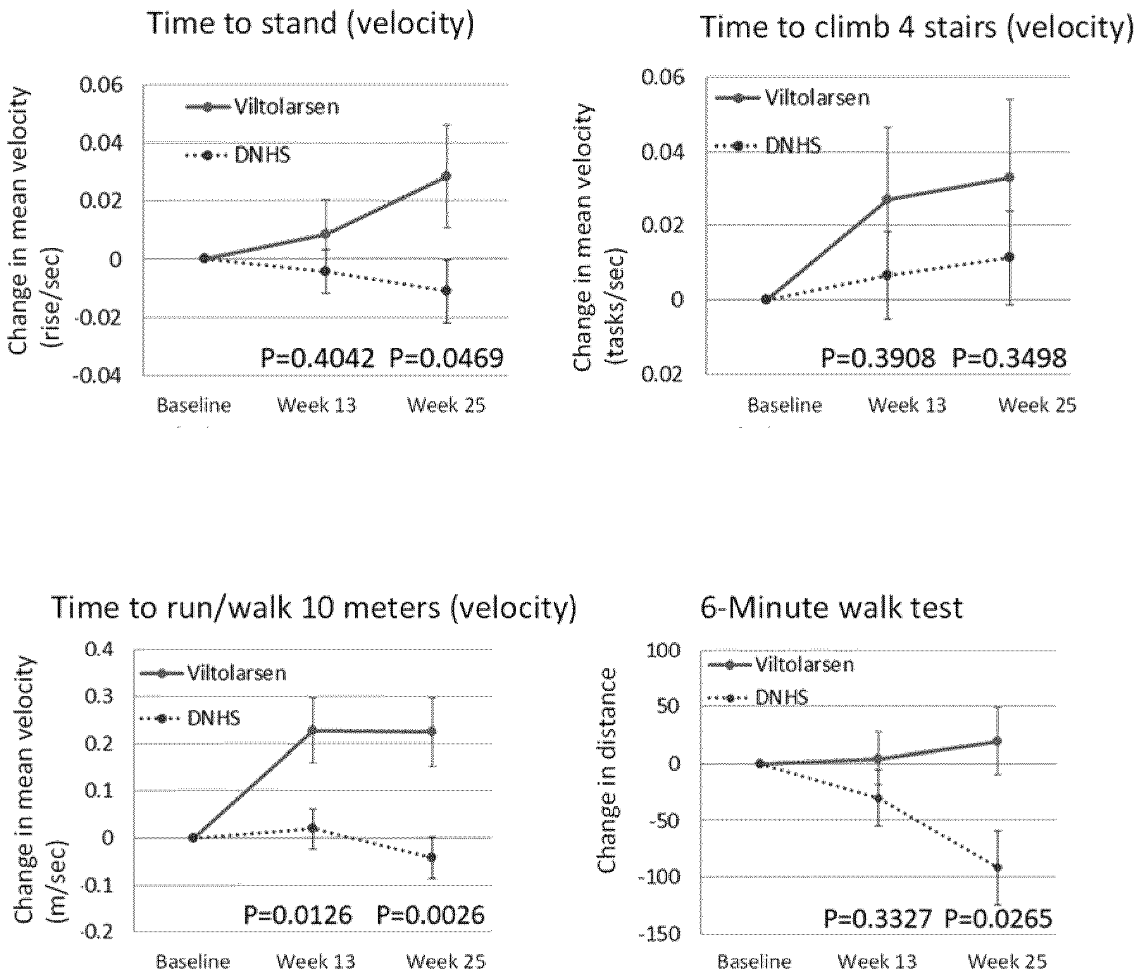


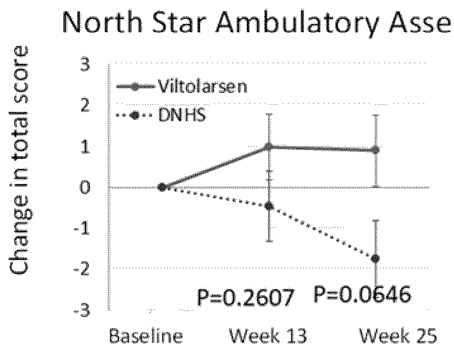


The observed sample sizes for DNHS

| | Baseline | Week 13 | Week 25 |
|---|---|---|---|
| TTSTAND | 69 | 43 | 46 |
| TTCLIMB | 69 | 44 | 45 |
| TTRW | 69 | 44 | 46 |
| 6MWT | 18 | 14 | 11 |
| NSAA | 20 | 13 | 13 |

Figure 5

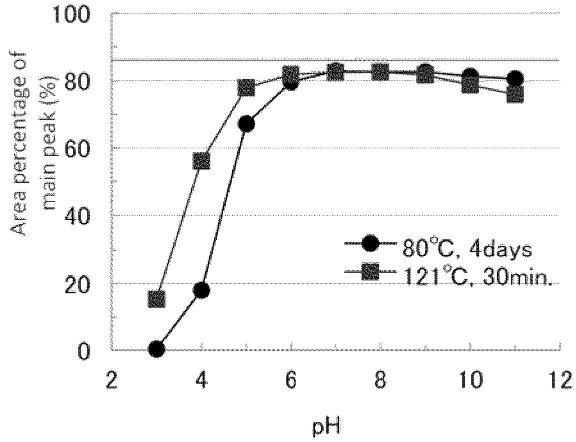


Figure 6

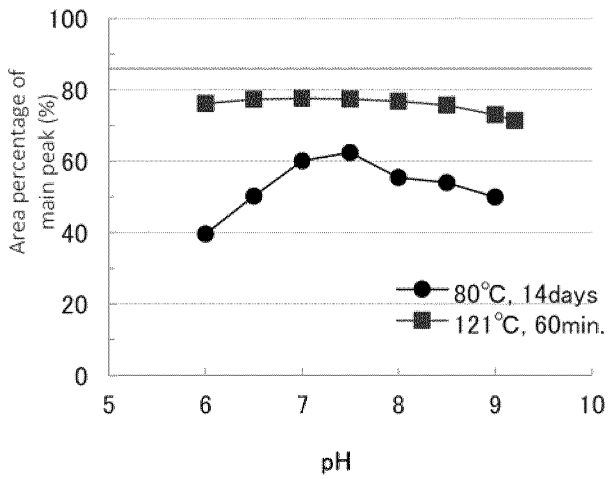

Figure 7

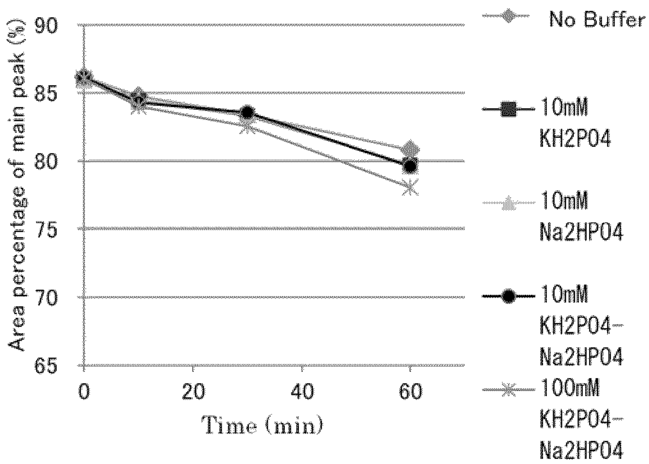
Area percentage of main peak (%)
90
85
80
75
70
65
0
20
40
60
Time (min)
No Buffer
10mM KH2PO4
10mM Na2HPO4
10mM KH2PO4-Na2HPO4
100mM KH2PO4-Na2HPO4

Figure 8

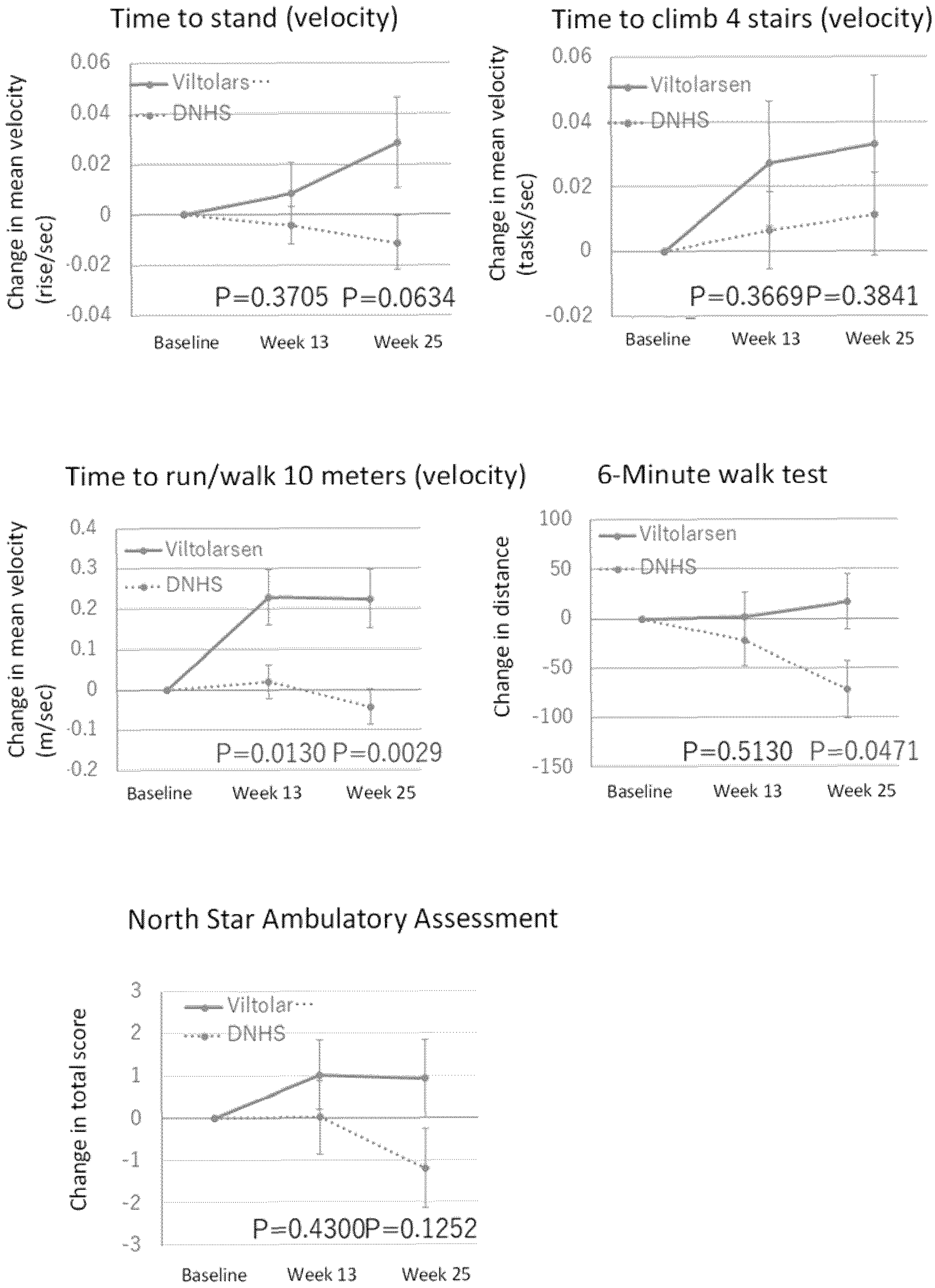
Time to stand (velocity)
Change in mean velocity (rise/sec)
0.06
0.04
0.02
0
-0.02
-0.04
Viltolars…
DNHS
P=0.3705 P=0.0634
Baseline
Week 13
Week 25
Time to climb 4 stairs (velocity)
Change in mean velocity (tasks/sec)
0.06
0.04
0.02
0
-0.02
Viltolarsen
DNHS
P=0.3669P=0.3841
Baseline
Week 13
Week 25
Time to run/walk 10 meters (velocity)
Change in mean velocity (m/sec)
0.4
0.3
0.2
0.1
0
-0.1
-0.2
Viltolarsen
DNHS
P=0.0130 P=0.0029
Baseline
Week 13
Week 25
6-Minute walk test
Change in distance
100
50
0
-50
-100
-150
Viltolarsen
DNHS
P=0.5130 P=0.0471
Baseline
Week 13
Week 25
North Star Ambulatory Assessment
Change in total score
3
2
1
0
-1
-2
-3
Viltolar…
DNHS
P=0.4300P=0.1252
Baseline
Week 13
Week 25

Figure 9

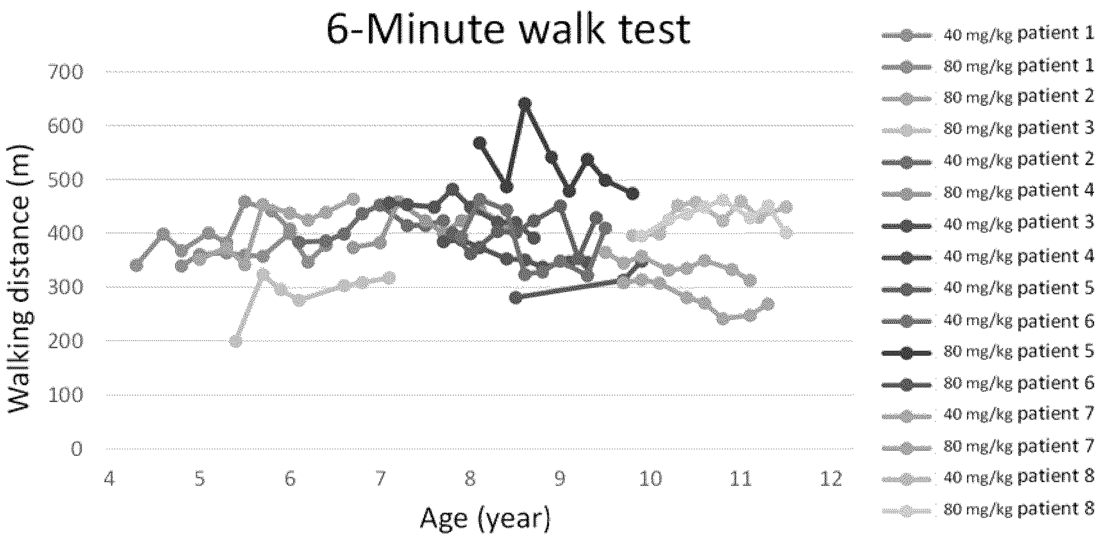


Figure 10

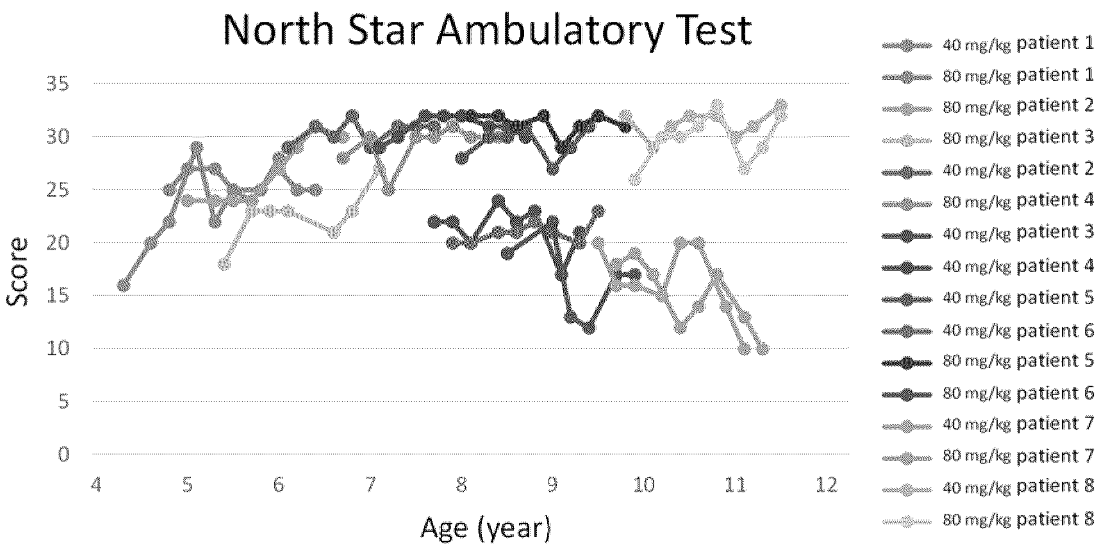

Figure 11

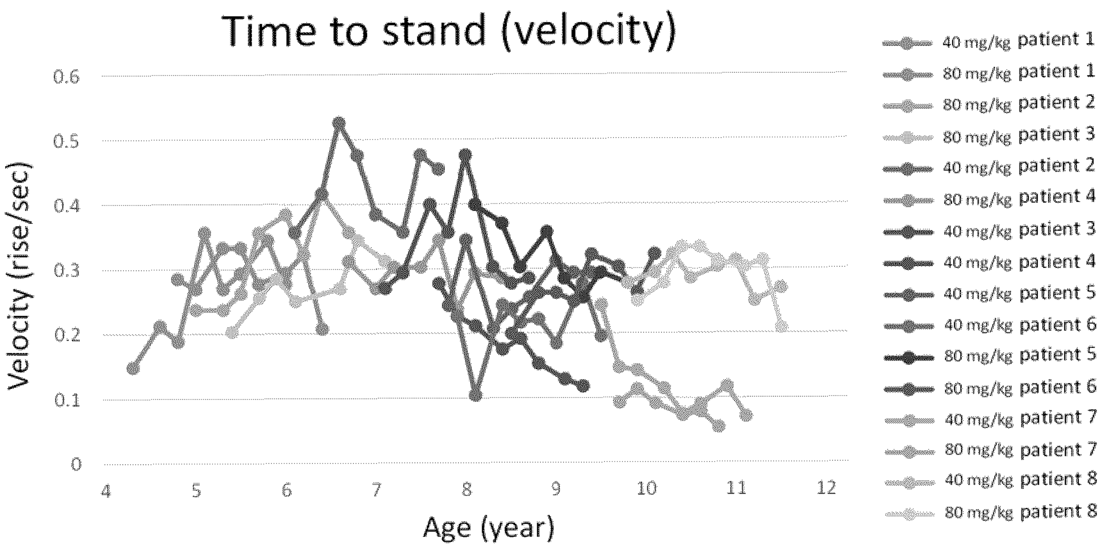


Figure 12

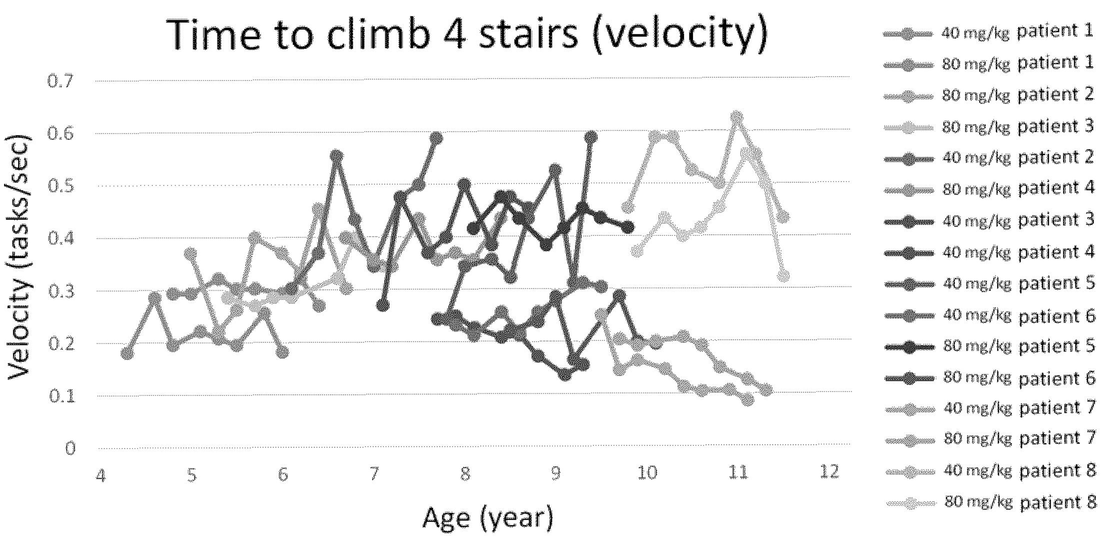

Figure 13

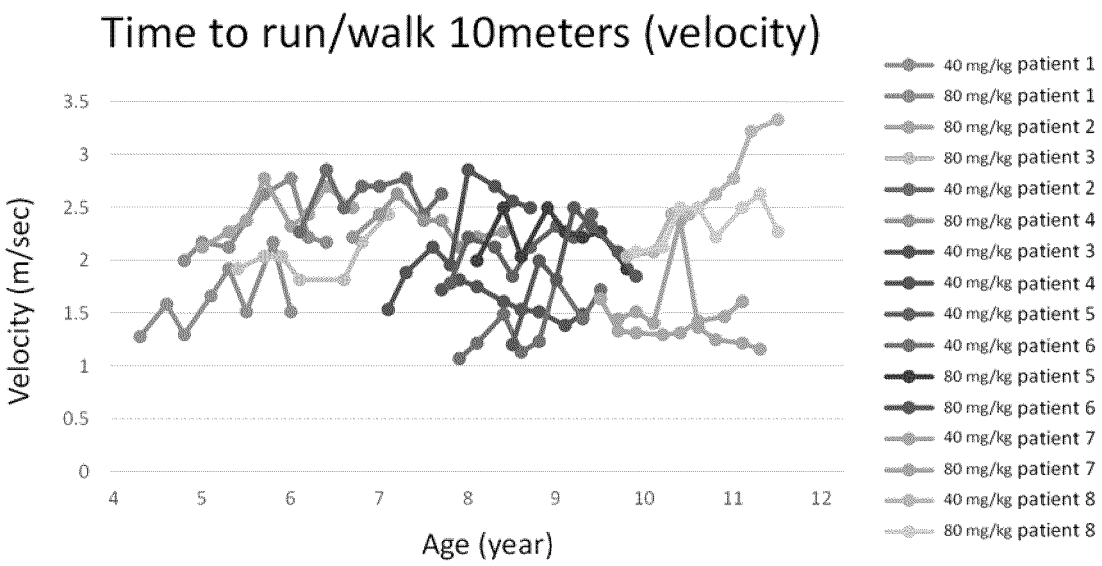


Figure 14

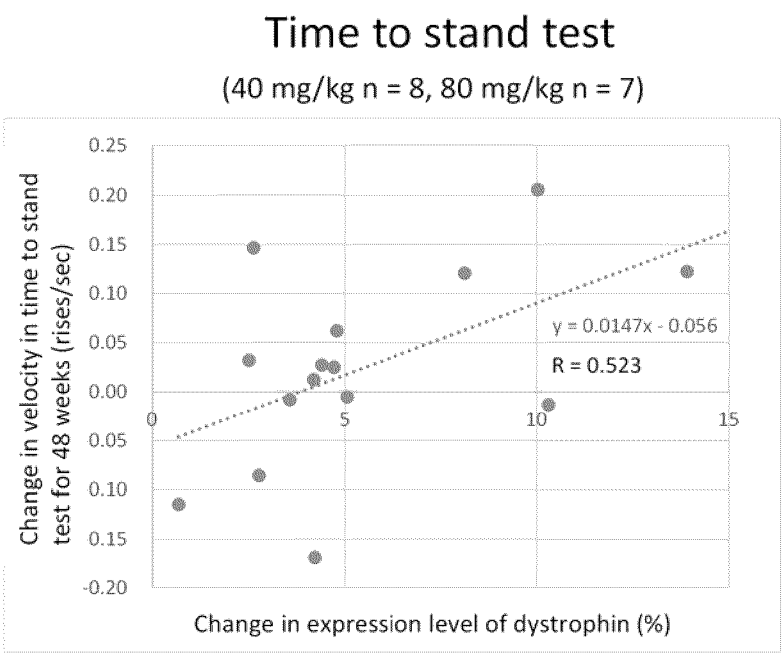

Figure 15

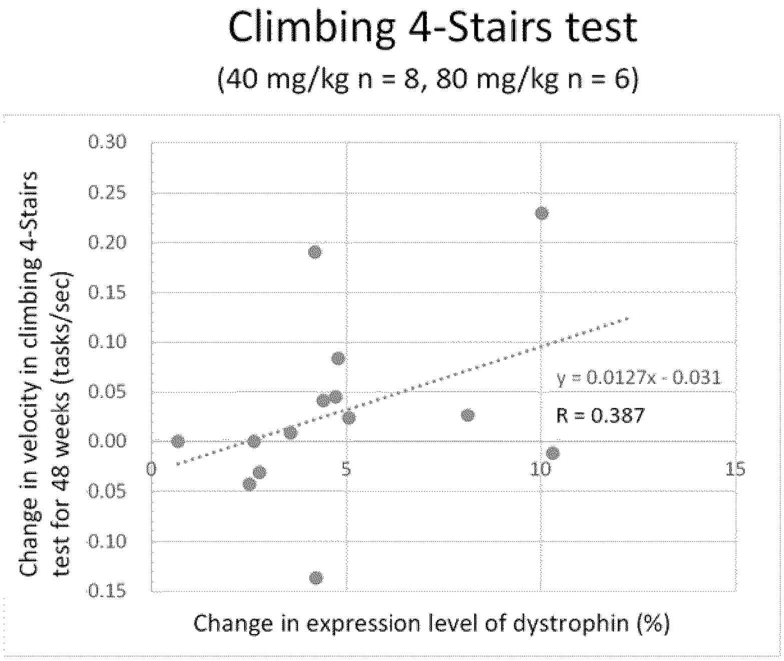


Figure 16

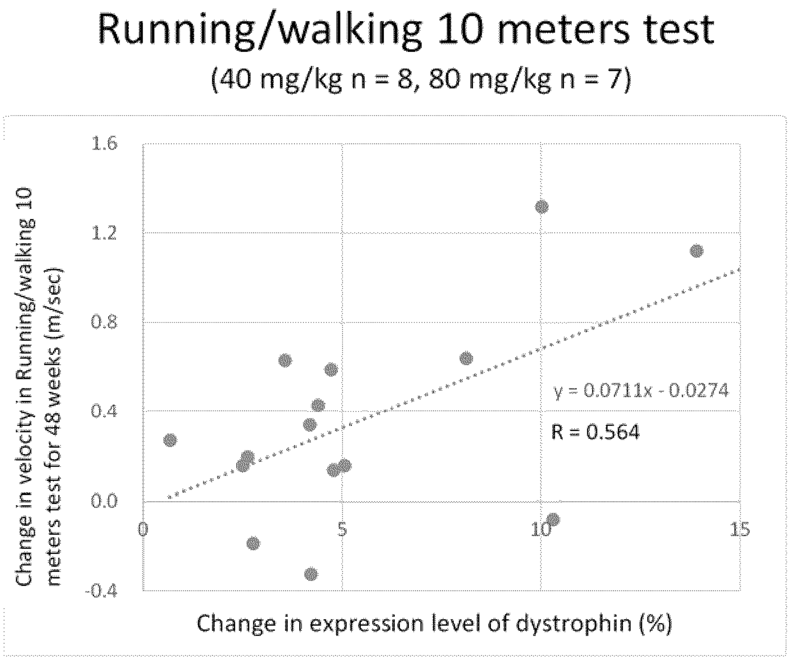

## INTERNATIONAL SEARCH REPORT

International application No.
PCT/JP2019/026393

A. CLASSIFICATION OF SUBJECT MATTER
Int.Cl. A61K31/7088(2006.01)i, A61P21/04(2006.01)i, C12N15/113(2010.01)n, C12N15/12(2006.01)n

According to International Patent Classification (IPC) or to both national classification and IPC

B. FIELDS SEARCHED
Minimum documentation searched (classification system followed by classification symbols)
Int.Cl. A61K31/00-33/44, A61K9/00-9/72, A61K47/00-47/69, A61P21/00, C12N15/00-15/90

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580(JDreamIII), CAplus/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2014-054250 A (NIPPON SHINYAKU CO., LTD.) 27 March 2014, claims 1, 2, paragraphs [0013], [0087]-[0096], [0108], [0135]-[0142], [0154]-[0157], SEQ ID NO.: 35<br>& US 2013/0211062 A1, claims 1, 2, paragraphs [0034], [0164]-[0174], [0258]-[0269], [0303]-[0313], table 2 & EP 2612917 A1 | 21<br>1-20, 22 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15.08.2019 | 27.08.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2019/026393

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2017/059131 A1 (SAREPTA THERAPEUTICS, INC.) 06 April 2017, claim 1, page 23, line 26 to page 24, line 5, page 55, line 25 to page 65, line 5 & US 2019/0054113 A1 & JP 2018-529715 A | 1-20, 22 |
| A | KOMAKI, H. et al., Systemic administration of the antisense oligonucleotide NS-065/NCNP-01 for skipping of exon 53 in patients with Duchenne muscular dystrophy, Science Translational Medicine, 18 April 2018, vol. 10, eaan0713, pp. 1-11 | 1-22 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006000057 A
- WO 2004048570 A
- US 20100168212 A
- WO 2010048586 A
- US 62690270 B
- US 62739386 B
- WO 2006129594 A
- WO 2006038608 PCT
- WO 1991009033 A
- WO 2009064471 A
- US 9079934 B2
- US 9506058 B2


### Non-patent literature cited in the description


- **MONACO A. P. et al.** *Genomics,* 1988, vol. 2, 90-95
- **MATSUO M.** *Brain Dev,* 1996, vol. 18, 167-172
- **WILTON S. D. et al.** *Molecular Therapy,* 2007, vol. 15, 1288-96
- **ANNEMIEKE AARTSMA-RUS et al.** *Neuromuscular Disorders,* 2002, vol. 12, S71-S77
- **LINDA J. POPPLEWELL et al.** *Neuromuscular Disorders,* 2010, vol. 20 (2), 102-10
- **BLADEN C. L. et al.** *Human Mutation,* 2015, vol. 36, 395-402
- **ROBERTS, RG. et al.** *Genomics,* 1993, vol. 16, 536-538
- **CARLIN ; ARTHUR.** Basic Local Alignment Search Tool. *Proc. Natl. Acad. Sci. USA,* 1990, 872264-2268
- *Proc Natl Acad Sci USA,* 1993, vol. 90, 5873
- **ALTSCHUL SF et al.** *J Mol Biol,* 1990, vol. 215, 403
- **ENYA et al.** *Bioorganic & Medicinal Chemistry,* 2008, vol. 18, 9154-9160
- **P. E. NIELSEN ; M. EGHOLM ; R. H. BERG ; O. BUCHARDT.** *Science,* 1991, vol. 254, 1497
- **M. EGHOLM ; O. BUCHARDT ; P. E. NIELSEN ; R. H. BERG.** *Jacs.,* 1992, vol. 114, 1895
- **K. L. DUEHOLM ; M. EGHOLM ; C. BEHRENS ; L. CHRISTENSEN ; H. F. HANSEN ; T. VULPIUS ; K. H. PETERSEN ; R. H. BERG ; P. E. NIELSEN ; O. BUCHARDT.** *J. Org. Chem.,* 1994, vol. 59, 5767
- **L. CHRISTENSEN ; R. FITZPATRICK ; B. GILDEA ; K. H. PETERSEN ; H. F. HANSEN ; T. KOCH ; M. EGHOLM ; O. BUCHARDT ; P. E. NIELSEN ; J. COULL.** *J. Pept. Sci.,* 1995, vol. 1, 175
- **T. KOCH ; H. F. HANSEN ; P. ANDERSEN ; T. LARSEN ; H. G. BATZ ; K. OTTESON ; H. ORUM.** *J. Pept. Res.,* 1997, vol. 49, 80
- **ANNEMIEKE AARTSMA-RUS ; MATTIE BREMMER-BOUT ; ANNEKE A.M. JANSON ; JOHAN T. DEN DUNNEN ; GERT-JAN B. VAN OMMEN ; JUDITH C.T. VAN DEUTEKOM.** Targeted exon skipping as a potential gene correction therapy for Duchenne muscular dystrophy. *Neuromuscular Disorders,* 2002, vol. 12, S71-S77
- **HOFFMAN EP ; BROWN RH ; KUNKEL LM.** Dystrophin: the protein product of the Duchenne muscular dystrophy locus. *Cell,* 1987, vol. 51, 919-928
- **BLADEN CL ; SALGADO D ; MONGES S ; FONCUBERTA ME ; KEKOU K ; KOSMA K ; DAWKINS H ; LAMONT L ; ROY AJ ; CHAMOVA T.** The TREAT-NMD DMD Global Database: analysis of more than 7,000 Duchenne muscular dystrophy mutations. *Hum Mutat.,* 2015, vol. 36 (4), 395-402
- **SAKTHIVEL MURUGAN S.M. ; ARTHI CHANDRAMOHAN ; BREMADESAM RAMAN LAKSHMI.** Use of multiplex ligation-dependent probe amplification (MLPA) for Duchenne muscular dystrophy (DMD) gene mutation analysis. *Indian Journal of Medical Research,* September 2010, vol. 132, 303-311
- **CIRAK S ; ARECHAVALA-GOMEZA V ; GUGLIERI M ; FENG L ; TORELLI S ; ANTHONY K ; ABBS S ; GARRALDA ME ; BOURKE J ; WELLS DJ.** Exon skipping and dystrophin restoration in patients with Duchenne muscular dystrophy after systemic phosphorodiamidate morpholino oligomer treatment: an open-label, phase 2, dose-escalation study. *Lancet,* 2011, vol. 378, 595-605
- **VOIT T ; TOPALOGLU H ; STRAUB V ; MUNTONI F ; DECONINCK N ; CAMPION G ; DE KIMPE SJ ; EAGLE M ; GUGLIERI M ; HOOD S.** Safety and efficacy of drisapersen for the treatment of Duchenne muscular dystrophy (DEMAND II): an exploratory, randomised, placebo-controlled phase 2 study. *Lancet Neurol.,* 2014, vol. 13 (10), 987-96

- **YOKOTA T ; NAKAMURA A ; NAGATA T ; SAITO T ; KOBAYASHI M ; AOKI Y ; ECHIGOYA Y ; PARTRIDGE T ; HOFFMAN EP ; TAKEDA S.** Extensive and Prolonged Restoration of Dystrophin Expression with Vivo-Morpholino-Mediated Multiple Exon Skipping in Dystrophic Dogs. *Nucleic Acid Ther,* 2012, vol. 22 (5), 306-15
- **ERIC P. HOFFMAN et al.** Characterization of Dystrophin in Muscle-Biopsy Specimens from Patients with Duchenne's or Becker's Muscular Dystrophy. *N. Engl. J. Med.,* 1988, vol. 318, 1363-1368
- **KENJI ROWEL Q LIM et al.** Eteplirsen in the treatment of Duchenne muscular dystrophy. *Drug Des. Devel. Ther.,* 2017, vol. 11, 533-545, https://www.ncbi.nlm.nih.gov/pmc/articles/PMC5338848
- **ABRESCH RT ; HAN JJ ; ESCOLAR DM ; FLORENCE JM ; DUONG T ; ARRIETA A ; CLEMENS PR ; HOFFMAN EP ; CNAAN A.** CINRG Investigators. The cooperative international neuromuscular research group Duchenne natural history study - a longitudinal investigation in the era of glucocorticoid therapy: design of protocol and the methods used. *Muscle Nerve.,* 2013, vol. 48 (1), 32-54
- **HENRICSON EK ; ABRESCH RT ; CNAAN A ; HU F ; DUONG T ; ARRIETA A ; HAN J ; ESCOLAR DM ; FLORENCE JM ; CLEMENS PR.** CINRG Investigators. The cooperative international neuromuscular research group Duchenne natural history study: glucocorticoid treatment preserves clinically meaningful functional milestones and reduces rate of disease progression as measured by manual muscle testing and other commonly used clinical trial outcome measures. *Muscle Nerve.,* 2013, vol. 48 (1), 55-67
- **MCDONALD CM ; HENRICSON EK ; ABRESCH RT ; DUONG T ; JOYCE NC ; HU F ; CLEMENS PR ; HOFFMAN EP ; CNAAN A ; GORDISH-DRESSMAN H.** CINRG Investigators. Long-term effects of glucocorticoids on function, quality of life, and survival in patients with Duchenne muscular dystrophy: a prospective cohort study. *Lancet,* 2018, vol. 391 (10119), 451-461
- The 6-minute Walk Test as a New Outcome Measure in Duchenne Muscular Dystrophy. **CRAIG M. MCDONALD ; MD, ERIK K. HENRICSON ; MPH, JAY J. HAN ; MD, R. TED ABRESCH MS ; ALINA NICORICI, BS ; GARY L. ELFRING, MS ; LEONE ATKINSON MD ; PHD, ALLEN REHA BS ; SAMIT HIRAWAT MD ; LANGDON L. MILLER MD.** Muscle & Nerve. Wiley Periodicals, Inc, April 2010, vol. 41, 500-510
- The 6-minute Walk Test and Other Clinical Endpoints in Duchenne Muscular Dystrophy: Reliability, Concurrent Validity, and Minimal Clinically Important Differences from a Multicenter Study. **CRAIG M. MCDONALD ; MD, ERIK K. HENRICSON ; MPH, R. TED ABRESCH ; MS, JULAINE FLORENCE ; PHD, MICHELLE EAGLE ; PHD, EDUARD GAPPMAIER ; PHD, ALLAN M. GLANZMAN ; DPT, ROBERT SPIEGEL, MD ; JAY BARTH, MD ; GARY ELFRING, MS.** Muscle & Nerve. Wiley Periodicals, Inc, September 2013, vol. 48, 357-368
- **MAYHEW JE ; FLORENCE JM ; MAYHEW TP ; HENRICSON EK ; LESHNER RT ; MCCARTER RJ et al.** Reliable surrogate outcome measures in multicenter clinical trials of Duchenne muscular dystrophy. *Muscle & Nerve,* 2007, vol. 35 (1), 36-42
- Reliability of the North Star Ambulatory Assessment in a multicentric setting. **E.S. MAZZONE ; S. MESSINA ; G. VASCO ; M. MAIN ; M. EAGLE ; A. D'AMICO ; L. DOGLIO ; L. POLITANO ; F. CAVALLARO ; S. FROSINI.** Neuromuscular Disorders. Elsevier B.V, July 2009, vol. 19, 458-461
- *Hum Mutat.,* 2018, vol. 39
- *Hum Mutat.,* 2008, vol. 29 (5), 728-37
- *Hum Mutat.,* 2018, vol. 39, 1193-1202
- *J Neurol Neurosurg Psychicary,* 2016, vol. 87, 149-55
- *Muscle Nerve,* 2013, vol. 48, 55-67

SEQUENCE LISTING

<110> NIPPON SHINYAKU CO., LTD.

<120> COMPOSITION CONTAINING ANTISENSE OLIGONUCLEOTIDE AND USE THEREOF TO TREAT DUCHENNE MUSCULAR DYSTROPHY

<130> G2329

<150> US 62/690,270
<151> 2018-06-26

<150> US 62/739,386
<151> 2018-10-01

<160> 6

<170> PatentIn version 3.5

<210> 1
<211> 212
<212> DNA
<213> Homo sapiens

<400> 1
ttgaaagaat tcagaatcag tgggatgaag tacaagaaca ccttcagaac cggaggcaac 60

agttgaatga aatgttaaag gattcaacac aatggctgga agctaaggaa gaagctgagc 120

aggtcttagg acaggccaga gccaagcttg agtcatggaa ggagggtccc tatacagtag 180

atgcaatcca aaagaaaatc acagaaacca ag 212

<210> 2
<211> 21
<212> DNA
<213> Homo sapiens

<400> 2
gaacaccttc agaaccggag g 21

<210> 3
<211> 21
<212> DNA
<213> Artificial

<220>
<223> Synthetic Nucleic Acid

<400> 3
cctccggttc tgaaggtgtt c 21

<210> 4
<211> 13
<212> PRT
<213> Artificial

<220>
<223> Synthetic Peptide

```
<400>  4

Ile Phe Leu Thr Glu Gln Pro Leu Glu Gly Leu Glu Lys
1               5                   10


<210>  5
<211>  14
<212>  PRT
<213>  Artificial

<220>
<223>  Synthetic Peptide

<400>  5

Val Ala Val Gly Gln Glu Gln Ala Phe Ser Val Asn Thr Arg
1               5                   10


<210>  6
<211>  15
<212>  PRT
<213>  Artificial

<220>
<223>  Synthetic Peptide

<400>  6

Ser Pro Phe Val Val Asn Val Ala Pro Pro Leu Asp Leu Ser Lys
1               5                   10                  15
```